# EUROPEAN PATENT APPLICATION

(11) **EP 1 529 531 A1**
(43) Date of publication of application: **11.05.2005**
(21) Application number: 03025380.1
(22) Date of filing: 04.11.2003
(51) Int. Cl.: A61K 31/495, C07D 498/04, A61P 25/00, A61P 31/00

(54) **Oxadiazolopyrazine derivatives as pharmaceutically active compounds**

(71) Applicant: 4SC AG, 82152 Martinsried (DE)
(72) Inventor: Aulinger-Fuchs, Katharina, Dr., 82061 Neuried (DE); Herz, Thomas, Dr., 82131 Stockdorf (DE); Krauss, Rolf, Dr., 82152 Planegg-Martinsried (DE); Kubbutat, Michael, Dr., 79227 Schallstadt (DE); Lang, Martin, Dr., 82166 Gräfelfing (DE); Schächtele, Christoph, Dr., 79108 Freiburg (DE); Totzke, Frank, Dr., 79098 Freiburg (DE)
(74) Representative: Strehl Schübel-Hopf & Partner

(57) **Abstract**

The present invention relates to furazanopyrazine derivatives of the general formula (I): 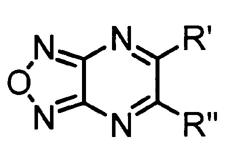 wherein:
R' represents -NR¹R² or -OR⁹
R" represents -NR⁵-NR³R⁴, -NR⁵-OR^{b}, -O-NR³R⁴;
wherein R¹ to R⁹ in formula (1) represent independently of each other a variety of different substituents comprising alkyl, aryl, aralkyl, alkylaryl, heteroaryl groups and monofunctional moieties.

## Description

The present invention relates to furazanopyrazine derivatives, the use of the furazanopyrazine derivatives as pharmaceutically active agents, especially to treat protein kinase-dependent diseases and conditions, such as cancer, angiogenesis, atherosclerosis, inflammatory diseases, neurodegenerative diseases and the like in mammals, as well as compositions containing at least one furazanopyrazine derivative and/or pharmaceutically acceptable salt thereof, and methods for preventing and/or treating such diseases. Furthermore, a process for preparing said furazanopyrazine derivatives is disclosed.

### Background of the invention

Protein kinases play a central role in the regulation of cellular functions. This includes processes like cell growth and division, cell differentiation and cell death, but also many other cellular activities. Protein kinases catalyse the transfer of phosphate residues from ATP on target proteins which as a consequence of this protein kinase mediated phosphorylation change their three-dimensional structure and thereby their physiological function. Depending on the amino acid which becomes phosphorylated by a protein kinase these enzymes are grouped in two families, the so-called serine/threonine protein kinases and the tyrosine protein kinases.
Based on the human genome project it is known that in human beings their exist 518 DNA sequences which encode for a protein kinase-like protein sequence. In the last about 20 years for several of this 518 proteins it could be shown that modifications in their related gene sequences (e.g. point mutations, deletions or gene amplifications) result in pathological changes of the cellular activities of the corresponding protein kinase. This is in particular true for protein kinases which are involved in cell proliferation and cell cycle control, in survival of cells and cell death, in tumor angiogenesis, and in formation of tumor metastases.
Several so-called oncogenes are pathologically modified genes which in their proto-oncogenic form encode for protein kinases involved in normal, physiological regulation of cell growth and division.
Since protein kinases are key regulators of cell functions and since they can show dysregulated enzymatic activity in cells they are promising targets for the development of therapeutic agents. There are many ongoing drug discovery projects in the pharmaceutical industry with the goal to identify modulators of protein kinases. The major focus is currently on protein kinases involved in inflammation and cancer, but besides this protein kinases are currently discussed as promising targets in almost every disease area.
In the tumor field the first protein kinase inhibitor (Gleevec) has already reached the market. In addition, a great number of protein kinase inhibitors are currently in various phases of clinical development. In most cases these compounds are either targeting subtypes of the EGF (Epidermal Growth Factor) receptor or of the VEGF (Vascular Endothelial Growth Factor) receptor. All these compounds have been developed with the goal to specifically inhibit one particular protein kinase, for which there is evidence that it interferes with one of the four major molecular processes of tumor progression. These four processes are (1) cell proliferation/cell cycle control, (2) regulation of programmed cell death (apoptosis) and cell survival, (3) tumor angiogenesis and (4) tumor metastasis.
The present invention relates to furazanopyrazine derivatives which may be useful for inhibition of protein kinases involved in diseases besides cancer, but which are especially useful as anti-tumor agents. This includes monospecific protein kinase inhibitors, which preferentially inhibit one protein kinase which is causatively involved in tumor progression, but also so-called multi-target protein kinase inhibitors, which inhibit at least two different protein kinases which play a role in two or more different molecular mechanism of tumor progression. As an example, such a compound could be an inhibitor of tumor angiogenesis and, in addition, also a stimulator of apoptosis.
The concept of multi-target protein kinase inhibitors is a new approach although the idea of developing "multiplex protein kinase inhibitors" has already been described by J. Adams et al., Current Opinion in Chemical Biology 6, 486-492, 2002. Therein compounds are described, which, at the same time, inhibit several protein kinases, which however all are involved in one molecular mechanism of tumor progression, namely tumor angiogenesis.

In WO 02/44378, "WNT signalling assay, method and uses thereof", 5,6-diaminofurazanopyrazines as potential kinase inhibitors are mentioned. Furazanopyrazine derivatives are known to be antimicrobials, antibacterials, herbicides and plant growth regulators (E. Fernandez et al., Tetrahedron Lett., 43, 2002, 4741-4745, GB 2122492, US 3850929). The synthesis of 5,6-disubstituted furazanopyrazines is described by different groups (I. B. Starchenkov, Chemistry of Heterocyclic Compounds, 33(10), 1997, 1219-1233, A. Gasco et al., Journal of Heterocyclic Chemistry, 6, 1969, 769-770).

The object of the present invention is solved by the teaching of the independent claims. Further advantageous features, aspects and details of the invention are evident from the dependent claims, the description, the figures, and the examples of the present application. Considering the lack of currently available treatment options for the majority of the conditions associated with protein kinases like ABL1, AKT1, AKT2, AKT3, Aurora-A, Aurora-B, Aurora-C, BRK, CDK1/CycB, CDK2/CycA,CDK2/CycE, CDK3/CycE, CDK4/CycD1, CDK5, CDK6/CycD1, CK2, COT, EGF-R, EPHA1, EPHB2, EPHB4, ERBB2, ERBB4, FAK, FGF-R1, FGF-R3, FGF-R4, FGR, FLT3, IGF1-R, IKK-beta, INS-R, IRAK4, JAK2, KIT, MET, MUSK, PBK, PCTAIRE1, PDGFR-alpha, PDGFR-beta, PIM1, PKC-alpha, PKC-beta1, PKC-beta2, PKC-delta, PKC-epsilon, PKC-eta, PKC-gamma, PKC-iota, PKC-mu, PKC-theta, PKC-zeta, PLK1, PRK1, RET, SGK3, SNK, S6K, SNK, SRC, SYK, TIE2, TSF1, TSK2, VEGF-R1, VEGF-R2, VEGF-R3, WEE1, especially with protein kinases like EGF-R (cell proliferation), Aurora-B (cell cycle control), IGF1-R (apoptosis), VEGF-R2 (angiogenesis) and SRC kinase (metastasis), there is still a great need for new therapeutic agents that inhibit these protein targets. Furazanopyrazine derivatives are a new group of protein kinase inhibitors which show differential inhibition of protein kinases, each of which can be assigned to one of the four molecular mechanisms of tumor development.

The present invention is also directed to novel compounds of the general formula (I) and pharmaceutically acceptable salts thereof: 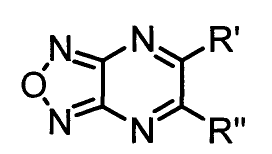 wherein:
R' represents -NR¹R² or -OR⁹
R" represents -NR⁵-NR³R⁴ , -NR⁵-OR^{b} -O-NR³R⁴;

R¹ represents hydrogen, C₁-C₆-alkyl, C₁-C₆-alkenyl, C₁-C₆-alkinyl, C₃-C₈-cycloalkyl, -COOR⁷, -SO₂R⁷, -CO-R⁹, -SO₂NHR⁹, -SO₂N(R⁹)₂, -OH, -SH, C₁-C₆-alkoxy, C₁-C₆-alkylthio, C₁-C₆-hydroxyalkyl, C₁-C₆-haloalkyloxy, C₅-C₁₅-aryl, heteroaryl or R¹ together with R⁵ form a 5- 6- or 7-membered unsaturated or saturated heterocyclic ring, which has optionally 1 to 3 substituents R⁸;
R² represents hydrogen, C₁-C₆-alkyl, C₁-C₆-alkenyl, C₁-C₆-alkinyl, C₃-C₈-cycloalkyl, -COOR⁷, -SO₂R⁷; -SO₂NHR⁹, -SO₂N(R⁹)₂, C₁-C₆-alkoxy, C₁-C₆-alkylthio, C₁-C₆-hydroxyalkyl, C₁-C₆-haloalkyloxy, C₅-C₁₅-aryl or heteroaryl;

R³ represents hydrogen, C₁-C₆-alkyl, C₁-C₆-alkenyl, C₁-C₆-alkinyl, C₃-C₈-cycloalkyl, -COOR⁷, -SO₂R⁷, -CO-R⁹, -SO₂NHR⁹, -SO₂N(R⁹)₂, -OH, -SH, C₁-C₆-alkoxy, C₁-C₆-alkylthio, C₁-C₆-hydroxyalkyl, C₁-C₆-haloalkyloxy, C₅-C₁₅-aryl heteroaryl, or
R³ represents a double bond to the carbonylic carbon atom of a mono- or oligosaccaride
and R⁴ is absent, or R³ represents 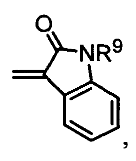 which has optionally 1 to 3 substituents R⁸
and R⁴ is absent or R³ represents 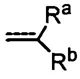 and is bonded to N via a single or double bond, and wherein
R^{a} and R^{b} are each independently hydrogen, C₁-C₆-alkyl, C₁-C₆-alkenyl, C₁-C₆-alkinyl, C₃-C₈-cycloalkyl, -COOR⁷, -SO₂R⁷, -CO-R⁹, -SO₂NHR⁹, -SO₂N(R⁹)₂, -OH, -SH, C₁-C₆-alkoxy, C₁-C₆-alkylthio, C₁-C₆-hydroxyalkyl, C₁-C₆-haloalkyloxy, C₅-C₁₅-aryl, heteroaryl, or R^{a} together with R⁴ form a 5-, 6- or 7-membered unsaturated or saturated heterocyclic ring, which has optionally 1 to 3 substituents R⁸,
or R^{a} together with R⁵ form a 5-, 6- or 7-membered unsaturated or saturated heterocyclic ring, which has optionally 1 to 3 substituents R⁸;

R⁴ represents hydrogen, C₁-C₆-alkyl, C₁-C₆-alkenyl, C₁-C₆-alkinyl, C₃-C₈-cycloalkyl, -COOR⁷, -SO₂R⁷, -SO₂NHR⁹, -SO₂N(R⁹)₂, C₁-C₆-alkoxy, C₁-C₆-alkylthio, C₁-C₆-hydroxyalkyl, C₁-C₆-haloalkyloxy, C₅-C₁₅-aryl,or heteroaryl or R⁴ together with R⁵ form a 5-, 6- or 7-membered unsaturated or saturated heterocyclic ring, which has optionally 1 to 3 substituents R⁸, or R⁴ represents a bond in case R⁴ and R^{a} form a ring system, and R⁴ is absent in case R³ is bonded to N via a double bond;
R⁵ represents hydrogen, C₁-C₆-alkyl, C₁-C₆-alkenyl, C₁-C₆-alkinyl, C₃-C₈-cycloalkyl, -COOR⁷, -SO₂R⁷, -SO₂NHR⁹, -SO₂N(R⁹)₂, C₁-C₆-alkoxy, C₁-C₆-alkylthio, C₁-C₆-hydroxyalkyl, C₁-C₆-haloalkyloxy, C₅-C₁₅-aryl, heteroaryl or R⁵ represents a bond in case R⁵and R⁴ or R⁵ and R¹ or R⁵ and R^{a} form a ring system;
R⁷ represents hydrogen, C₁-C₆-alkyl, C₁-C₆-alkenyl, C₁-C₆-alkinyl, C₃-C₈-cycloalkyl, C₅-C₁₅-aryl or heteroaryl;

The C₁-C₆-alkyl, C₃-C₈-cycloalkyl, C₅-C₁₅-aryl, and heteroaryl group can optionally be substituted by one or more substituents R⁸.

R⁸ represents independently of each other hydrogen, -COOR⁹, -CONHR⁹, -F, -Cl, -Br, -I, -CO-R⁹, -SO₂NR^{9'}R⁹, -NR⁹R^{9'}, -NR⁹-NR^{9'}, -O-CO-NR⁹R^{9'}, -NR⁹-CO-N⁹R^{9'}, -NO₂, -CN, -OH, -SH, -NR⁹-CO-(C₁-C₆)-haloalkyl, -NR⁹-SO₂-(C₁-C₆)-haloalkyl, C₁-C₆-alkyl, C₁-C₆-aminoalkyl, C₁-C₆-alkoxy, C₁-C₆-alkylthio, C₁-C₆-hydroxyalkylamino, C₁-C₆-hydroxyalkyl, amine, C₁-C₆-haloalkyloxy, C₅-C₁₅-aryl or heteroaryl;

R^{8'} represents independently of each other hydrogen, -COOR⁹, -CONHR⁹, -F, -Cl, -Br, -I, -CO-R⁹, -SO₂NR^{9'}R⁹, -NR⁹R^{9'}, -NR⁹-NR^{9'}, -O-CO-NR⁹R^{9'}, -NR⁹-CO-N⁹R^{9'}, -NR⁹-CO-(C₁-C₆)-haloalkyl, -NO₂, -CN, -NR⁹-SO₂-(C₁-C₆)-haloalkyl, C₁-C₆-alkyl, C₁-C₆-aminoalkyl, -OH, -SH, C₁-C₆-alkoxy, C₁-C₆-alkylthio, C₁-C₆-hydroxyalkylamino, C₁-C₆-hydroxyalkyl, amine, C₁-C₆-haloalkyloxy, C₅-C₁₅-aryl or heteroaryl;

R⁹ represents hydrogen, C₁-C₆-hydroxyalkyl, C₁-C₆-alkyl, C₁-C₆-aminoalkyl, C₅-C₁₅-aryl or heteroaryl;

R^{9'} represents hydrogen, C₁-C₆-hydroxyalkyl, C₁-C₆-alkyl, C₁-C₆-aminoalkyl, C₅-C₁₅-aryl or heteroaryl.

An alkyl group denotes a C₁-C₆-alkyl, C₁-C₆-alkenyl, C₁-C₆-alkinyl, or C₃-C₈-cycloalkyl residue.
The C₁-C₆-alkyl, C₁-C₆-alkenyl, C₁-C₆-alkinyl, and C₃-C₈-cycloalkyl residue may be selected from the group comprising -CH₃, -C₂H₅, -CH=CH₂, -C≡CH, -C₃H₇, -cyclo-C₃H₅, -CH(CH₃)₂, -CH₂-CH=CH₂, -C(CH₃)=CH₂, -CH=CH-CH₃, -C≡C-CH₃, -CH₂-C≡CH, -C₄H₉, -cyclo-C₄H₇, -CH₂-CH(CH₃)₂, -CH(CH₃)-C₂H₅,-C(CH₃)₃, -C₅H₁₁, -cyclo-C₅H₉, -C₆H₁₃, -cyclo-C₆H₁₁, -C(R¹⁰)₃, -CR¹⁰(R^{10'})₂, -CR¹⁰(R^{10'})R^{10''}, -C₂(R¹⁰)₅, -CH₂-C(R¹⁰)₃, -CH₂-CR¹⁰(R^{10'})₂, -CH₂-CR¹⁰(R^{10'})R^{10''},-C₃(R¹⁰)₇, -C₂H₄-C(R¹⁰)₃, -C₂H₄-CH=CH₂, -CH=CH-C₂H₅, -CH=C(CH₃)₂, -CH₂-CH=CH-CH₃, -CH=CH-CH=CH₂, -C₂H₄-C≡CH, -C≡C-C₂H₅, -CH₂-C≡C-CH₃,-C≡C-CH=CH₂, -CH=CH-C≡CH, -C≡C-C≡CH, -C₂H₄-CH(CH₃)₂, -CH(CH₃)-C₃H₇,-CH₂-CH(CH₃)-C₂H₅, -CH(CH₃)-CH(CH₃)₂, -C(CH₃)₂-C₂H₅, -CH₂-C(CH₃)₃, -C₃H₆-CH=CH₂, -CH=CH-C₃H₇, -C₂H₄-CH=CH-CH₃, -CH₂-CH=CH-C₂H₅, -CH₂-CH=CH-CH=CH₂, -CH=CH-CH=CH-CH₃, -CH=CH-CH₂-CH=CH₂, -C(CH₃)=CH-CH=CH₂,-CH=C(CH₃)-CH=CH₂, -CH=CH-C(CH₃)=CH₂, -CH₂-CH=C(CH₃)₂, -C(CH₃)=C(CH₃)₂, -C₃H₆-C≡CH, -C≡C-C₃H₇, -C₂H₄-C≡C-CH₃,
-CH₂-C≡C-C₂H₅, -CH₂-C≡C-CH=CH₂, -CH₂-CH=CH-C≡CH, -CH₂-C≡C-C≡CH,-C≡C-CH=CH-CH₃, -CH=CH-C≡C-CH₃, -C≡C-C≡C-CH₃, -C≡C-CH₂-CH=CH₂,-CH=CH-CH₂-C≡CH, -C≡C-CH₂-C≡CH, -C(CH₃)=CH-CH=CH₂, -CH=C(CH₃)-CH=CH₂, -CH=CH-C(CH₃)=CH₂, -C(CH₃)=CH-C≡CH, -CH=C(CH₃)-C≡CH, -C≡C-C(CH₃)=CH₂, -C₃H₆-CH(CH₃)₂, -C₂H₄-CH(CH₃)-C₂H₅,-CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -C₄H₈-CH=CH₂, -CH=CH-C₄H₉, -C₃H₆-CH=CH-CH₃, -CH₂-CH=CH-C₃H₇, -C₂H₄-CH=CH-C₂H₅, -CH₂-C(CH₃)-C(CH₃)₂, -C₂H₄-CH=C(CH₃)₂, -C₄H₈-C≡CH,-C≡C-C₄H₉, -C₃H₆-C≡C-CH₃, -CH₂-C≡C-C₃H₇, -C₂H₄-C≡C-C₂H₅, -cyclo-C₇H₁₃, -cyclo-C₈H₁₅ and R¹⁰, R^{10'} and R^{10"} represent independently of each other -H, -F, -Cl, -Br, or -1.
an alkoxy group denotes an O-alkyl group, the alkyl group being as defined above;
an alkylthio group denotes an S-alkyl group, the alkyl group being as defined above.
an haloalkyl group denotes an alkyl group which is substituted by one to five halogen atoms, the alkyl group being as defined above;
a hydroxyalkyl group denotes an HO-alkyl group, the alkyl group being as defined above;
an haloalkyloxy group denotes an alkoxy group which is substituted by one to five halogen atoms, the alkyl group being as defined above;
a hydroxyalkyl group denotes an HO-alkyl- group, the alkyl group being as defined above;
a hydroxyalkylamino group denotes an (HO-alkyl)₂-N- group or HO-alkyl-NH- group, the alkyl group being as defined above;
an alkylamino group denotes an HN-alkyl or N-dialkyl group, the alkyl group being as defined above;
a halogen group is chlorine, bromine, fluorine or iodine, fluorine being preferred;
a mono- or oligosaccharide, which is bonded through the double bond of the carbonyl to the nitrogen atom, wherein one or more of the OH groups could be protected by an acetyl or benzyl group, is preferably a monosaccharides like glucose, galactose, fructose, fucose, ribose, glucosamine, N-acteylglucosamine, galactoseamine, N-acetylgalactoseamine or mannose;
a 5-, 6-, 7- or 8-membered unsaturated or saturated heterocyclic ring, which is formed between R⁴ and R⁵ are for example:

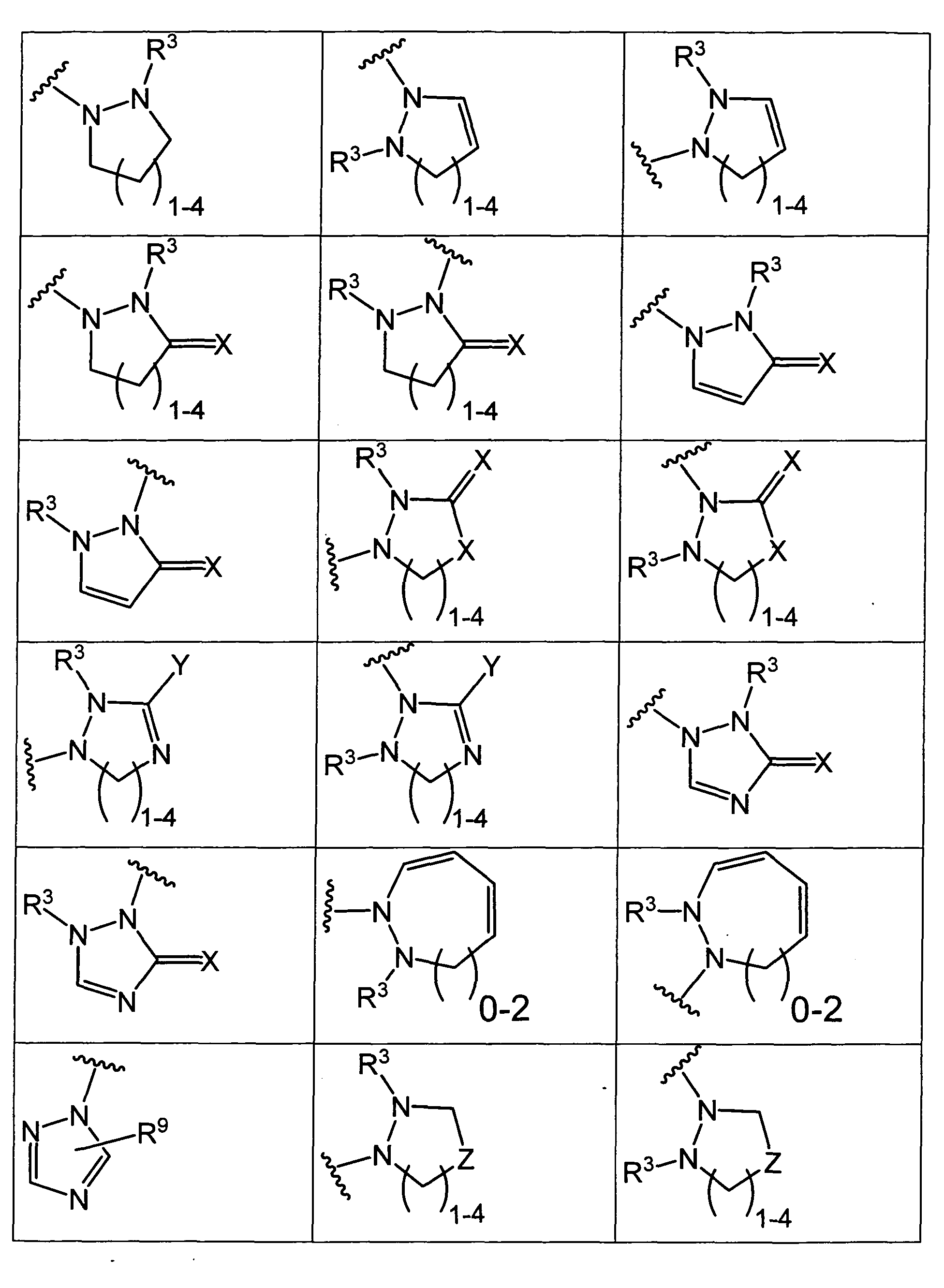

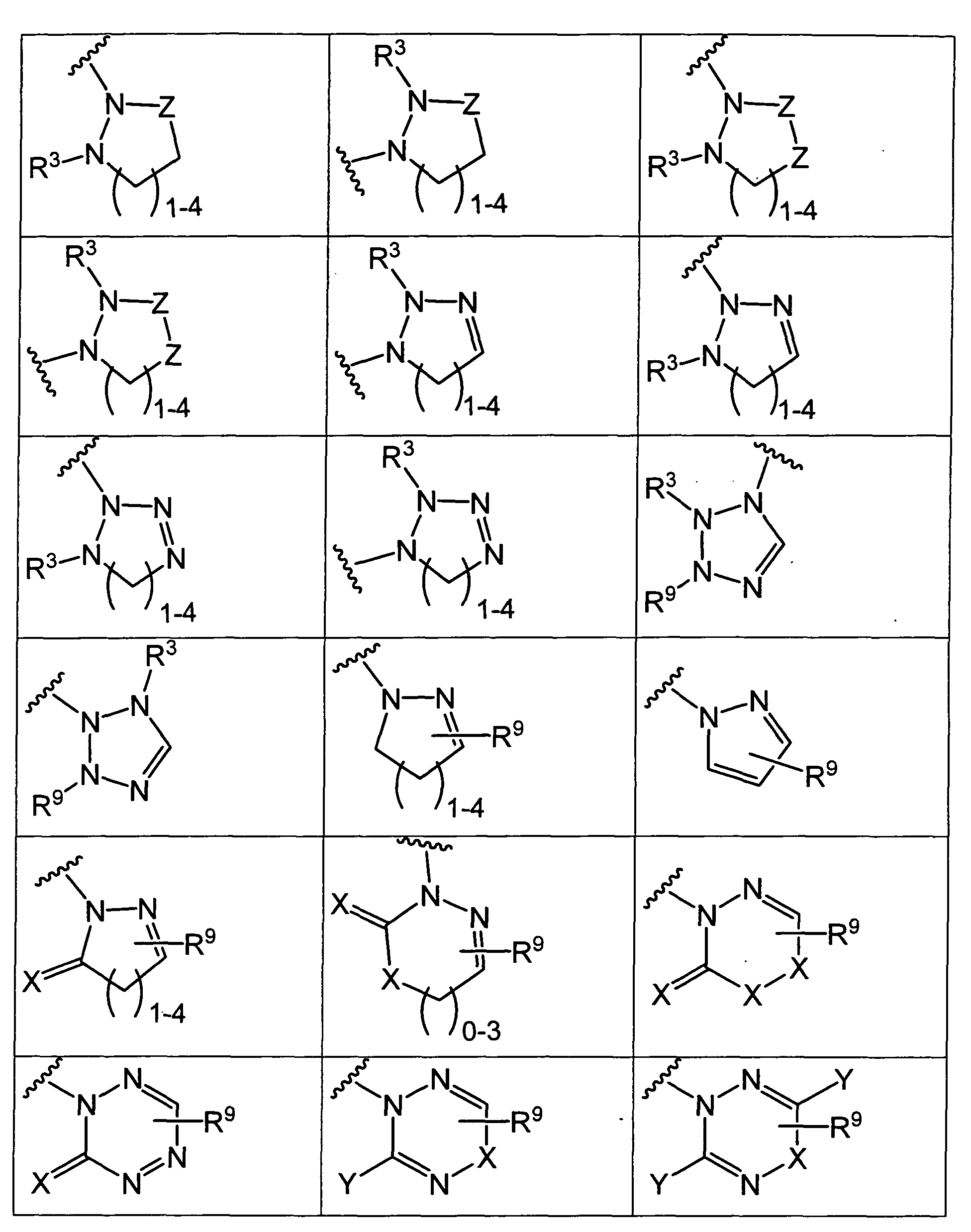

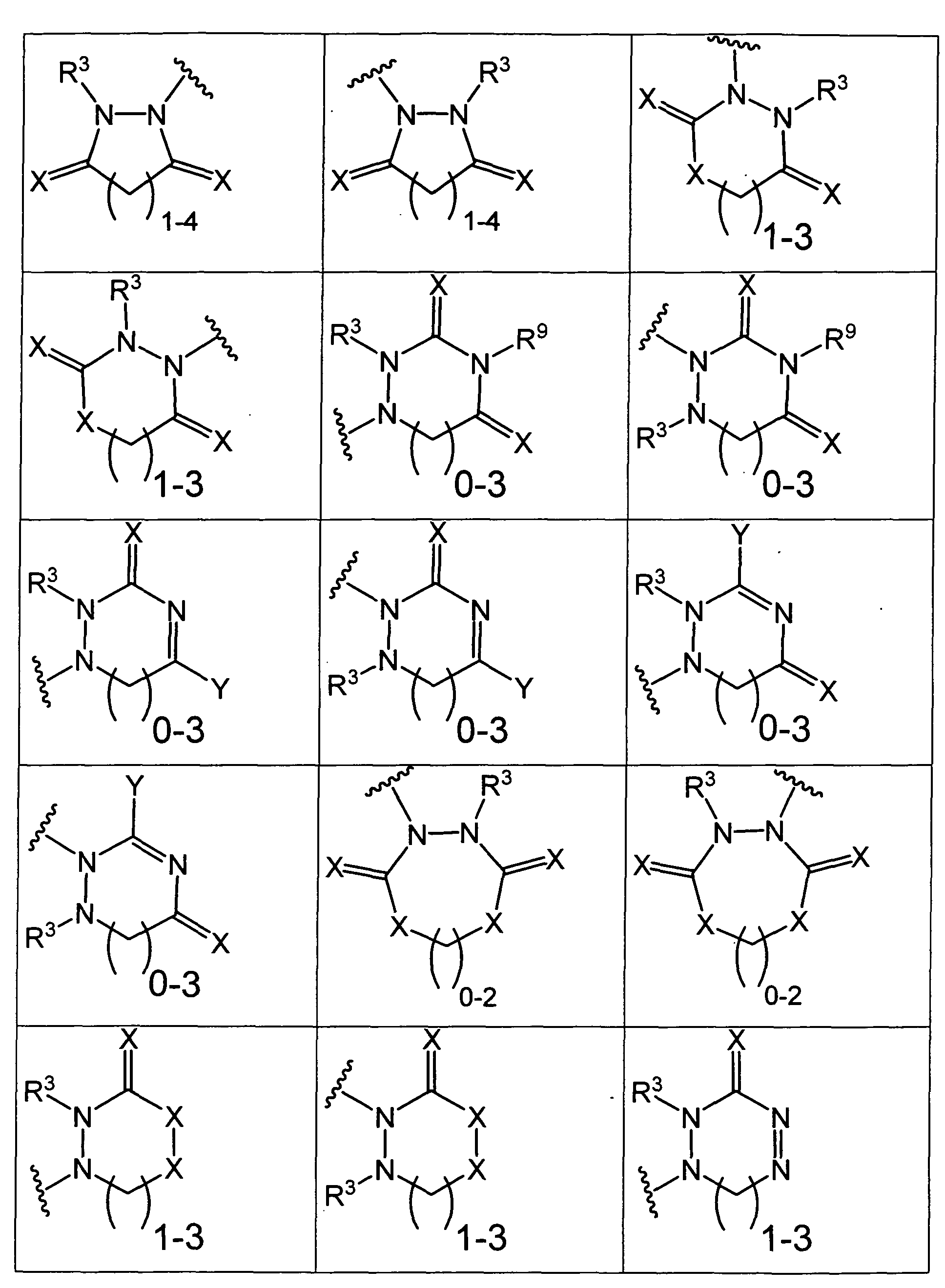

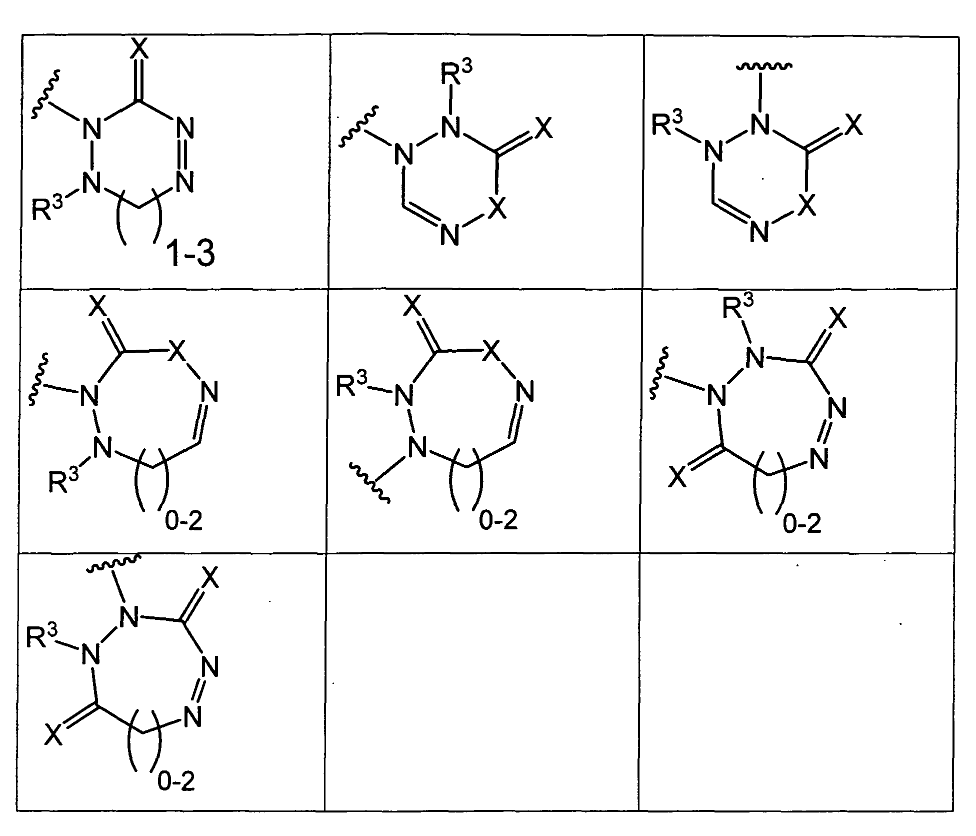

or tautomers thereof
wherein X represents O, S or NR⁹; Y represents OR⁹, SR⁹ or NR⁹R^{9'}; Z represents CR⁸R^{8'}, SO, SO₂, O, S or NR⁹.

The C₅-C₁₅-aryl represents -Ph, -CH₂Ph, -C₂H₄Ph, -CH=CH-Ph, -C≡C-Ph, -o-C₆H₄-R⁸, -m-C₆H₄-R⁸, -P-C₆H₄-R⁸, -o-CH₂-C₆H₄-R⁸, -m-CH₂-C₆H₄-R⁸, -p-CH₂-C₆H₄-R⁸, wherein R⁸ is as defined above or 1-naphthyl, 2-naphthyl, 1-anthracyl, 2-anthracyl optionally substituted by one or more R⁸ , which is as defined above.

A heteroaryl group represents a 5- or 6-membered heterocyclic group which contains at least one heteroatom like O, N, or S. This heterocyclic group can be fused to another ring. For example, this group can be selected from a thiazole-2-yl, thiazole-4-yl, thiazole-5-yl, isothiazole-3-yl, isothiazole-4-yl, isothiazole-5-yl, 1,2,4-oxadiazole-3-yl, 1,2,4-oxadiazole-5-yl, 1,2,4-thiadiazole-3-yl, 1,2,4-thiadiazole-5-yl, 1,2,5-oxadiazole-3-yl, 1,2,5-oxadiazole-4-yl, 1,2,5-thiadiazole-3-yl, 1-imidazolyl, 2-imidazolyl, 1,2,5-thiadiazol-4-yl, 4-imidazolyl, 1-pyrrolyl, 2-pyrrolyl, 3-pyrrolyl, 1-pyrrolididnyl, 2-pyrrolidinyl, 3-pyrrolidinyl, 2-furanyl, 3-furanyl, 2-thienyl, 3-thienyl, 2-pyridyl, 3-pyridyl, 4-pyridyl, 2-pyrimidyl, 4-pyrimidyl, 5-pyrimidyl, 2-pyrimidinyl, 4-pyrimidinyl, 5-pyrimidinyl, 3-pyridazinyl, 4-pyridazinyl, 2-pyrazinyl, 1-pyrazolyl, 3-pyrazolyl, 4-pyrazolyl, indolyl, isoindolyl, indolinyl, benzofuranyl, benzo[b]thiophenyl, benzo[b]thienyl, benzooxazolyl, benzoisoxazolyl, benzotriazolyl, benzimidazolyl, benzothiazolyl, quinazolinyl, quinoxazolinyl, oxazole-2-yl, oxazole-4-yl, oxazole-5-yl, oxadizole-2-yl, oxadiazole-5-yl, isoxazole-3-yl, isoxazole-4-yl, isoxazole-5-yl, acridinyl, tetrazol-5-yl, triazol-2-yl, carbazolyl, piperidin-1-yl, piperidin-2-yl, piperidin-3-yl, piperidin-4-yl, quinolinyl, tetrahydroquinolinyl, isoquinolinyl, tetrahydroisoquinolinyl, pyrido[3,4-d]pyrimidinyl, 5-phenyl-furan-2-yl, 5-phenyl-furan-3-yl, 4-phenyl-furan-2-yl, 4-phenyl-furan-3-yl group. This heterocyclic group can optionally be substituted by one or more substituents R⁸, where R⁸ is as defined above.

Beside these residues the C₅-C₁₅-aryl, C₃-C₈-cycloalkyl and heteroaryl represent independently of each other 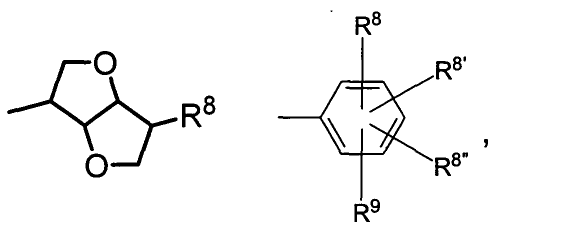 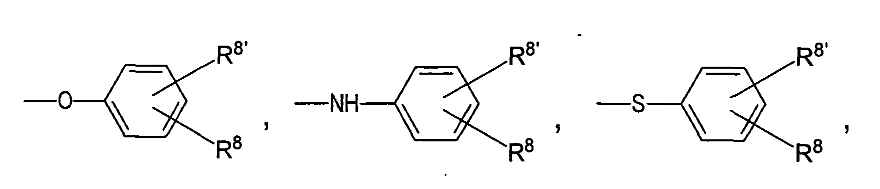 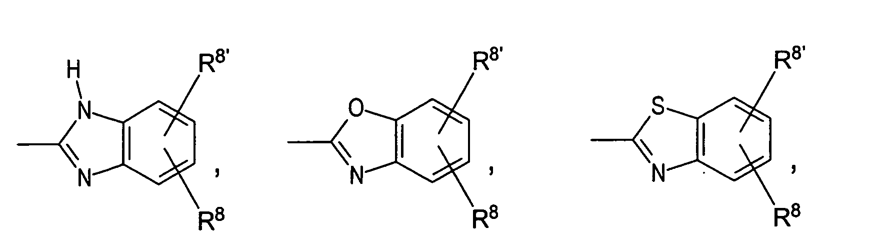 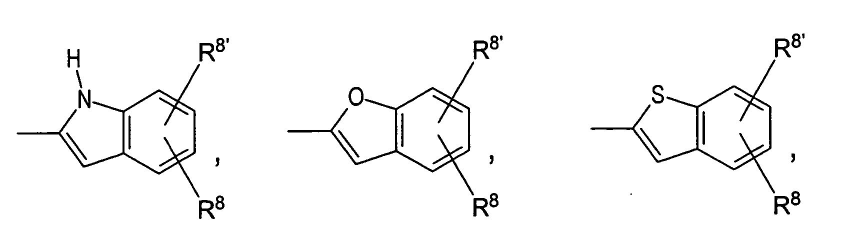 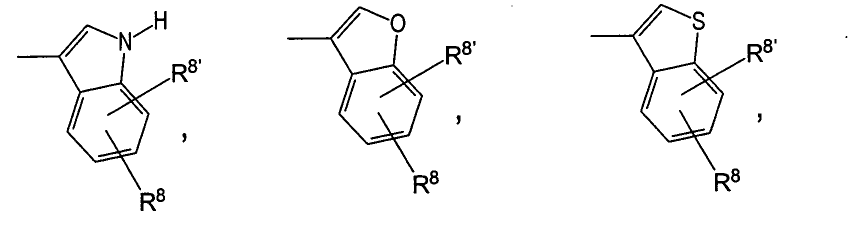 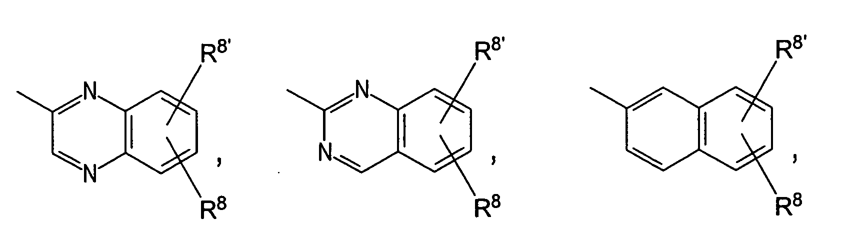 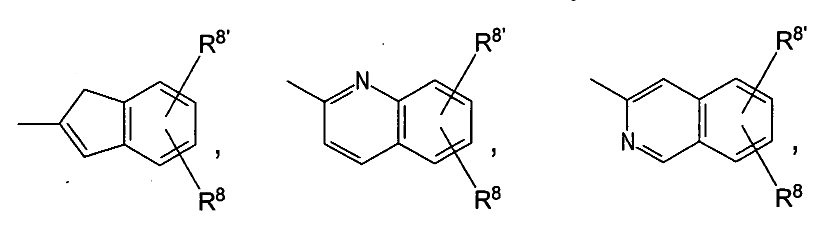 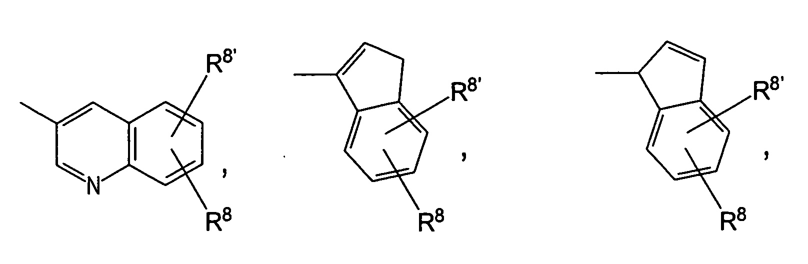 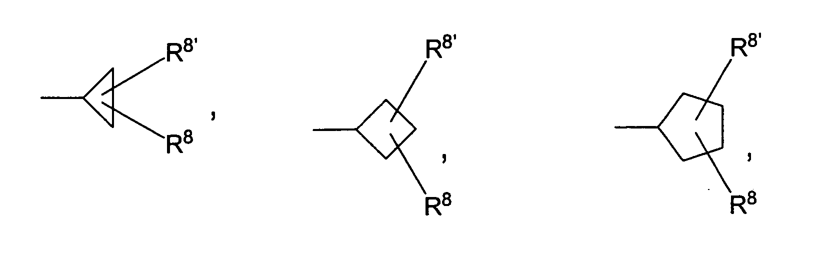 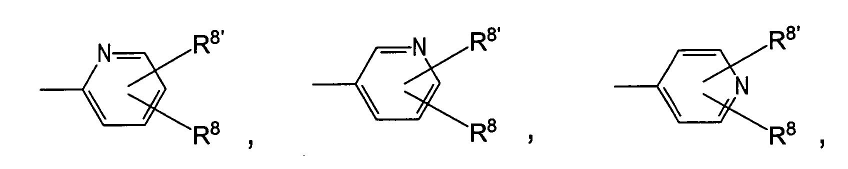 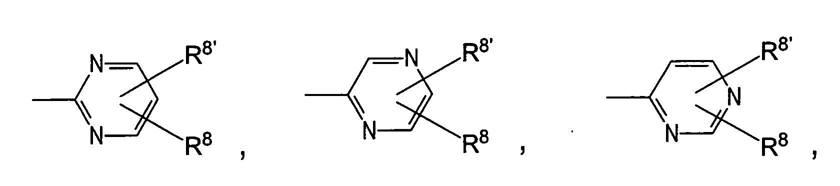 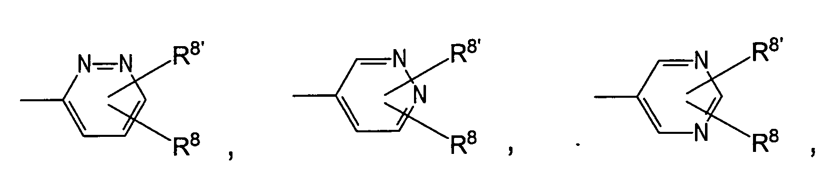 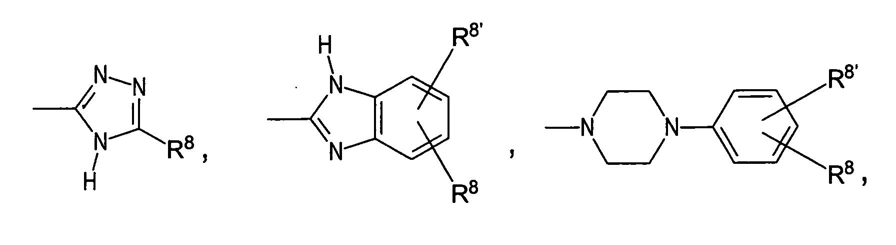 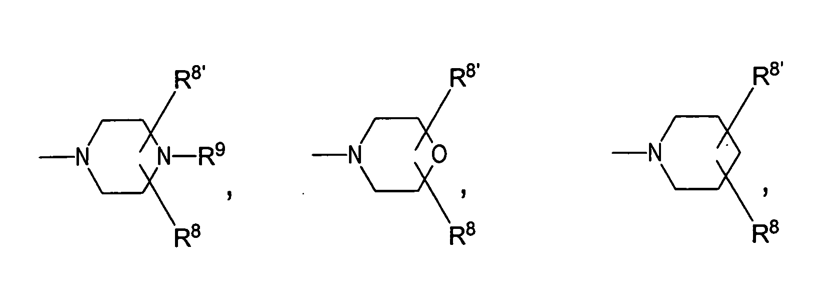 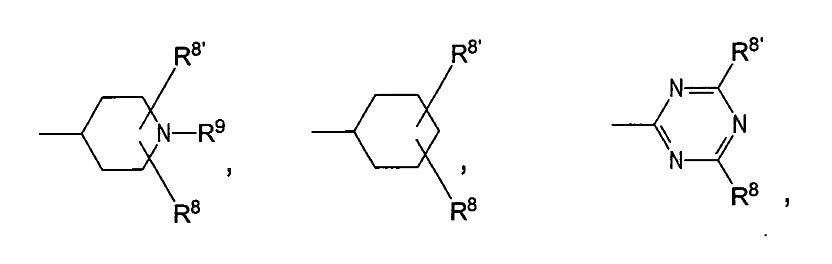 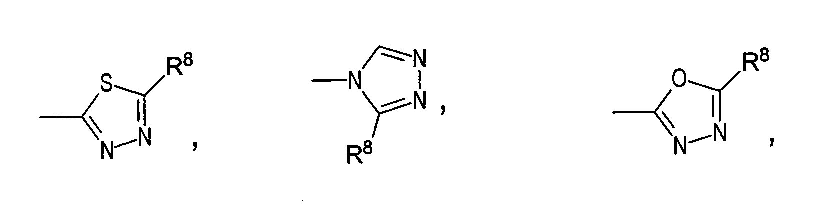 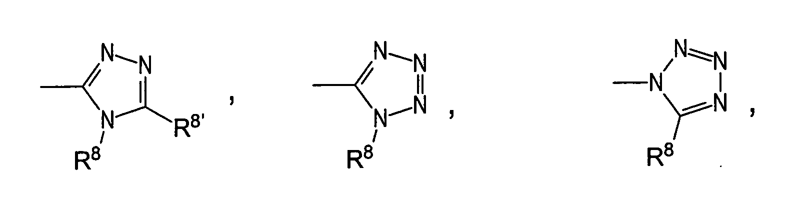 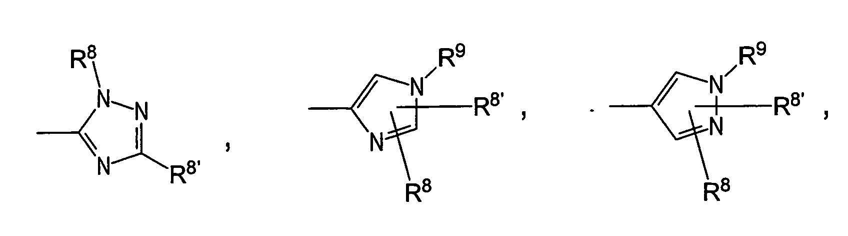 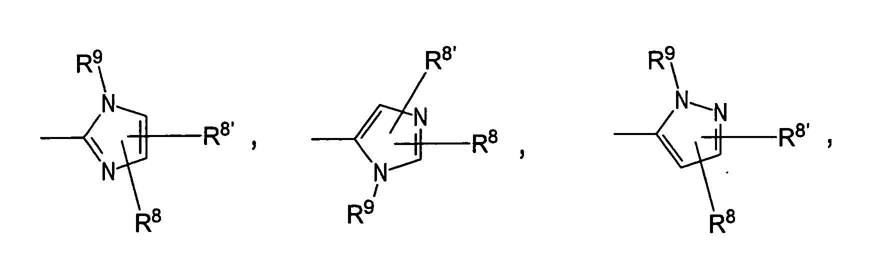 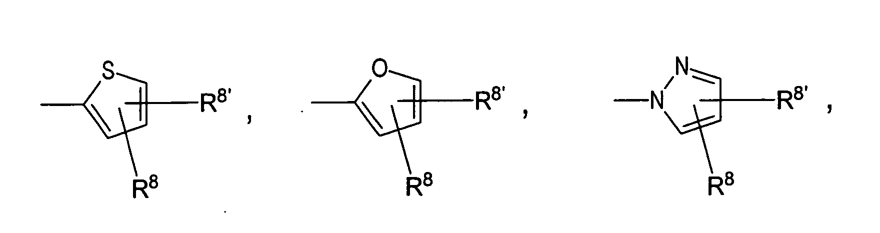 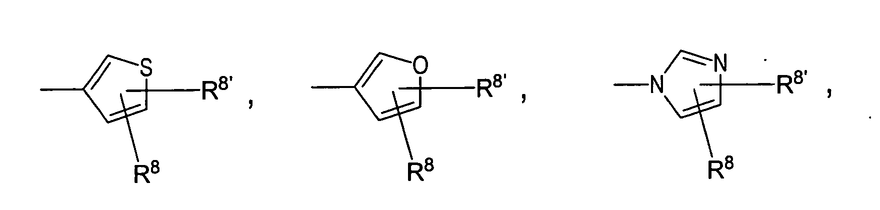 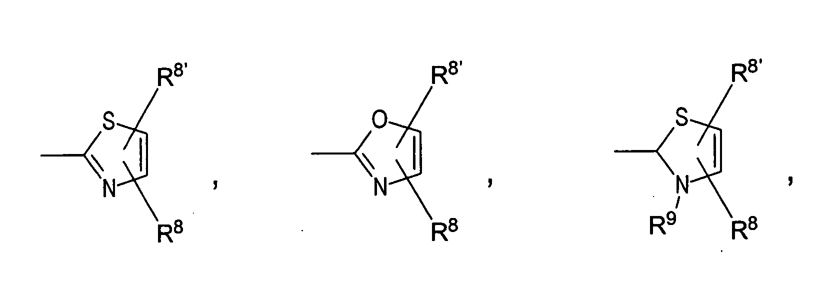 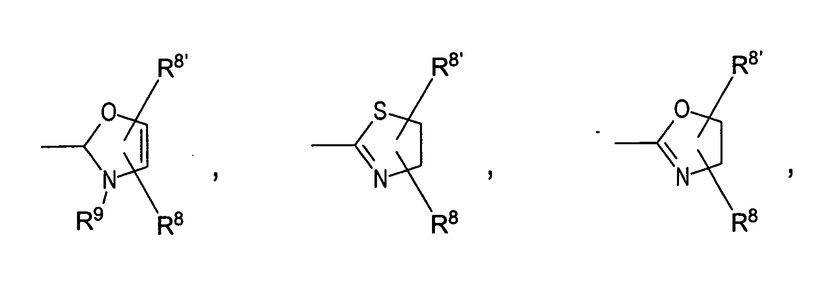 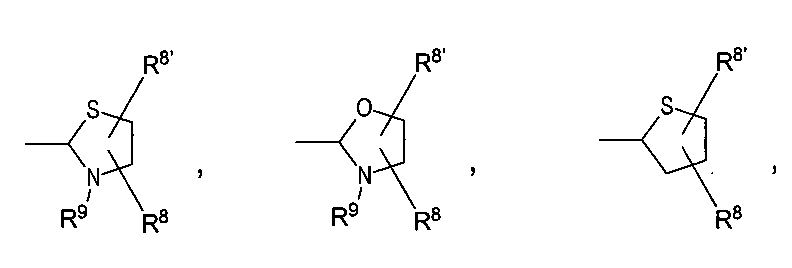 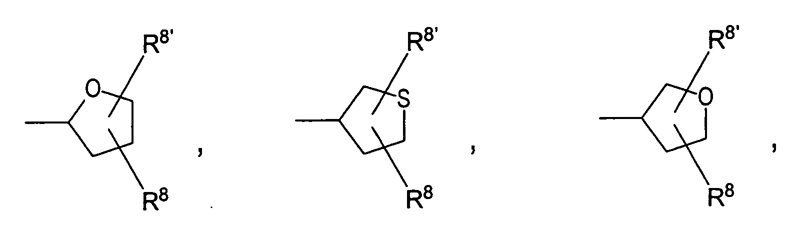 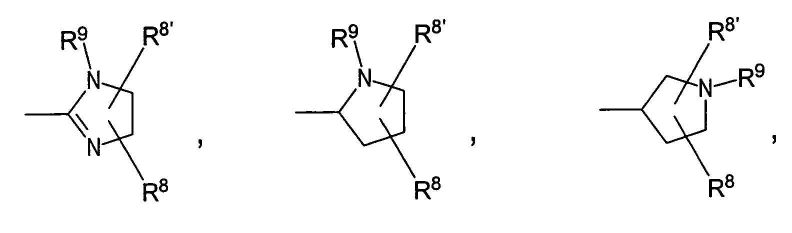 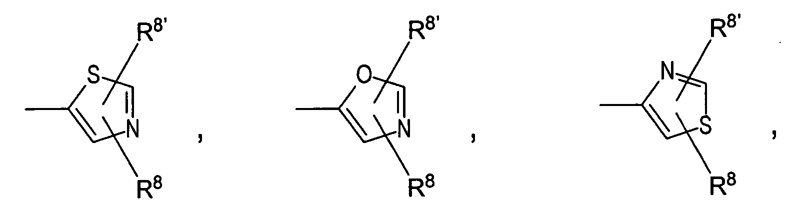 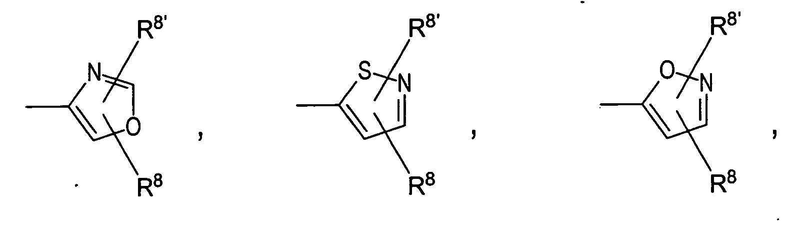 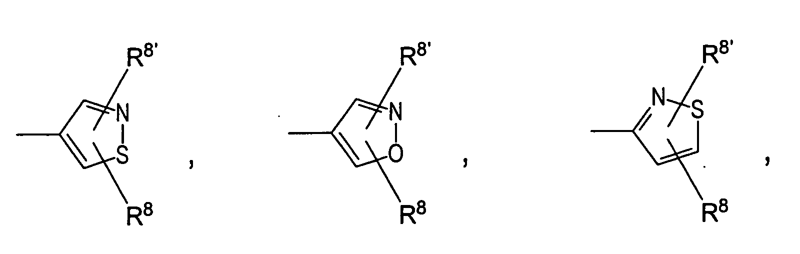 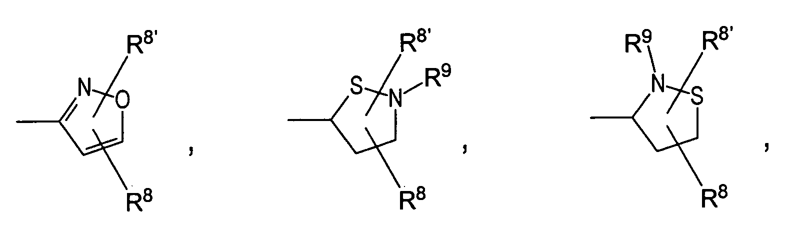 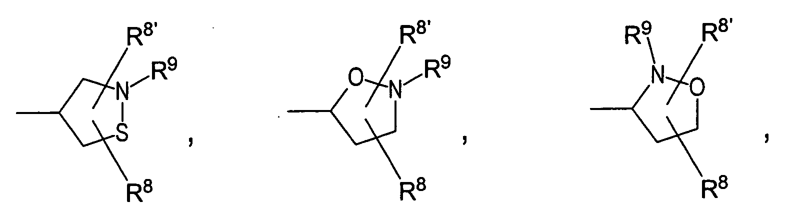 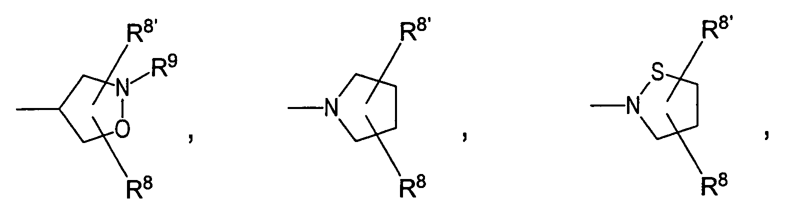 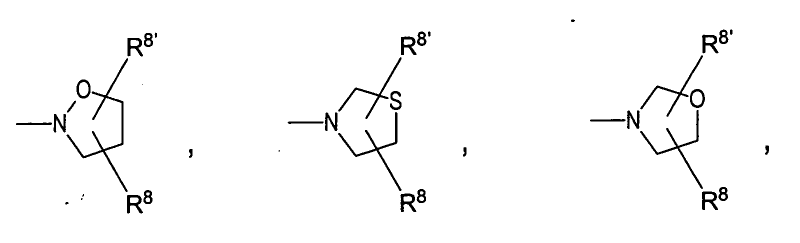 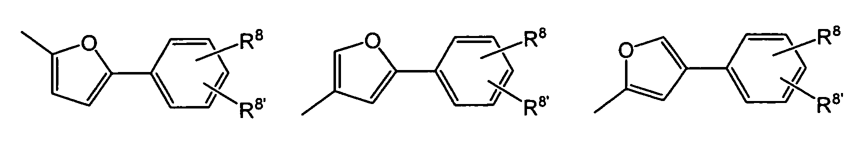 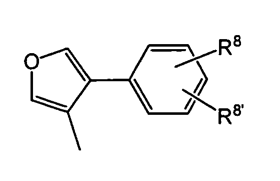 wherein R⁸, R^{8'} and R⁹ have the meanings as defined above; and R^{8"} represents independently of each other hydrogen, -COOR⁹, -CONHR⁹, -F, -Cl, -Br, -I, -NO₂,-CO-R⁹, -SO₂NHR⁹, -SO₂N(R⁹)₂, -NR⁹-CO-(C₁-C₆)-haloalkyl, -OH, -SH, -CN,-NR⁹-SO₂-(C₁-C₆)-haloalkyl, C₁-C₆-alkyl, C₁-C₆-aminoalkyl, C₁-C₆-alkoxy, C₁-C₆-alkylthio, C₁-C₆-hydroxyalkylamino, C₁-C₆-haloalkyloxy, C₅-C₁₅-aryl or heteroaryl.

In a preferred embodiment of the invention, in compounds of formula (I), the alkoxy group is preferably a methoxy, ethoxy, isopropoxy, t-butoxy or pentoxy group;

In a further particularly preferred embodiment of the invention, in compounds of formula (I), R' is NR¹R².

In a further particularly preferred embodiment of the invention, in compounds of formula (I), R" is NR⁵-NR³R⁴.

In a further particularly preferred embodiment of the invention, in compounds of formula (I), R¹, R⁴ or R⁵ are independently of each other are hydrogen or methyl.

In a further preferred embodiment of the invention, in compounds of formula (I), R² is aryl or heteroaryl more preferred R² is an optionally substituted phenyl.

In a further preferred embodiment of the invention, in compounds of formula (I), R² is phenyl substituted with one or two halogen groups.

In a further preferred embodiment of the invention, in compounds of formula (I), R² is phenyl substituted with a halogen group, preferably substituted in meta or para position to the amine.

In a further preferred embodiment of the invention, in compounds of formula (I), R² is phenyl substituted with two halogen groups, preferably substituted in 3,4- position to the amine.

In a further preferred embodiment of the invention, in compounds of formula (I), R⁴ is aryl or heteroaryl more preferred R⁴ is an optionally substituted phenyl.

In a further particularly preferred embodiment of the invention, in compounds of formula (I), R³ is 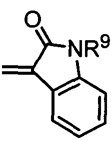 or

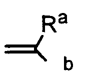

In a further preferred embodiment of the invention, in compounds of formula (1), R^{b} is aryl or heteroaryl, more preferred R^{b} is an optionally by R⁸ substituted phenyl, an optionally by R⁸ substituted 5-phenyl-furan-2-yl, an optionally by R⁸ substituted furan-2-yl, or an optionally by R⁸ substituted benzofuranoyl.

In a further preferred embodiment of the invention, in compounds of formula (I), R^{b} is phenyl substituted with one or two or three hydroxy groups.

In a further preferred embodiment of the invention, in compounds of formula (I), R^{b} is phenyl substituted with a hydroxy group in ortho position to the amino group.

In a further preferred embodiment of the invention, in compounds of formula (I), R^{b} is phenyl substituted with a hydroxy group and one or two halogen groups.

In a further preferred embodiment of the invention, in compounds of formula (1), R^{b} is phenyl substituted with a hydroxy group in ortho position to the amino group and one or two halogen groups.

In a further preferred embodiment of the invention, in compounds of formula (1), R^{b} is phenyl substituted with a hydroxy group, a methoxy group and a halogen group.

In a further preferred embodiment of the invention, in compounds of formula (I), R^{b} is phenyl substituted with a hydroxy group in ortho position to the amino group and a halogen group and a methoxy group.

In a further preferred embodiment of the invention, in compounds of formula (I), R^{b} is phenyl substituted with one or more methoxy groups.

In a further preferred embodiment of the invention, in compounds of formula (I), R^{b} is phenyl substituted with a methoxy group in meta or para position to the amino group.

In a further preferred embodiment of the invention, in compounds of formula (I), R^{b} is phenyl substituted with a methoxy group in meta or para position to the amino group and a hydroxy group in ortho position to the amino group.

In a further preferred embodiment of the invention, in compounds of formula (1), R^{b} is phenyl substituted with a methoxy group in meta or para position to the amino group and a hydroxy group in ortho position to the amino group and a halogen group.

In a further preferred embodiment of the invention, in compounds of formula (I), R^{b} is phenyl substituted with one or more caboxyl groups.

In a further preferred embodiment of the invention, in compounds of formula (I), R^{b} is phenyl substituted with a carboxyl group in para position to the amino group.

In a further preferred embodiment of the invention, in compounds of formula (I), R^{b} is 5-phenyl-furan-2-yl substituted by a carboxyl, an amide or a nitro group.

In a further preferred embodiment of the invention, in compounds of formula (I), R^{b} is 5-phenyl-furan-2-yl substituted by a carboxyl and a halogen group.

In a further preferred embodiment of the invention, in compounds of formula (I), R^{b} is 5-phenyl-furan-2-yl substituted by a carboxyl and a hydroxy group.

In a further preferred embodiment of the invention, in compounds of formula (I), R^{b} is 5-phenyl-furan-2-yl substituted by a carboxyl and a methoxy group.

One possibility for the synthesis of compounds of formula (I), wherein R' is =NR¹R² and R" is -NR⁵-NR³R⁴ comprises the step of reacting a compound of formula (II) with a compound of formula (III). This reaction is described for example in Starchenkov, I. B.; Andrianov, V. G. *Chem. Heterocycl. Compd.* 1997, *33*, 1219-1233; *Khim. Geterotsikl. Soedin.* 1997, 1402-1416.

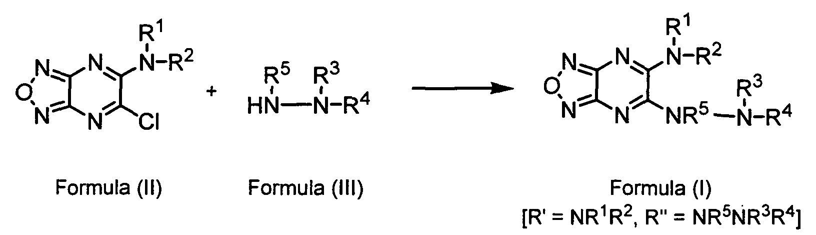

One possibility for the synthesis of compounds of formula (II) comprises the step of reacting 5,6-dichloro-[1,2,5]oxadiazolo[3,4-b]pyrazine with a compound of formula (IV). This reaction is described for example in Starchenkov, I. B.; Andrianov, V. G. *Chem. Heterocycl. Compd.* 1997, *33*, 1219-1233; *Khim. Geterotsikl. Soedin.* 1997, 1402-1416, and in Fernández, E.; García-Ochoa, S.; Huss, S.; Mallo, A.; Bueno, J. M.; Micheli, F.; Paio, A.; Piga, E.; Zarantello, P. *Tetrahedron Lett.* 2002, *43*, 4741-4745.

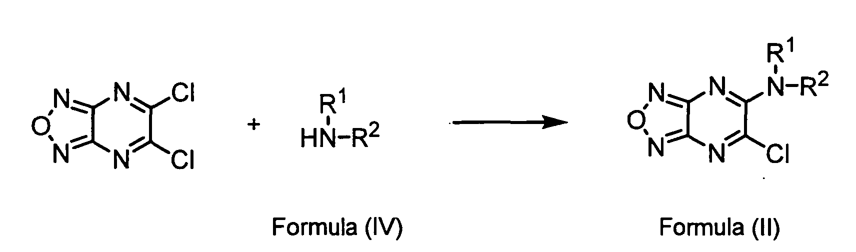

Alternatively, 5,6-dichloro-[1,2,5]oxadiazolo[3,4-b]pyrazine can be first reacted with a compound of formula (III) and then with a compound of formula (IV).

One possibility for the synthesis of derivatives of compounds of formula (I), wherein R' is -NR¹R² and R" is -NR⁵-N=CR^{a}R^{b} comprises the step of reacting a compound of formula (I), wherein R' is -NR¹R² and R" is -NR⁵-NH₂, with a compound of formula (V). Instead of a compound of formula (V), synthetic equivalents thereof such as acetals, ketals, imines can also be used. Such reactions are described for example in: Duni 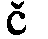 , M.; Korun 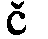 ev, D.; Kova 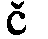 evi 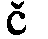 , K.; Polak, L. In *Methoden der Organischen Chemie* (Houben-Weyl), Vol. E14b, Teil 1, Georg-Thieme-Verlag, Stuttgart, New York, **1990**, pp. 461-506, and literature cited herein. These furazanopyrazines of formula (I) can be separated by precipitation from the reaction mixture, or by chromatography, for example by HPLC chromatography. The synthesis includes that the terminal amino group of compounds of formula (I), wherein R' is -NR¹R² and R" is -NR⁵-NH₂, or salts thereof, can be protected by one or two of the usual protecting groups like *tert*-butyloxycarbonyl (Boc), benzyloxycarbonyl (Cbz, Z), 2,2,2-trichloro-ethoxycarbonyl (Troc), carbamates, cyclic imides and other groups well known to those skilled in the art. Further protecting groups are also described in Greene, T. W.; Wuts, P. G. M. "Protective Groups in Organic Synthesis", 3^{rd} edition, John Wiley & Sons, New York, 1999, and in Kocienski, P. J., "Protecting Groups", 2^{nd} edition, Thieme Verlag, Stuttgart, 2000.

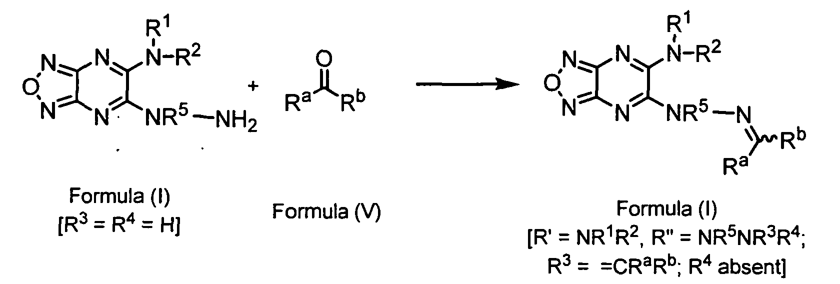

Compounds of formula (I) can also be prepared by reacting 5,6-dichloro-[1,2,5]oxadiazolo[3,4-b]pyrazine with a compound of formula (III) and subsequently with other nucleophiles such as alcohols, thiols and the like, or vice versa. Examples are described in Starchenkov, I. B.; Andrianov, V. G. *Chem. Heterocycl. Compd* 1997, *33*, 1219-1233; *Khim. Geterotsikl. Soedin.* 1997, 1402-1416.

The starting material, 5,6-dichloro-[1,2,5]oxadiazolo[3,4-b]pyrazine can be prepared from [1,2,5]oxadiazolo[3,4-b]pyrazine-5,6-diol, for example by applying a mixture of phosphorus pentachloride and phosphorus oxychloride. This step is for example described in Fernandez, E.; García-Ochoa, S.; Huss, S.; Mallo, A.; Bueno, J. M.; Micheli, F.; Paio, A.; Piga, E.; Zarantonello, P. *Tetrahedron Lett.* 2002*, 43,* 4741-4745*,* or in Starchenkov, I. B.; Andrianov, V. G.; *Chem. Heterocycl. Compd* 1997, *33*, 1219-1233; *Khim. Geterotsikl. Soedin.* 1997, 1402-1416. 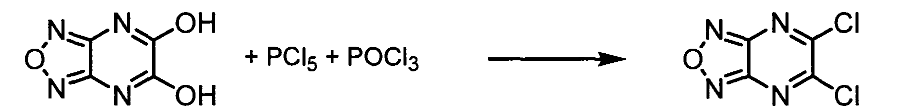 Instead the mixture of phosphorus pentachloride and phosphorus oxychloride thionylchloride can be used.
[1,2,5]oxadiazolo[3,4-b]pyrazine-5,6-diol is commercially available. Alternatively, this compound can be prepared according to Gasco, A.; Ruá, G.; Menziani, E.; Nano, G. M.; Tappi, G. *J. Heterocycl. Chem.* 1969, *6*, 769-770.

One method for the synthesis of compounds of formula (V), wherein R^{a} is 5-arylfuryl and R^{b} = H comprises the step of reacting the diazonium salt of an arylamine with substituted or unsubstituted furfural. This step is for example described in Burch, H. A.; White, R. E.; Wright, G. C.; Goldenberg, M. M. *J. Pharm. Sci.* **1980**, *69,* 107-110, in Pong, S. F.; Pelosi, S. S.; Wessels, F. L.; Yu, C.-N.; Bums, R. H.; White, R. E.; Anthony, Jr., D. R.; Ellis, K. O.; Wright, G. C.; White, Jr., R. L. *Arzneim.-Forsch.*/*Drug Res.* **1983**, *33(II),* 1411-1416, and in Belagali, S. L.; Kumar, K. H.; Boja, P.; Himaja, M. *Ind. J Chem.* **1998**, *37b,* 370-375.
Another method for the synthesis of compounds of formula (V), wherein R^{a} is 5-arylfuryl and R^{b} = H represents the cross coupling reaction of boronic acid derivatives with substituted or unsubstituted 5-halogenofurfurals. This reaction is described for example in Feuerstein, M.; Doucet, H.; Santelli, M. *Tetrahedron Lett.* **2001**, *42,* 5659-5662.
Another method for the synthesis of compounds of formula (V), wherein R^{a} is 5-arylfuryl and R^{b} = H represents the cross coupling reaction of an aryl halogenide with a 5-formylfuran-2-boronic acid derivative. This reaction is for example described in McClure, M. S.; Roschangar, F.; Hodson, S. J.; Millar, A.; Osterhout, M. H. *Synthesis* **2001,** 1681-1685.
Another method for the synthesis of compounds of formula (V), wherein R^{a} is 5-arylfuryl and R^{b} = H represents the palladium-catalyzed reaction of an aryl halogenide with furfural. This reaction is for example described in McClure, M. S.; Glover, B., McSorley, E., Millar, A., Osterhout, M. H., and Roschangar, F. *Org. Lett.* **2001,** *3*, 1677-1680.

Other aspects of the present invention relate to furazanopyrazine derivatives of the general formula (I) as new pharmaceutically active agents, especially for the preparation of a pharmaceutical composition for the treatment of diseases which are cured or relieved by the inhibition of one or several kinases and/or phosphatases. The compounds of the general formula (I) were surprisingly identified as potent inhibitors.

Another method is directed to the prophylaxis and/or treatment of infectious diseases, including opportunistic infections in a mammal, including a human. Said method comprises administering to the mammal an amount of at least one compound of the general formula (I) or compounds of the general formula (Ia) and/or pharmaceutically acceptable salts thereof, effective to prevent and/or treat said infectious disease and/or opportunistic infection.
The infectious disease can be selected from the group comprising AIDS, Alveolar Hydatid Disease (AHD, Echinococcosis), Amebiasis (Entamoeba histolytica Infection), Angiostrongylus Infection, Anisakiasis, Anthrax, Babesiosis (Babesia Infection), Balantidium Infection (Balantidiasis), Baylisascaris Infection (Raccoon Roundworm), Bilharzia (Schistosomiasis), Blastocystis hominis Infection (Blastomycosis), Boreliosis, Botulism, Brainerd Diarrhea, Brucellosis, BSE (Bovine Spongiform Encephalopathy), Candidiasis, Capillariasis (Capillaria Infection), CFS (Chronic Fatigue Syndrome), Chagas Disease (American Trypanosomiasis), Chickenpox (Varicella-Zoster virus), Chlamydia pneumoniae Infection, Cholera, Chronic Fatigue Syndrome, CJD (Creutzfeldt-Jakob Disease), Clonorchiasis (Clonorchis Infection), CLM (Cutaneous Larva Migrans, Hookworm Infection), Coccidioidomycosis, Conjunctivitis, Coxsackievirus A16 (Hand, Foot and Mouth Disease), Cryptococcosis, Cryptosporidium Infection (Cryptosporidiosis), Culex mosquito (Vector of West Nile Virus), Cutaneous Larva Migrans (CLM), Cyclosporiasis (Cyclospora Infection), Cysticercosis (Neurocysticercosis), Cytomegalovirus Infection (CMV), Dengue / Dengue Fever, Dipylidium Infection (Dog and Cat Flea Tapeworm), Ebola Virus Hemorrhagic Fever, Echinococcosis (Alveolar Hydatid Disease), Encephalitis, Entomoeba coli Infection, Entomoeba dispar Infection, Entamoeba hartmanni Infection, Entamoeba histolytica Infection (Amebiasis), Entamoeba polecki Infection, Enterobiasis (Pinworm Infection), Enterovirus Infection (Non-Polio), Epstein-Barr Virus Infection, Escherichia coli Infection, Foodbome Infection, Foot and mouth Disease, Fungal Dermatitis, Gastroenteritis, Group A streptococcal Disease, Group B streptococcal Disease, Hansen's Disease (Leprosy), Hantavirus Pulmonary Syndrome, Head Lice Infestation (Pediculosis), Helicobacter pylori Infection, Hematologic Disease, Hendra Virus Infection, Hepatitis, Herpes Zoster (Shingles), HIV Infection, Human Ehrlichiosis, Human Parainfluenza Virus Infection, Influenza, Isosporiasis (Isospora Infection), Lassa Fever, Leishmaniasis, Kala-azar (Kala-azar, Leishmania Infection), Leprosy, Lice (Body lice, Head lice, Pubic lice), Lyme Disease, Malaria, Marburg Hemorrhagic Fever, Measles, Meningitis, Mosquito-borne Diseases, Mycobacterium avium Complex (MAC) Infection, Naegleria Infection, Nosocomial Infections, Nonpathogenic Intestinal Amebae Infection, Onchocerciasis (River Blindness), Opisthorciasis (Opisthorcis Infection), Parvovirus Infection, Plague, PCP (Pneumocystis carinii Pneumonia), Polio, Q Fever, Rabies, Respiratory Syncytial Virus (RSV) Infection, Rheumatic Fever, Rift Valley Fever, River Blindness (Onchocerciasis), Rotavirus Infection, Roundworms Infection, Salmonellosis, Salmonella Enteritidis, Scabies, Shigellosis, Shingles, Sleeping Sickness, Smallpox, Streptococcal Infection, Tapeworm Infection (Taenia Infection), Tetanus, Toxic Shock Syndrome, Tuberculosis, Ulcers (Peptic Ulcer Disease), Valley Fever, Vibrio parahaemolyticus Infection, Vibrio vulnificus Infection; Viral Hemorrhagic Fever, Warts, Waterborne infectious Diseases, West Nile Virus Infection (West Nile Encephalitis), Whooping Cough, Yellow Fever.

Furthermore, the present invention refers to a method for preventing and/or treating diseases like cell proliferation disorders, cardiovascular disorders, immunological diseases, inflammatory diseases, neuroimmunological diseases, autoimmune diseases preferably in mammal, most preferably in human. Said method comprises administering to the mammal an amount of at least one furazanopyrazine derivative of the general formula (I) and/or pharmaceutically acceptable salts thereof, effective to prevent and/or treat cell proliferation disorders, cardiovascular disorders, immunological diseases, inflammatory diseases, neuroimmunological diseases, and/or autoimmune diseases.

The compounds of the present invention are also useful for the treatment of diseases which are caused by malignant cell proliferation, such as all forms of hematological and solid cancer. Therefore the compounds according to the invention and pharmaceutical compositions prepared therewith are generally useful for regulating cell activation, cell proliferation, cell survival, cell differentiation, cell cycle, cell maturation and cell death or to induce systemic changes in metabolism such as changes in sugar, lipid or protein metabolism.

They can also be used to support cell generation poiesis, including blood cell growth and generation (prohematopoietic effect) after depletion or destruction of cells, as caused by, for example, toxic agents, radiation, immunotherapy, growth defects, malnutrition, malabsorption, immune dysregulation, anemia and the like or to provide a therapeutic control of tissue generation and degradation, and therapeutic modification of cell and tissue maintenance and blood cell homeostasis.
The compounds according to the invention and pharmaceutical compositions prepared therewith are generally useful for the treatment of cell proliferation disorders, for the treatment or prophylaxis of cardiovascular disorders, immunological diseases and conditions (as for instance inflammatory diseases, neuroimmunological diseases, autoimmune diseases or other).
These diseases and conditions include but are not limited to cancer as hematological (e.g. leukemia, lymphoma, myeloma) or solid tumors (for example breast, prostate, liver, bladder, lung, esophageal, stomach, colorectal, genitourinary, gastrointestinal, skin, pancreatic, brain, uterine, colon, head and neck, ovarian, melanoma, astrocytoma, small cell lung cancer, glioma, basal and squameous cell carcinoma, sarcomas as Kaposi's sarcoma and osteosarcoma), tumor angiogenesis or metastasis, treatment of disorders involving T-cells such as acute or chronic graft rejection or other host versus graft or graft versus host reactions, aplastic anaemia and DiGeorge syndrome, Graves' disease, lupus erythematosus, Sjogren's Syndrome, fibrosis, uveitis, rhinitis, asthma or athropathy, in particular, arthrosis, all forms of rheumatism or arthritis as rheumatoid arthritis, rheumatoid spondylitis, osteoarthritis, gouty arthritis, multiple sclerosis, pancreatitis, glomerulonephritis, ulcerative colitis, sickle cell anaemia or other forms of anaemia progressive retinal atrophy, inflammatory bowel disease, Morbus Crohn, chronic pulmonary inflammatory disease as well as other chronic or acute inflammations or inflammatory diseases, chronic diarrhea, insulin dependent diabetes mellitus and non-insulin dependent diabetes mellitus as well as diabetic conditions as glaucoma, retinopathy or microangiopathy and other metabolic diseases, ocular conditions as ocular or macula edema, ocular neovascular disease, scleritis, radial keratomy, uveitis, vitritis, myopia, chronical retinal detachment, post-laser treatment complications, conjunctivitis, Stargardt's disease, Eales disease or retinopathy, dermatological disorders such as psoriasis and acute immunological events and disorders such as sepsis, septic shock, endotoxic shock, Gram-negative sepsis, toxic shock sydrome, acute respiratory distress syndrome, stroke, reperfusion injury, CNS injury, serious forms of allergy, Alzheimer's disease or pyresis, atherosclerosis, ischemia/reperfusion injury as for instance stroke or infarct, cardiovascular diseases, vascular smooth muscle proliferation conditions as postsurgical vascular stenosis, restenosis, angina pectoris chronic pulmonary inflammatory disease, silicosis, pulmonary sarcosis, fibrosis, Heppel-Lindau disease, gangrene, necrosis, benign prostate hyperplasia, bone resorption disease as osteoporosis as well as neurodegenerative disorders.

The inventive furazanopyrazine compounds of the general formula (I) and/or pharmaceutically acceptable salts thereof are administered in a dosage corresponding to an effective concentration in the range of 0.001 - 50 µM, preferably in the range of 0.001 - 10 µM, more preferably in the range of 0.001 - 1 µM, and most preferably in the range of 0.001 - 0.1 µM.

In a further aspect the present invention relates to pharmaceutical compositions comprising at least one compound of the general formula (I) as an active ingredient together with one or more pharmaceutically acceptable carrier(s), excipient(s) and/or diluent(s).

The furazanopyrazine compounds of the present invention can form pharmaceutically acceptable salts with organic and inorganic acids. Examples of suitable acids for such acid addition salt formation are hydrochloric acid, hydrobromic acid, sulfuric acid, phosphoric acid, acetic acid, citric acid, oxalic acid, malonic acid, salicylic acid, p-aminosalicylic acid, malic acid, fumaric acid, succinic acid, ascorbic acid, maleic acid, sulfonic acid and other mineral or carboxylic acids well known to those skilled in the art. The salts are prepared by contacting the free base form with a sufficient amount of the desired acid to produce a salt in the conventional manner.
It is also possible to obtain acid addition salts with amino acids like methionine, tryptophane, lysine or arginine, especially with furazanopyrazine compounds of the general formula (I) containing a carboxylic acid residue.
Depending upon the substituents on the inventive furazanopyrazine compounds, one can form salts with bases too. Thus, for example, if there are carboxylic acid substituents in the molecule, salts may be formed with inorganic as well as organic bases such as, for example, NaOH, KOH, NH₄OH, tetraalkylammonium hydroxide, guanidinium, and the like.

The compounds of formula (I) can also be used in the form of a precursor (prodrug) or a suitably modified form, that releases the active compound *in vivo.* Such precursors can be obtained for example by masking an amine with an alkyl group or as an imine, or a free acid group or hydroxy group with an ester group, which is then in turn transformed into the free amino group or acid group or hydroxy function *in vivo* [F. W. Sum et al. Bioorg. & Med. Chem. Lett. 9 (1999), 1921-1926; Ada Rephaeli et al. Drug Development Research 50 (2000) 379-391; H. Ishikawa, Current Med. Chem. 6 (1999), 575-597].

The compounds of the general formula (I) or compounds can also be administered in form of their pharmaceutically active salts optionally using substantially nontoxic pharmaceutically acceptable carriers, excipients or diluents. The medications of the present invention are prepared in a conventional solid or liquid carrier or diluents and a conventional pharmaceutically-made adjuvant at suitable dosage level in a known way. The preferred preparations are in administratable form which is suitable for oral application. These administratable forms, for example, include pills, tablets, film tablets, coated tablets, capsules, powders and deposits.
Furthermore, the subject of the present invention also includes pharmaceutical preparations for parenteral, including dermal, intradermal, intragastrical, intracutaneous, intravasal, intravenous, intramuscular, intraperitoneal, intranasal, intravaginal, intrabuccal, percutaneous, rectal, subcutaneous, sublingual, topical or transdermal application, which in addition to typical vehicles and diluents contain a furazanopyrazine compound of the general formula (I) and/or a pharmaceutically acceptable salt thereof as active ingredient. Within the disclosed methods the pharmaceutical compositions of the present invention, containing furazanopyrazine derivatives of the general formula (I) as active ingredients, will typically be administered in admixture with suitable carrier materials selected with respect to the intended form of administration, i.e. oral tablets, capsules (either solid-filled, semi-solid filled or liquid filled), powders for constitution, oral gels, elixirs, dispersible granules, syrups, suspensions, and the like, and consistent with conventional pharmaceutical practices. For example, for oral administration in the form of tablets or capsules, the active drug component may be combined with any oral nontoxic pharmaceutically acceptable inert carrier, such as lactose, starch, sucrose, cellulose, magnesium stearate, dicalcium phosphate, calcium sulfate, talc, mannitol, ethyl alcohol (liquid forms) and the like.
Moreover, when desired or needed, suitable binders, lubricants, disintegrating agents and coloring agents may also be incorporated in the mixture. Powders and tablets may be comprised of from about 5 to about 95 percent inventive composition.
Suitable binders include starch, gelatin, natural sugars, com-sweeteners, natural and synthetic gums such as acacia, sodium alginate, carboxymethylcellulose, polyethylene glycol and waxes. Among the lubricants, there may be mentioned for use in these dosage forms, boric acid, sodium benzoate, sodium acetate, sodium chloride, and the like. Disintegrants include starch, methylcellulose, guar gum and the like. Sweetening and flavoring agents and preservatives may also be included where appropriate. Some of the terms noted above, namely disintegrants, diluents, lubricants, binders and the like, are discussed in more detail below.
Additionally, the compositions of the present invention may be formulated in sustained release form to provide the rate controlled release of any one or more of the components or active ingredients to optimize the therapeutic effects, i.e. antihistaminic activity and the like. Suitable dosage forms for sustained release include layered tablets containing layers of varying disintegration rates or controlled release polymeric matrices impregnated with the active components and shaped in tablet form or capsules containing such impregnated or encapsulated porous polymeric matrices.
Liquid form preparations include solutions, suspensions and emulsions. As an example may be mentioned water or water-propylene glycol solutions for parenteral injections or addition of sweeteners and opacifiers for oral solutions, suspensions and emulsions. Liquid form preparations may also include solutions for intranasal administration.
Aerosol preparations suitable for inhalation may include solutions and solids in powder form, which may be in combination with a pharmaceutically acceptable carrier such as inert compressed gas, e.g. nitrogen.
For preparing suppositories, a low melting wax such as a mixture of fatty acid glycerides such as cocoa butter is first melted, and the active ingredient is dispersed homogeneously therein by stirring or similar mixing. The molten homogeneous mixture is then poured into convenient sized molds, allowed to cool and thereby solidifies.
Also included are solid form preparations which are intended to be converted, shortly before use, to liquid form preparations for either oral or parenteral administration. Such liquid forms include solutions, suspensions and emulsions.
The inventive furazanopyrazine compounds of the present invention may also be deliverable transdermally. The transdermal compositions may take the form of creams, lotions, aerosols and/or emulsions and can be included in a transdermal patch of the matrix or reservoir type as are conventional in the art for this purpose.
The term capsule refers to a special container or enclosure made of methyl cellulose, polyvinyl alcohols, or denatured gelatins or starch for holding or containing compositions comprising the active ingredients. Hard shell capsules are typically made of blends of relatively high gel strength bone and pork skin gelatins. The capsule itself may contain small amounts of dyes, opaquing agents, plasticizers and preservatives.

Tablet means compressed or molded solid dosage form containing the active ingredients with suitable diluents. The tablet can be prepared by compression of mixtures or granulations obtained by wet granulation, dry granulation or by compaction well known to a person skilled in the art.
Oral gels refers to the active ingredients dispersed or solubilized in a hydrophilic semi-solid matrix.
Powders for constitution refer to powder blends containing the active ingredients and suitable diluents which can be suspended in water or juices.
Suitable diluents are substances that usually make up the major portion of the composition or dosage form. Suitable diluents include sugars such as lactose, sucrose, mannitol and sorbitol, starches derived from wheat, corn rice and potato, and celluloses such as microcrystalline cellulose. The amount of diluents in the composition can range from about 5 to about 95% by weight of the total composition, preferably from about 25 to about 75%, more preferably from about 30 to about 60% by weight. The term disintegrants refers to materials added to the composition to help it break apart (disintegrate). Suitable disintegrants include starches, "cold water soluble" modified starches such as sodium carboxymethyl starch, natural and synthetic gums such as locust bean, karaya, guar, tragacanth and agar, cellulose derivatives such as methylcellulose and sodium carboxymethylcellulose, microcrystalline celluloses and cross-linked microcrystalline celluloses such as sodium croscarmellose, alginates such as alginic acid and sodium alginate, clays such as bentonites, and effervescent mixtures. The amount of disintegrant in the composition can range from about 2 to about 20% by weight of the composition, more preferably from about 5 to about 10% by weight.
Binders characterize substances that bind or "glue" powders together and make them cohesive by forming granules, thus serving as the "adhesive" in the formulation. Binders add cohesive strength already available in the diluent or bulking agent. Suitable binders include sugars such as sucrose, starches derived from wheat, corn rice and potato; natural gums such as acacia, gelatin and tragacanth; derivatives of seaweed such as alginic acid, sodium alginate and ammonium calcium alginate; cellulosic materials such as methylcellulose and sodium carboxymethylcellulose and hydroxypropylmethylcellulose; polyvinyl-pyrrolidone; and inorganics such as magnesium aluminum silicate. The amount of binder in the composition can range from about 2 to about 20% by weight of the composition, more preferably from about 3 to about 10% by weight, even more preferably from about 3 to about 6% by weight.
Lubricant refers to a substance added to the dosage form to enable the tablet, granules, etc. after it has been compressed, to release from the mold or die by reducing friction or wear. Suitable lubricants include metallic stearates such as magnesium stearate, calcium stearate or potassium stearate; stearic acid; high melting point waxes; and water soluble lubricants such as sodium chloride, sodium benzoate, sodium acetate, sodium oleate, polyethylene glycols and D,L-leucine. Lubricants are usually added at the very last step before compression, since they must be present on the surfaces of the granules and in between them and the parts of the tablet press. The amount of lubricant in the composition can range from about 0.2 to about 5% by weight of the composition, preferably from about 0.5 to about 2%, more preferably from about 0.3 to about 1.5% by weight.
Glidents are materials that prevent caking and improve the flow characteristics of granulations, so that flow is smooth and uniform. Suitable glidents include silicon dioxide and talc. The amount of glident in the composition can range from about 0.1% to about 5% by weight of the total composition, preferably from about 0.5 to about 2% by weight. Coloring agents are excipients that provide coloration to the composition or the dosage form. Such excipients can include food grade dyes and food grade dyes adsorbed onto a suitable adsorbent such as clay or aluminum oxide. The amount of the coloring agent can vary from about 0.1 to about 5% by weight of the composition, preferably from about 0.1 to about 1%.

### Examples

The following compounds (examples 1 to 61) were commercially available.
Example 1: **(6-{*N*'-[5-(4-Chloro-3-nitro-phenyl)-furan-2-yhnethylene]-hydrazino}-[1,2,5] oxadiazolo [3,4-b] pyrazin-5-yl)-(2,3-dimethyl-phenyl)-amine.**
Example 2**:2-Chloro-5-(5-{[6-(2,3-dimethyl-phenylamino)-[1,2,5]oxadiazolo[3,4-b]pyrazin-5-yl]-hydrazonomethyl)-furan-2-yl)-benzoic acid.**
Example 3: **5-{6-[*N*'-(2-Benzyloxy-3,5-dibromo-benzylidene)-hydrazino]-[1,2,5] oxadiazolo [3,4-b] pyrazin-5-ylamino}-1,3-dihydro-benzoimidazol-2-one.**
Example 4: **2-Chloro-5-(5-{[6-(2-fluoro-phenylamino)-[1,2,5]ozadiazolo[3,4-b]pyrazin-5-yl]-hydrazonomethyl)-furan-2-yl)-benzoic acid.**
Example 5: **(4-Iodo-phenyl)-{6-[*N*'-(5-methyl-furan-2-ylmethylene)-hydrazino]-[1,2,5]oxadiazolo [3,4-b] pyrazin-5-yl}-amine.**
Example 6: **3-(5-{[6-(2,3-Dimethyl-phenylamino)-[1,2,5]oxadiazolo[3,4-b]pyrazin-5-yl]-hydrazonomethyl}-furan-2-yl)-benzoic acid.**
Example 7: **4-{[6-(2-Fluoro-phenylamino)-[1,2,5]oxadiazolo[3,4-b]pyrazin-5-yl]-hydrazonomethyl}-2-methoxy-6-nitro-phenol.**
Example 8: **{6-[*N*'-(4-Chloro-3-nitro-benzylidene)-hydrazino]-[1,2,5]oxadiazolo[3,4-b]pyrazin-5-yl}-(2-fluoro-phenyl)-amine.**
Example 9: **(2-Methoxy-phenyl)-(6-{*N'*-[1-(4-methyl-furazan-3-yl)-ethylidene]-hydrazino}-[1,2,5]oxadiazolo[3,4-b] pyrazin-5-yl)-amine.**
Example 10: **2-{[6-(2,3-Dimethyl-phenylamino)-[1,2,5]oxadiazolo[3,4-b]pyrazin-5-yl]-hydrazonomethyl}-4-nitro-phenol.**
Example 11: **{6-[*N*'-(4-Nitro-benzylidene)-hydrazino]-[1,2,5]oxadiazolo[3,4-b]pyrazin-5-yl}-*m*-tolyl-amine.**
Example 12: **(4-Chloro-phenyl)-(6-{*N*'-[5-(3-nitro-phenyl)-furan-2-ylmethylene]-hydrazino}-[1,2,5]oxadiazolo[3,4-b]pyrazin-5-yl)-amine.**
Example 13: **2-Chloro-5-(5-{[6-(2-fluoro-phenylamino)-[1,2,5]oxadiazolo[3,4-b]pyrazin-5-yl]-hydrazonomethyl}-furan-2-yl)-benzoic acid.**
Example 14: **2-Bromo-4-chloro-6-{[6-(3,4-dichloro-phenylamino)-[1,2,5] oxadiazolo [3,4-b] pyrazin-5-yl]-hydrazonomethyl}-phenol.**
Example 15: **(6-{*N*'-[5-(2,4-Dichloro-phenyl)-furan-2-ylmethylene]-hydrazino}-[1,2,5]oxadiazolo[3,4-b]pyrazin-5-yl)-(4-fluoro-phenyl)-amine.**
Example 16: **{6-[*N*'-(4-Chloro-3-nitro-benzylidene)-hydrazino]-[1,2,5]oxadiazolo[3,4-b]pyrazin-5-yl}-(4-fluoro-phenyl)-amine.**
Example 17: **{6-[*N'*-(2,4-Dichloro-benzylidene)-hydrazino]-[1,2,5]oxadiazolo[3,4-b]pyrazin-5-yl}-*m*-tolyl-amine.**
Example 18: **(6-{*N*'-(5-(4-Bromo-phenyl)-furan-2-ylmethylene]-hydrazino}-[1,2,5]ozadiazolo[3,4-b]pyrazin-5-yl)-(2,5-dimethyl-phenyl)-amine.**
Example 19: **(4-Bromo-phenyl)-[6-(*N*'-furan-2-ylmethylene-hydrazino)-[1,2,5]oxadiazolo[3,4-b] pyrazin-5-yl]-amine.**
Example 20: **4-{[6-(2-Fluoro-phenylamino)-[1,2,5]oxadiazolo[3,4-b]pyrazin-5-yl]-hydrazonomethyl}-benzoic acid methyl ester.**
Example 21: **2-Bromo-6-{[6-(3-bromo-phenylamino)-[1,2,5]oxadiazolo[3,4-b]pyrazin-5-yl]-hydrazonomethyl}-4-chloro-phenol.**
Example 22: **(6-{*N*'-[5-(4-Chloro-3-nitro-phenyl)-furan-2-ylmethylene]-hydrazino}-[1,2,5] oxadiazolo [3,4-b] pyrazin-5-yl)-(2,3-dimethyl-phenyl)-amine.**
Example 23: **5-(5-{[6-(2-Fluoro-phenylamino)-[1,2,5]oxadiazolo[3,4-b]pyrazin-5-yl]-hydrazonomethyl}-furan-2-yl)-2-hydroxy-benzoic acid.**
Example 24: **(2-Methoxy-phenyl)-{6-[*N*'-(6-nitro-benzo[1,3]dioxol-5-ylmethylene)-hydrazino]-[1,2,5]oxadiazolo[3,4-b]pyrazin-5-yl}-amine.**
Example 25: **{6-[*N'*-(2-Chloro-5-nitro-benzylidene)-hydrazino]-[1,2,5]oxadiazolo[3,4-b]pyrazin-5-yl}-cyclohexyl-amine.**
Example 26: **2-{*N*'-[6-(2-Iodo-phenylamino)-[1,2,5]oxadiazolo[3,4-b]pyrazin-5-yl]-hydrazino}-ethanol.**
Example 27: **(2,3-Dimethyl-phenyl)-{6-[*N*'-(3-phenyl-1*H*-pyrazol-4-ylmethylene)-hydrazino]-[1,2,5]oxadiazolo[3,4-b]pyrazin-5-yl}-amine.**
Example 28: **(4-Methoxy-phenyl)-{6-[*N'*-(3-phenyl-1*H*-pyrazol-4-ylmethylene)-hydrazino]-[1,2,5] oxadiazolo [3,4-b] pyrazin-5-yl}-amine.**
Example 29: **(6-{N'-[5-(2,4-Dimethyl-5-nitro-phenyl)-furan-2-ylmethylene]-hydrazino}-[1,2,5]oxadiaxolo[3,4-b]pyrazin-5-yl)-(2-fluoro-phenyl)-amine.**
Example 30: **2,4-Dibromo-6-{[6-(4-chloro-phenylamino)-[1,2,5]oxadiazolo[3,4-b]pyrazin-5-yl]-hydrazonomethyl}-phenol.**
Example 31: **{6-[*N*'-(4-Bromo-3-nitro-benzylidene)-hydrazino]-[1,2,5]oxadiazolo[3,4-b]pyrazin-5-yl}-(3-bromo-phenyl)-amine.**
Example 32: **4-Bromo-2-{[6-(4-fluoro-phenylamino)-[1,2,5]oxadiazolo[3,4-b]pyrazin-5-yl]-hydrazonomethyl}-phenol.**
Example 33: **(6-{*N*'-(5-(2,5-Dimethyl-3-nitro-phenyl)-furan-2-ylmethylene]-hydrazino}-[1,2,5]oxadiazolo[3,4-b]pyrazin-5-yl)-*p*-tolyl-amine.**
Example 34: **(4-Fluoro-phenyl)-{6-[*N*'-(3-nitro-benzylidene)-hydrazino]-[1,2,5] oxadiazolo [3,4-b] pyrazin-5-yl)-amine.**
Example 35: **2-{[6-(3-Bromo-phenylamino)-[1,2,5]oxadiazolo[3,4-b]pyrazin-5-yl]-hydrazonomethyl}-4,6-diiodo-phenol.**
Example 36: **(6-{N'-[5-(4-Chloro-3-nitro-phenyl)-furan-2-ylmethylene]-hydrazino}-[1,2,5]oxadiazolo[3,4-b]pyrazin-5-yl)-(3-trifluoromethyl-phenyl)-amine.**
Example 37: **4-{6-[*N*'-(2-Hydroxy-3-nitro-benzylidene)-hydrazino]-[1,2,5]oxadiazolo[3,4-b]pyrazin-5-ylamino}-benzoic acid ethyl ester.**
Example 38: **(6-[*N*'-(4-Methoxy-benzylidene)-hydrazinol-[1,2,5]oxadiazolo[3,4-b]pyrazin-5-yl}-*p*-tolyl-amine.**
Example 39: **(4-Fluoro-phenyl)-(6-{*N*'-[5-(4-nitro-phenyl)-furan-2-ylmethylene]-hydrazino}-[1,2,5]oxadiazolo[3,4-b]pyrazin-5-yl)-amine.**
Example 40: **(4-Methoxy-phenyl)-{6-[*N*'-(3,4,5-trimethoxy-benzylidene)-hydrazino]-[1,2,5] oxadiazolo[3,4-b]pyrazin-5-yl}-amine.**
Example 41: **(6-{*N'*-[5-(2-Chloro-phenyl)-furan-2-ylmethylene]-hydrazino}-[1,2,5] ozadiazolo [3,4-b] pyrazin-5-yl)-(2,3-dimethyl-phenyl)-amine.**
Example 42: **4-Bromo-2-[(6-*o*-tolylamino-[1,2,5]ozadiazolo[3,4-b]pyrazin-5-yl)-hydrazonomethyl]-phenol.**
Example 43: **4-[6-(*N*'-Naphthalen-1-ylmethylene-hydrazino)-[1,2,5]oxadiazolo[3,4-b]pyrazin-5-ylamino]-benzoic acid ethyl ester.**
Example 44: **(2-Iodo-phenyl)-{6-[*N*'-(2-methyl-1*H*-indol-3-ylmethylene)-hydrazino]-[1,2,5]ozadiazolo[3,4-b]pyrazin-5-yl}-amine.**
Example 45: **{6-[*N*-'(3-Bromo-4-methoxy-benzylidene)-hydrazino]-[1,2,5]oxadiazolo[3,4-b]pyrazin-5-yl}-(2-methoxy-phenyl)-amine.**
Example 46: **4-{[6-(4-Chloro-phenylamino)-[1,2,5]ozadiazolo[3,4-b]pyrazin-5-yl]-hydrazonomethyl}-2-methozy-phenol.**
Example 47: **2,4-Diiodo-6-{[6-(2-methozy-phenylamino)-[1,2,5]oxadiazolo[3,4-b]pyrazin-5-yl]-hydrazonomethyl}-phenol.**
Example 48: ***o*-Tolyl-{6-[*N*'-(2-trifluoromethyl-benzylidene)-hydrazino]-[1,2,5]ozadiazolo[3,4-b]pyrazin-5-yl}-amine.**
Example 49: **[6-(*N*'-Naphthalen-1-ylmethylene-hydrazino)-[1,2,5]oxadiazolo[3,4-b]pyrazin-5-yl]-*o*-tolyl-amine.**
Example 50: **{6-[*N*'-(2,4-Dichloro-5-nitro-benzylidene)-hydrazino]-[1,2,5]ozadiazolo[3,4-b]pyrazin-5-yl}-*p*-tolyl-amine.**
Example 51: **{6-[*N*'-(3-Bromo-4-methoxy-benzylidene)-hydrazino]-[1,2,5] oxadiazolo [3,4-b] pyrazin-5-yl}-(4-fluoro-phenyl)-amine.**
Example 52: **5-(5-{[6-(4-Bromo-phenylamino)-[1,2,5]oxadiazolo[3,4-b]pyrazin-5-yl]-hydrazonomethyl}-furan-2-yl)-2-chloro-benzoic acid.**
Example 53: **2-Chloro-5-(5-1[6-(4-fluoro-phenylamino)-[1,2,5]oxadiazolo[3,4-b]pyrazin-5-yl]-hydrazonomethyl}-furan-2-yl)-benzoic acid.**
Example 54: **{*6-*[*N'*-(3,4-Dimethoxy-benzylidene)-hydrazino]-[1,2,5]oxadiazolo[3,4-b]pyrazm-5-yl}-(4-fluoro-phenyl)-amine.**
Example 55: **5-(6-{*N*'-[2-(2,4-Dichloro-benzyloxy)-3,5-diiodo-benzylidene]-hydraxino}-[1,2,5]oxadiazolo[3,4-b]pyrazin-5-ylamino)-1,3-dihydro-benzoimidazol-2-one.**
Example 56: **(4-Chloro-phenyl)-(6-{*N'*-[5-(3-nitro-phenyl)-furan-2-ylmethylene]-hydrazino}-[1,2,5] oxadiazolo [3,4-b] pyrazin-5-yl)-amine.**
Example 57: **(6-{*N*'-[5-(4-Chloro-3-nitro-phenyl)-furan-2-ylmethylene]-hydrazino}-[1,2,5] oxadiazolo [3,4-b] pyrazin-5-yl)-(4-fluoro-phenyl)-amine.**
Example 58: **2-{[6-(4-Methoxy-phenylamino)-(1,2,5]oxadiazolo[3,4-b]pyrazin-5-yl]-hydrazonomethyl}-6-nitro-phenol.**
Example 59: **2-{[6-(4-Fluoro-phenylamino)-[1,2,5]oxadiazolo[3,4-b]pyrazin-5-yl]-hydrazonomethyl)-6-nitro-phenol.**
Example 60: **3-{5-((6-*m*-Tolylamino-[1,2,5]oxadiazolo[3,4-b]pyrazin-5-yl)-hydrazonomethyl]-furan-2-yl}-benzoic acid.**
Example 61: **4-{6-[*N*'-(3-Phenyl-1*H*-pyrazol-4-ylmethylene)-hydrazino]-[1,2,5]oxadiazolo[3,4-b]pyrazin-5-ylaminol-benzoic** acid ethyl ester.

The following section describes the synthesis of compounds of formula (I).

NMR spectra: Bruker Avance 300 MHz. The spectra were recorded in DMSO-*d*_{*6*} at 300 MHz (¹H NMR) and 75 MHz (¹³C NMR), respectively, using the residual solvent peak as an internal standard (δ_{H} = 2.49, δ_{C} = 39.70).
Analytical LC/ESI-MS: 2 x Waters 600 Multisolvent Delivery System. 50 µl sample loop. Column, Chromolith Speed ROD RP18e (Merck, Darmstadt), 50 x 4.6 mm, with 2 µm prefilter (Merck). Eluent A, H₂O + 0.1 % HCO₂H; eluent B, MeCN. Gradient, 5 % B to 100 % B within 5 min; flow, 3 ml/min. Waters LCZ single quadrupol mass spectrometer with electrospray source. MS method, MS8minPM-80-800-20V; positive/negative ion mode scanning, *m*/*z* 80 - 800 in 1 s; capillary, 3.5 kV; cone voltage, 20 V; multiplier voltage, 400 V; probe and desolvation gas temperature, 120° C and 350° C, respectively. Waters 2487 Dual λ Absorbance Detector, set to 254 nm.
Preparative HPLC-MS: Waters 600 Multisolvent Delivery System with peparative pump heads. 2000 µl or 5000 µl sample loop. Column, Waters X-Terra RP18, 7 µm, 19 x 150 mm with X-Terra RP 18 guard cartridge 7 µm, 19 x 10 mm; used at flow rate 20 ml/min or YMC ODS-A, 120 Å, 40 x 150 mm with X-Terra RP18 guard cartridge 7 µm, 19 x 10 mm; used at flow rate 50 ml/min. Make-up solvent: MeCN - H₂O - HCO₂H 80 : 20 : 0.05 (v:v:v). Eluent A, H₂O + 0.1 % HCO₂H; eluent B, MeCN. Different linear gradients from 5 - 100% Eluent B, adapted to sample. Injection volume: 500 µl - 2000 µl depending on sample. Waters ZQ single quadrupol mass spectrometer with electrospray source. Positive or negative ion mode scanning *m*/*z* 80 - 800 in 1 s; capillary, 3.5 kV or 3.0 kV; cone voltage, 20 V; multiplier voltage, 400 V; probe and desolvation gas temperature, 120° C and 350° C, respectively. Waters Fraction Collector II with mass-triggered fraction collection. Waters 996 photo diode array detector.

**5,6-Dichloro-[1,2,5]oxadiazolo[3,4-b]pyrazine.**
In a flask with reflux condenser and stirring bar, a mixture of phosphorus oxychloride (5.6 mL, 64.1 mmol), phosphorus pentachloride (12.5 g, 59.8 mmol), and [1,2,5]oxadiazolo[3,4-b]pyrazine-5,6-diol (4.39 g, 28.5 mmol) was heated to 95°C for 2 h (caution: HCl development). After cooling, the condenser was replaced with a distillation bridge and POCl₃ was distilled off with heating. The remaining residue was cooled in an ice bath and treated with cold water (caution: violent reaction of residual phosphorus chlorides may occur). The precipitate was filtered off and washed with two portions of cold water. The solid was largely dissolved by addition of acetone (15 mL) and then precipitated by addition of water (25 mL). The precipitate was filtered off and washed with cold water (2 x). After drying *in vacuo,* a beige solid (2.42 g, 12.7 mmol, 44 %) was obtained which was used without further purification.

**General synthesis of compounds of formula (I) wherein R' is -NR**^{**1**}**R**^{**2**} **and R" is -NH-NHBoc** (Boc = *tert*-butyloxycarbonyl).
In a flask with stirring bar, 5,6-dichloro-[1,2,5]oxadiazolo[3,4-b]pyrazine (0.420 g, 2.20 mmol) was dissolved in THF (2 mL). After cooling to 0°C, a solution of the appropriate amine (formula (IV); 2.00 mmol) in THF (2 mL) was added dropwise. After stirring for 10 min at 0°C, triethylamine (0.28 mL, 2.00 mmol) was added. After stirring for 1 h at 0°C, a solution of *tert*-butyl carbazate (formula (III); 0.291 g, 2.2 mmol) in THF (2 mL) was added followed by triethylamine (0.31 mL, 2.2 mmol). The order of adding the amine and *tert*-butyl carbazate may also be reversed. The mixture was stirred overnight at r.t. After removal of the solvent *in vacuo,* the residue was agitated with water (5 mL). The solid was filtered off and washed with water (2 x). The crude products weres purified by preparative HPLC or by recrystallisation. Yields covered a range between 20 and 80 %.

***N*'-(6-Amino-[1,2,5]oxadiazolo[3,4-b]pyrazin-5-yl)-hydrazinecarboxylic acid *tert-*butyl** ester was prepared from 5,6-dichloro-[1,2,5]oxadiazolo[3,4-b]pyrazine, *tert*-butyl carbazate, and ammonia (large excess, 7 M solution in methanol).
¹H NMR: δ = 1.46 (s, 9 H), 7.04 (s, br., 1 H), 8.07 (s, br., 1 H), 9.62 (s, br., 1 H), 11.19 (s, br., 1 H); LC/(+)-ESI-MS: *m*/*z* = 268 [M+H]⁺, 253 [268-CH₃]⁺, 212 [268-*iso*butene]⁺, 168 [212-CO₂]⁺; LC/(-)-ESI-MS: *m*/*z* = 266 [M-H]⁻, 192 [266-*t*-BuOH]⁻.

***N*'-(6-Methoxy-[1,2,5]oxadiazolo[3,4-b]pyrazin-5-yl)-hydrazinecarboxylic acid *tert***-butyl ester. This compound was obtained as a second product in the course of the preparation of *N*'-(6-amino-[1,2,5]oxadiazolo[3,4-b]pyrazin-5-yl)-hydrazinecarboxylic acid tert-butyl ester.
¹H NMR: δ = 1.43 (s, 9 H), 4.12 (s, 3 H), 9.34 (s, br., 1 H), 10.42 (s, br., 1 H). LC/(+)-ESI-MS: *m*/*z* = 283 [M+H]⁺, 268 [283-CH₃]⁺, 227 [283-*iso*butene]⁺, 183 [227-CO₂]⁺.
LC/(-)-ESI-MS: *m*/*z=* 281 [M-H]⁻, 207 [281-*t*-BuOH]⁻.

***N*'-(6-Phenylamino-[1,2,5]oxadiazolo[3,4-b]pyrazin-5-yl)-hydrazinecarboxylic acid *tert-*butyl ester** was prepared from 5,6-dichloro-[1,2,5]oxadiazolo[3,4-b]pyrazine, aniline, and tert-butyl carbazate.
¹H NMR: δ = 1.49 (s, 9 H), 7.17 (t, 1 H, *J=* 7.7 Hz), 7.41 (t, 2 H, *J=* 7.7 Hz), 7.80 (d, 2 H, *J=* 7.7 Hz), 9.32 (s, br., 1 H), 9.79 (s, br., 1 H), 11.40 (s, br., 1 H); LC/(+)-ESI-MS: *m*/*z =* 344 [M+H]⁺, 288 [344-*iso*butene]⁺, 244 [288-CO₂]⁺; LC/(-)-ESI-MS: *m*/*z* = 342 [M-H]⁻, 268 [342-*t*-BuOH]⁻.

***N*'-(6-Benzylamino-[1,2,5]oxadiazolo[3,4-b]pyrazin-5-yl)-hydrazinecarboxylic acid *tert-*butyl ester** was prepared from 5,6-dichloro-[1,2,5]oxadiazolo[3,4-b]pyrazine, benzylamine, and *tert*-butyl carbazate.
¹H NMR: δ = 1.46 (s, 9 H), 4.64 (d, 2 H, *J* = 6.4 Hz), 7.20-7.40 (m, 5 H), 8.27 (s, br., 1 H), 9.70 (s, br., 1 H), 11.29 (s, br., 1 H); LC/(+)-ESI-MS: *m*/*z* = 358 [M+H]⁺, 302 [358-*iso*butene]⁺, 258 [302-CO₂]⁺; LC/(-)-ESI-MS: *m*/*z* = 356 [M-H]⁻, 282 [M-*t*-BuOH]⁻.

***N*'-[6-(4-Fluoro-phenylamino)-[1,2,5]ozadiazolo[3,4-b]pyrazin-5-yl]-hydrazinecarboxylic acid *tert*-butyl ester** was prepared from 5,6-dichloro-[1,2,5]oxadiazolo[3,4-b]pyrazine, 4-fluoroaniline, and *tert*-butyl carbazate.
¹H NMR: δ = 1.49 (s, 9 *H*), 7.24 (dd, 2 H, ³*J*_{HH} *≈* ³*J*_{HF} ≈ 9 Hz), 7.81 (dd, 2 H, ³*J*_{HH} = 9.2 Hz, ⁴*J*_{HF} = 5.0 Hz), 9.40 (s, br., 1 H), 9.78 (s, br., 1 H), 11.40 (s, br., 1 H); LC/(+)-ESI-MS: *m*/*z* = 362 [M+H]⁺, 306 [362-*iso*butene]⁺, 262 [306-CO₂]⁺; LC/(-)-ESI-MS: *m*/*z* = 360 [M-H]⁻, 286 [M-*t*-BuOH]⁻.

***N*'-[6-(3-Chloro-phenylamino)-[1,2,5] oxadiazolo [3,4-b] pyrazin-5-yl]-hydrazinecarboxylic** acid ***tert*-butyl ester** was prepared from 5,6-dichloro-[1,2,5]oxadiazolo[3,4-b]pyrazine, 3-chloroaniline, and tert-butyl carbazate.
¹H NMR: δ = 1.49 (s, 9 H), 7.22 (d, 1 H*, J =* 8.1 Hz), 7.42 (dd,1 H, *J = J =* 8.1 Hz), 7.73 (d, 1 H, *J*= 8.1 Hz), 7.94 (s, 1 H), 8.06 (dd, 1 H*, J= J=* 1.7 Hz), 9.57 (s, br., 1 H), 9.83 (s, br., 1 H), 11.45 (s, br., 1 H); LC/(+)-ESI-MS: *m*/*z* = 378 [M+H]⁺, 322 [378-*iso*butene]⁺, 278 [322-CO₂]⁺; LC/(-)-ESI-MS: *m*/*z* = 376 [M-H]⁻, 302 [M-*t*-BuOH]⁻.

***N*'-[6-(3-Chloro-4-fluoro-phenylamino)-[1,2,5]oxadiazolo[3,4-b]pyrazin-5-yl]-hydrazinecarboxylic acid *tert*-butyl ester** was prepared from 5,6-dichloro-[1,2,5]oxadiazolo[3,4-b]pyrazine, 3-chloro-4-fluoroaniline, and tert-butyl carbazate.
¹H NMR: δ = 1.49 (s, 9 H), 7.45 (dd, 1 H, *3J*_{HH} *=* ^{*3*}*J*_{HF} = 9.0 Hz), 7.77 (ddd, 1 H, ³*J*_{HH} = 9.0 Hz, ⁴*J*_{HF} = 4.3 Hz, ⁴*J*_{HH} = 2.7 Hz), 8.13 (dd, 1 H, ⁴*J*_{HF} = 6.8 Hz, ⁴*J*_{HH} = 2.7 Hz), 9.53 (s, br., 1 H), 9.76 (s, br., 1 H), 11.41 (s, br., 1 H); LC/(+)-ESI-MS: *m*/*z* = 396 [M+H]⁺, 381 [396-CH₃]⁺, 340 [396-*iso*butene]⁺, 296 [340-CO₂]⁺; LC/(-)-ESI-MS: *m*/*z* = 394 [M-H]⁻, 320 [394-*t*-BuOH]⁻.

***N*'-[6-(3,4-Dichloro-phenylamino)-[1,2,5]oxadiazolo[3,4-b]pyrazin-5-yl]-hydrazinecarboxylic acid *tert-*butyl ester** was prepared from 5,6-dichloro-[1,2,5]oxadiazolo[3,4-b]pyrazine, 3,4-dichloroaniline, and *tert-*butyl carbazate.
¹H NMR: δ = 1.49 (s, 9 H), 7.64 (d, 1 H, *J=* 8.9 Hz), 7.82 (dd, 1 H, *J=* 8.9 Hz, *J =* 2.5 Hz), 8.23 (d, 1 H, J= 2.5 Hz), 9.67 (s, br., 1 H), 9.80 (s, br., 1 H), 11.41 (s, br., 1 H); LC/(+)-ESI-MS: *m*/*z* = 412 [M+H, ³⁷Cl₂]⁺, 356 [412-*iso*butene]⁺, 312 [356-CO₂]⁺; LC/(-)-ESI-MS: *m*/*z* = 410 [M-H, ³⁷Cl₂]⁻.

***N'*-[6-(2-*tert*-Butoxycarbonylamino-phenylamino)-[1,2,5]oxadiazolo[3,4-b]pyrazin-5-yl]-hydrazinecarboxylic acid *tert*-butyl ester** was prepared from 5,6-dichloro-[1,2,5]oxadiazolo[3,4-b]pyrazine, (2-amino-phenyl)-carbamic acid *tert*-butyl ester, and *tert-*butyl carbazate. (2-Amino-phenyl)-carbamic acid *tert*-butyl ester was prepared from 1,2-phenylenediamine, di-*tert*-butyl dicarbonate, and triethylamine.
¹H NMR: δ = 1.44 (s, 9 H), 1.48 (s, 9 H), 7.19 ("td", 1 H, *J* ≈ 7.4 Hz, *J*=1.2 Hz), 7.25 ("td", 1 H, *J ≈* 7.4 Hz, *J*=1.2 Hz), 7.32 ("dd", 1 H, *J=* 7.6 Hz, *J=* 1.1 Hz), 7.94 (d, 1 H, *J* = 7.6 Hz), 8.93 (s, br., 1 H), 9.28 (s, br., 1 H), 9.73 (s, br., 1 H), 11.37 (s, br., 1 H); LC/(+)-ESI-MS: *m*/*z* = 459 [M+H]⁺, 403 [459-*iso*butene]⁺, 347 [403-*iso*butene]⁺, 303 [347-CO₂]⁺, 259 [303-CO₂]⁺; LC/(-)-ESI-MS: *m*/*z* = 457 [M-H]⁻, 383 [457-*t*-BuOH]⁻, 309 [383-*t*-BuOH]⁻.

**General synthesis of compounds of formula (I) wherein R' is -NR**^{**1**}**R**^{**2**} **and R" is-NH-NH**_{**2**}**.**
To the appropriate *N*'-(6-amino-[1,2,5]oxadiazolo[3,4-b]pyrazin-5-yl)-hydrazinecarboxylic acid *tert*-butyl ester (formula (I) wherein R' is -NR¹R² and R" is -NH-NHBoc; 1 mmol) was added a 4 M solution of HCl in 1,4-dioxane (4 mL). The mixture was stirred at r.t. and monitored by TLC. After completion of the reaction (2 h), the volatiles were removed in *vacuo.* The products were obtained as hydrochlorides with nearly quantitative yields and were used without further purification.

**6-Hydrazino-[1,2,5]oxadiazolo[3,4-blpyrazin-5-ylamine hydrochloride** was prepared from *N*'-(6-Amino-[1,2,5]oxadiazolo[3,4-b]pyrazin-5-yl)-hydrazinecarboxylic acid *tert*-butyl ester.

**(6-Methoxy-[1,2,5]oxadiazolo[3,4-b]pyrazin-5-yl)-hydrazine hydrochloride** was prepared from *N*'-(6-Methoxy-[1,2,5]oxadiazolo[3,4-b]pyrazin-5-yl)-hydrazinecarboxylic acid *tert*-butyl ester.

**(6-Hydrazino-[1,2,5]ozadiazolo[3,4-b]pyrazin-5-yl)-phenyl-amine** hydrochloride was prepared from *N*'-(6-Phenylamino-[1,2,5]oxadiazolo[3,4-b]pyrazin-5-yl)-hydrazinecarboxylic acid *tert-*butyl ester.

**Benzyl-(6-hydrazino-[1,2,5]ozadiazolo[3,4-b]pyrazin-5-yl)-amine** hydrochloride was prepared from *N*'-(6-Benzylamino-[1,2,5]oxadiazolo[3,4-b]pyrazin-5-yl)-hydrazinecarboxylic acid *tert*-butyl ester.

**(4-Fluoro-phenyl)-(6-hydrazino-[1,2,5]ozadiazolo[3,4-b]pyrazin-5-yl)-amine** hydrochloride was prepared from *N*'-[6-(4-Fluoro-phenylamino)-[1,2,5]oxadiazolo[3,4-b]pyrazin-5-yl]-hydrazinecarboxylic acid *tert*-butyl ester.

**(3-Chloro-phenyl)-(6-hydrazino-[1,2,5]oxadiazolo[3,4-b]pyrazin-5-yl)-amine** hydrochloride was prepared from *N*'-[6-(3-Chloro-phenylamino)-[1,2,5]oxadiazolo[3,4-b]pyrazin-5-yl]-hydrazinecarboxylic acid *tert*-butyl ester.

**(3-Chloro-4-fluoro-phenyl)-(6-hydrazino-[1,2,5]oxadiazolo[3,4-b]pyrazin-5-yl)-amine** hydrochloride was prepared from *N*'-[6-(3-Chloro-4-fluoro-phenylamino)-[1,2,5]oxadiazolo[3,4-b]pyrazin-5-yl]-hydrazinecarboxylic acid *tert-*butyl ester.

**(3,4-Dichloro-phenyl)-(6-hydrazino-[1,2,5]ozadiazolo[3,4-b]pyrazin-5-yl)-amine hydrochloride** was prepared from *N*'-[6-(3,4-Dichloro-phenylamino)-[1,2,5]oxadiazolo[3,4-b]pyrazin-5-yl]-hydrazinecarboxylic acid tert-butyl ester.

***N*-(6-Hydrazino-[1,2,5]ozadiazolo[3,4-b]pyrazin-5-yl)-benzene-1,2-diamine hydrochloride** was prepared from *N*'-[6-(2-*tert*-Butoxycarbonylamino-phenylamino)-[1,2,5]oxadiazolo[3,4-b]pyrazin-5-yl]-hydrazinecarboxylic acid tert-butyl ester.

**General Synthesis of compounds of formula (V), wherein R**^{**a**} **is 5-arylfuryl and R**^{**b**} **= H.**
The appropriate amine (10 mmol) was suspended in water (9 mL) and conc. hydrochloric acid (2 mL) was added. After heating to reflux, the mixture was cooled to 0°C. Under vigorous stirring, a solution of sodium nitrite (0.69 g, 10 mmol) in water (2.4 mL) was added dropwise at 0 to 5°C. The mixture was stirred for further 0.5 h at 0°C. Furfural (1.4 mL, 15 mmol) was added dropwise followed by a solution of copper(II)-chloride (0.26 g, 1.5 mmol) in water (1 mL). The mixture was allowed to warm to r.t. overnight. The precipitate was filterred off and washed with water. The crude product was purified by flash chromatography or recrystallization, respectively.

**4-(5-Formyl-furan-2-yl)-benzoic acid** was prepared from 4-aminobenzoic acid and furfural.
¹H NMR: δ = 7.43 (d, 1 H, *J=* 3.8 Hz), 7.67 (d, 1 H, J = 3.8 Hz), 7.98 (d, 2 H, *J=* 8.7 Hz), 8.05 (d, 2 H, *J=* 8.7 Hz), 9.65 (s, 1 H), 13.08 (s, br., 1 H); LC/(+)-ESI-MS: *m*/*z* = 217 [M+H]⁺; LC/(-)-ESI-MS: *m*/*z* = 215 [M-H]⁻.

**3-(5-Formyl-furan-2-yl)-benzoic acid** was prepared from 3-amino-benzoic acid and furfural.
¹H NMR: δ = 7.3 7 (d, 1 H, *J* = 3.7 Hz), 7.60 (dd, 1 H, *J = J =* 7.8 Hz), 7.65 (d, 1 H, *J =* 3.7 Hz), 7.98 ("d", 1 H, *J*= 7.8 Hz), 8.06 ("d", 1 H, *J*= 7.8 Hz), 8.38 ("s", 1 H), 9.63 (s, 1 H); LC/(+)-ESI-MS: *m*/*z* = 217 [M+H]⁺; LC/(-)-ESI-MS: *m*/*z* = 215 [M-H]⁻.

**2-Chloro-5-(5-formyl-furan-2-yl)-benzoic** acid was prepared from 5-amino-2-chloro-benzoic acid and furfural.
¹H NMR: δ = 7.04 (d, 1 H, *J =* 3.7 Hz), 7.62 (d, 1 H, *J* = 3.7 Hz), 7.71 (dd, 1 H, *J* = 8.3 Hz, *J=* 2.3 Hz), 7.76 (dd, 1 H, *J=* 2.3 Hz, *J=* 0.5 Hz), 7.78 (dd, 1 H, *J=* 8.3 Hz, *J*= 0.5 Hz), 9.60 (s, 1 H), 13.51 (s, br., 1 H); LC/(+)-ESI-MS: *m*/*z* = 251 [M+H]⁺; LC/(-)-ESI-MS: *m*/*z* = 249 [M-H]⁻.

**3-(5-Formyl-furan-2-yl)-benzamide** was prepared from 3-amino-benzamide and furfural.
¹H NMR: δ = 7.34 (d, 1 H, *J*= 3.7 Hz), 7.47 (s, br., 1 H), 7.59 (dd, 1 H, *J*= *J*= 7.8 Hz), 7.66 (d, 1 H, *J* = 3.7 Hz), 7.92 (dd, 1 H, *J*= 7.8 Hz, *J* =1.5 Hz), 8.00 (dd, 1 H, *J*= 7.8 Hz, *J=* 1.5 Hz), 8.11 (s, br., 1 H), 8.34 (dd, 1 H, *J=J=*1.5 Hz), 9.63 (s, 1 H); LC/(+)-ESI-MS: *m*/*z=* 216 [M+H]⁺.

**General synthesis of compounds of formula (I) wherein R' is -NR**^{**1**}**R**^{**2**} **and R" is-NR**^{**5**}**-N=CR**^{**a**}**R**^{**b**}**.**
The appropriate aldehyde (50 µmol) was dissolved in ethanol (0.5 mL). A solution of the appropriate hydrazine derivative (formula (I) wherein R' is -NR¹R² and R" is ―NH-NH₂) in ethanol (0.5 mL) was added, and the mixture was stirred at 70°C for 1 h. If no precipitate was formed on cooling to r.t., the product was purified by preparative HPLC. If a precipitate was formed, it was separated by centrifugation. The solid was agitated with ethanol (0.75 mL) and separated again by centrifugation. Yields covered a range between 20 and 90 %.
Example 62: **4-[(6-Amino-[1,2,5]oxadiazolo[3,4-b]pyrazin-5-yl)-hydrazonomethyl]**-benzoic acid was prepared from 6-hydrazino-[1,2,5]oxadiazolo[3,4-b]pyrazin-5-ylamine hydrochloride and 4-formyl-benzoic acid.
   ¹H NMR: δ = 7.66 (s, br., 1 H), 8.02 (d, 2 H, *J* = 8.3 Hz), 8.20 (d, 2 H, *J* = 8.3 Hz), 8.31 (s, br., 1 H), 8.61 (s, 1 H), 12.16 (s, br., 1 H), 13.09 (s, br., 1 H); LC/(+)-ESI-MS: *m*/*z* = 300 [M+H]⁺; LC/(-)-ESI-MS: *m*/*z* = 298 [M-H]⁻.
Example 63: **4-[(6-Amino-[1,2,5]oxadiazolo[3,4-b]pyrazin-5-yl)-hydrazonomethyl]-benzene-1,3-diol** was prepared from 6-hydrazino-[1,2,5]oxadiazolo[3,4-b]pyrazin-5-ylamine hydrochloride and 2,4-dihydroxy-benzaldehyde.
   ¹H NMR: δ = 6.33-6.38 (m, 2 H), 7.58 (s, br., 1 H), 7.71 (d, 1 H, *J=* 8.1 Hz), 8.15 (s, br., 1 H), 8.65 (s, 1 H), 10.05 (s, br., 1 H), 10.24 (s, br., 1 H), 12.03 (s, br., 1 H); LC/(+)-ESI-MS: *m*/*z* = 288 [M+H]⁺; LC/(-)-ESI-MS: *m*/*z* = 286 [M-H]⁻.
Example 64: **2-[(6-Amino-[1,2,5]oxadiazolo[3,4-blpyrazin-5-yl)-hydrazonomethyl]-5**-methoxy-phenol was prepared from 6-hydrazino-[1,2,5]oxadiazolo[3,4-b]pyrazin-5-ylamine hydrochloride and 2-hydroxy-4-methoxy-benzaldehyde.
   ¹H NMR: δ = 3.79 (s, 3 H), 6.49 (d, 1 H, *J*= 2.4 Hz), 6.53 (dd, 1 H, *J=* 8.6 Hz, *J=* 2.4 Hz), 7.62 (s, br., 1 H), 7.89 (d, 1 H, *J*= 8.6 Hz), 8.20 (s, br., 1 H), 8.71 (s, 1 H), 10.28 (s, br., 1 H), 12.08 (s, br., 1 H); LC/(+)-ESI-MS: *m*/*z* = 302 [M+H]⁺; LC/(-)-ESI-MS: *m*/*z* = 300 [M-H]-.
Example 65: **2-[(6-Amino-[1,2,5]oxadiazolo[3,4-b]pyrazin-5-yl)-hydrazonomethyl]-4**-methoxy-phenol was prepared from 6-hydrazino-[1,2,5]oxadiazolo[3,4-b]pyrazin-5-ylamine hydrochloride and 2-hydroxy-5-methoxy-benzaldehyde.
   ¹H NMR: δ = 3.76 (s, 3 H), 6.85 (d, 1 H, *J=* 8.9 Hz), 6.96 (dd, 1 H, *J*= 8.9 Hz, *J=* 3.0 Hz), 7.63 ("s", br., 2 H), 8.20 (s, br., 1 H), 8.77 (s, 1 H), 9.77 (s, br., 1 H), 12.06 (s, br., 1 H); LC/(+)-ESI-MS: *m*/*z* = 302 [M+H]⁺; LC/(-)-ESI-MS: *m*/*z* = 300 [M-H]⁻.
Example 66: **2-[(6-Amino-[1,2,5]ozadiazolo[3,4-b]pyrazin-5-yl)-hydrazonomethyl]-4**-chloro-phenol was prepared from 6-hydrazino-[1,2,5]oxadiazolo[3,4-b]pyrazin-5-ylamine hydrochloride and 5-bromo-2-hydroxy-3-methoxy-benzaldehyde.
   ¹H NMR: δ = 6.96 (d, 1 H, *J* = 8.8 Hz), 7.35 (dd, 1 H, *J* = 8.8 Hz, *J =* 2.8 Hz), 7.68 (s, br., 1 H), 8.25 (s, br., 1 H), 8.30 (d, br., 1 H, *J=* 2.0 Hz), 8.77 (s, 1 H), 10.45 (s, br., 1 H), 12.11 (s, br., 1 H); LC/(+)-ESI-MS: *m*/*z* = 306 [M+H]⁺; LC/(-)-ESI-MS: *m*/*z* = 304 [M-H]⁻.
Example 67: **3-{5-[(6-Amino-[1,2,5]oxadiazolo[3,4-b]pyrazin-5-yl)-hydrazonomethyl]-furan-2-yl}-benzoic** acid was prepared from 6-hydrazino-[1,2,5]oxadiazolo[3,4-b]pyrazin-5-ylamine hydrochloride and 3-(5-formyl-furan-2-yl)-benzoic acid.
   ¹H NMR: δ = 7.34 (d, 1 H, *J=* 3.6 Hz), 7.39 (d, 1 H, *J=* 3.6 Hz), 7.63 (t, 1 H, *J=* 7.7 Hz), 7.64 (s, br., 1 H), 7.93 (ddd, 1 *H, J=* 7.7 *Hz, J=* 1.6 *Hz, J=* 1.2 Hz), 8.13 (ddd, 1 *H, J=* 7.7 Hz, *J=* 1.6 Hz, *J=* 1.2 Hz), 8.27 (s, br., 1 H), 8.39 (dd, 1 H, *J= J=* 1.6 Hz), 8.42 (s, 1 H), 11.97 (s, br., 1 H), 13.13 (s, br., 1 H); LC/(+)-ESI-MS: *m*/*z* = 366 [M+H]⁺; LC/(-)-ESI-MS: *m*/*z* = 364 [M-H]⁻.
Example 68: **6-{*N*'-[5-(3-Nitro-phenyl)-furan-2-ylmethylene]-hydrazino}-[1,2,5]oxa-diazolo[3,4-b]pyrazin-5-ylamine** was prepared from 6-hydrazino-[1,2,5]oxadiazolo[3,4-b]pyrazin-5-ylamine hydrochloride and 5-(3-nitro-phenyl)-furan-2-carbaldehyde.
   ¹H NMR: δ = 7.41 (d, 1 H, *J=* 3.6 Hz), 7.50 (d, 1 H, *J=* 3.6 Hz), 7.65 (s, br., 1 H), 7.79 (t, 1 H, *J=* 7.9 Hz), 8.20 (ddd, 1 H, *J=* 8.2 Hz, *J=* 2.3 Hz, *J=* 0.8 Hz), 8.29 (s, br., 1 H), 8.32 (d, br., 1 H, *J ≈* 8 Hz), 8.44 (s, 1 H), 8.63 (t, 1 H, *J=* 1.8 Hz), 11.98 (s, br., 1 H); LC/(+)-ESI-MS: *m*/*z* = 367 [M+H]⁺; LC/(-)-ESI-MS: *m*/*z* = 365 [M-H]⁻.
Example 69: **6-[*N*'-(5-Phenyl-furan-2-ylmethylene)-hydrazinol-[1,2,5]oxadiazolo[3,4-b]pyrazin-5-ylamine** was prepared from 6-hydrazino-[1,2,5]oxadiazolo[3,4-b]pyrazin-5-ylamine hydrochloride and 5-phenyl-furan-2-carbaldehyde.
   ¹H NMR: δ = 7.22 (d, 1 H, *J=* 3.6 Hz), 7.36 (d, 1 H, *J=* 3.6 Hz), 7.38 (t, 1 H, *J=* 7.3 Hz), 7.49 (t, 1 H, *J=* 7.3 Hz), 7.64 (s, br., 1 H), 7.90 (d, 1 H, *J=* 7.3 Hz), 8.27 (s, br., 1 H), 8.41 (s, 1 H), 11.90 (s, br., 1 H); LC/(+)-ESI-MS: *m*/*z* = 322 [M+H]⁺; LC/(-)-ESI-MS: *m*/*z* = [M-H]⁻.
Example 70: **5-15-[(6-Amino-[1,2,5]oxadiazolo[3,4-b]pyrazin-5-yl)-hydrazonomethyl]-furan-2-yl}-2-chloro-benzoic** acid was prepared from 6-hydrazino-[1,2,5]oxadiazolo[3,4-b]pyrazin-5-ylamine hydrochloride and 2-chloro-5-(5-formyl-furan-2-yl)-benzoic acid.
   ¹H NMR: δ = 7.00 (d, 1 H, *J=* 3.7 Hz), 7.37 (d, 1 H, *J=* 3.7 Hz), 7.67 (dd, 1 H, *J =* 7.3 Hz, *J=* 2.3 Hz), 7.68 (s, 1 H), -7.7 (s, br., 1 H), 7.83-7.88 (m, 1 H), 8.27 (s, br., 1 H), 8.37 (s, 1 H); LC/(+)-ESI-MS: *m*/*z* = 400 [M+H]⁺; LC/(-)-ESI-MS: *m*/*z* = 398 [M-H]⁻.
Example 71: **6-(*N*'-Furan-2-ylmethylene-hydrazino)-[1,2,5]oxadiazolo[3,4-b]pyrazin-5-ylamine** was prepared from 6-hydrazino-[1,2,5]oxadiazolo[3,4-b]pyrazin-5-ylamine hydrochloride and furan-2-carbaldehyde.
   ¹H NMR: δ = 6.72 (dd, 1 *H, J =* 3.5 Hz, *J=* 1.8 Hz), 7.27 (d, 1 H, *J =* 3.5 Hz), 7.62 (s, br., 1 H), 7.94-7.96 (m, 1 H), 8.24 (s, br., 1 H), 8.38 (s, 1 H), 11.91 (s, br., 1 H); LC/(+)-ESI-MS: *m*/*z* = 246 [M+H]⁺; LC/(-)-ESI-MS: *m*/*z* = 244 [M-H]⁻.
Example 72: **6-{*N*'-[5-(4-Chloro-phenyl)-furan-2-ylmethylene]-hydrazino}-[1,2,5]oxadiazolo[3,4-b]pyrazin-5-ylamine** was prepared from 6-hydrazino-[1,2,5]oxadiazolo[3,4-b]pyrazin-5-ylamine hydrochloride and 5-(4-chloro-phenyl)-furan-2-carbaldehyde.
   ¹H NMR: δ = 7.27 (d, 1 H, *J=* 3.6 Hz), 7.35 (d, 1 H, *J=* 3.6 Hz), 7.56 (d, 2 H, *J=* 8.6 Hz), 7.64 (s, br., 1 H), 7.91 (d, 2 H, *J=* 8.6 Hz), 8.27 (s, br., 1 H), 8.40 (s, 1 H), 11.90 (s, br., 1 H); LC/(+)-ESI-MS: *m*/*z* = 356 [M+H]⁺; LC/(-)-ESI-MS: *m*/*z* = 354 [M-H]⁻.
Example 73: **3-[(6-Amino-[1,2,5]oxadiazolo[3,4-b]pyrazin-5-yl)-hydrazono]-1,3-dihydro-indol-2-one** was prepared from 6-hydrazino-[1,2,5]oxadiazolo[3,4-b]pyrazin-5-ylamine hydrochloride and isatin.
   ¹H NMR: δ = 6. 8 (d, 1 H, *J*= 7.7 Hz), 7.00 (ddd, 1 H, *J = J =* 7.7 *Hz, J =* 0.9 Hz), 7.38 (ddd, 1 H, *J= J=* 7.7 Hz, *J =* 1.2 Hz), 7.80 (s, br., 1 H), 8.02 (d, 1 H, *J=* 7.7 Hz), 8.44 (s, br., 1 H), 10.76 (s, 1 H), 12.15 (s, br., 1 H); LC/(+)-ESI-MS: *m*/*z* = 297 [M+H]⁺; LC/(-)-ESI-MS: *m*/*z* = 295 [M-H]⁻.
Example 74: **6-[*N*'-(2-Methyl-1*H*-indol-3-ylmethylene)-hydrazino]-[1,2,5]ozadiazolo[3,4-b]pyrazin-5-ylamine** was prepared from 6-hydrazino-[1,2,5]oxadiazolo[3,4-b]pyrazin-5-ylamine hydrochloride and 2-methyl-1*H*-indole-3-carbaldehyde.
   ¹H NMR: δ = 2.58 (s, 3 H), 7.11-7.19 (m, 2 H), 7.31-7.37 (m, 1 H), 7.62 (s, br., 1 H), 8.13 (s, br., 1 H), 8.54-8.59 (m, 1 H), 8.83 (s, 1 H), 11.75 (s, br., 1 H); LC/(+)-ESI-MS: *m*/*z* = 309 [M+H]⁺; LC/(-)-ESI-MS: *m*/*z* = 307 [M-H]⁻.
Example 75: **2-[(6-Phenylamino-[1,2,5]oxadiazolo[3,4-b]pyrazin-5-yl)-hydrazono-methyl]-benzoic acid** was prepared from (6-hydrazino-[1,2,5]oxadiazolo[3,4-b]pyrazin-5-yl)-phenyl-amine hydrochloride and 2-formyl-benzoic acid.
   ¹H NMR: δ = 7.17 (tt, 1 H, *J* = 7.4 Hz, *J*= 1.1 Hz), 7.42 (dd, 2 H, *J =* 8.5 Hz, *J =* 7.4 Hz), 7.61 (td, 1 H, *J =* 7.5 Hz, *J =* 1.5 Hz), 7.69 (td, 1 H, *J =* 7.5 Hz, *J =* 1.5 Hz), 7.93 ("d", 2 H, *J* ≈ 8.5 Hz), 8.70 (d, 1 H, *J*= 7.7 Hz), 9.38 (s, 1 H), 9.86 (s, br., 1 H), 12.25 (s, br., 1 H), 13.34 (s, br., 1 H); LC/(+)-ESI-MS: *m*/*z* = 376 [M+H]⁺; LC/(-)-ESI-MS: *m*/*z* = 374 [M-H]⁻.
Example 76: **3-[(6-Phenylamino-[1,2,5]oxadiazolo[3,4-b]pyrazin-5-yl)-hydrazono-methyl]-benzoic acid** was prepared from (6-hydrazino-[1,2,5]oxadiazolo[3,4-b]pyrazin-5-yl)-phenyl-amine hydrochloride and 3-formyl-benzoic acid.
   ¹H NMR: δ = 7.19 (tt, 1 H, *J* = 7.4 Hz, *J* = 1.1 Hz), 7.43 (dd, 2 H, *J* = 8.4 Hz, *J* = 7.5 Hz), 7.64 (t, 1 H, *J =* 7.8 Hz), 7.93 (dd, 2 H, *J* = 8.4 Hz, *J* = 1.1 Hz), 8.07 (dt, 1 H, *J =* 7.8 Hz, *J* = 1.4 Hz), 8.39 (dt, 1 H, *J* = 7.8 Hz, *J* = 1.4 Hz), 8.58 (t, 1 H, *J* = 1.4 Hz), 8.76 (s, 1 H), 9.80 (s, br., 1 H), 12.34 (s, br., 1 H), 13.15 (s, br., 1 H); LC/(+)-ESI-MS: *m*/*z* = 376 [M+H]⁺; LC/(-)-ESI-MS: *m*/*z* = 374 [M-H]⁻.
Example 77: **4-[(6-Phenylamino-[1,2,5]oxadiazolo[3,4-b]pyrazin-5-yl)-hydrazono-methyl]-benzoic acid** was prepared from (6-hydrazino-[1,2,5]oxadiazolo[3,4-b]pyrazin-5-yl)-phenyl-amine hydrochloride and 4-formyl-benzoic acid.
   ¹H NMR: δ = 7.19 (tt, 1 H, *J=* 7.4 Hz, *J=* 1.1 Hz), 7.43 (td, *J*= 8.4 Hz, 7.4 Hz), 7.94 (dd, 2 H, *J=* 8.4 Hz, *J*= 1.1 Hz), 8.04 (d, 2 H, *J=* 8.5 Hz), 8.23 (d, 2 H, *J=* 8.5 Hz), 8.76 (s, 1 H), 9.80 (s, br., 1 H), 12.36 (s, br., 1 H), 13.13 (s, br., 1 H); LC/(+)-ESI-MS: *m*/*z* = 376 [M+H]⁺; LC/(-)-ESI-MS: *m*/*z* = 374 [M-H]⁻.
Example 78: **2-[(6-Phenylamino-[1,2,5]ozadiazolo[3,4-b]pyrazin-5-yl)-hydrazono-methyl]-phenol** was prepared from (6-hydrazino-[1,2,5]oxadiazolo[3,4-b]pyrazin-5-yl)-phenyl-amine hydrochloride and 2-hydroxy-benzaldehyde.
   ¹H NMR: δ = 6.94 ("t", 1 H, *J* ≈ 8.5 Hz), 6.97 ("d", 1 H, *J* ≈ 7.5 Hz), 7.18 (tt, 1 H, *J* = 7.4 Hz, *J*= 1.1 Hz), 7.33-7.40 (m, 1 H), 7.36 (t, 1 H, 7.43 ("t", 2 H, *J* ≈ 8 Hz), 7.95 (d, 2 H, *J*= 8.5 Hz, *J*= 1.1 Hz), 8.09 (d, br., 1 H, *J* ≈ 7.5 Hz), 8.97 (s, 1 H), 9.81 (s, br., 1 H), 10.20 (s, br., 1 H), 11.09 (s, br., 1 H), 12.32 (s, br., 1 H); LC/(+)-ESI-MS: *m*/*z* = 348 [M+H]⁺; LC/(-)-ESI-MS: *m*/*z =* 346 [M-H]⁻.
Example 79: **3-[(6-Phenylamino-[1,2,5]oxadiazolo[3,4-b]pyrazin-5-yl)-hydrazono-methyl]-phenol** was prepared from (6-hydrazino-[1,2,5]oxadiazolo[3,4-b]pyrazin-5-yl)-phenyl-amine hydrochloride and 3-hydroxy-benzaldehyde.
   ¹H NMR: δ = 6.90-6.96 (m, 1 H), 7.18 (tt, 1 H, *J* = 7.4 Hz, *J* = 1.1 Hz), 7.29 (t, 1 H, *J* = 7.9 Hz), 7.42 (dd, 2 H, *J* = 8.5 Hz, *J =* 7.4 Hz), 7.46-7.51 (m, 2 H), 7.93 (dd, 2 H, *J* = 8.5 Hz, *J* = 1.1 Hz), 8.59 (s, 1 H), 9.60 (s, br., 1 H), 9.77 (s, br., 1 H), 12.22 (s, br., 1 H); LC/(+)-ESI-MS: *m*/*z* = 348 [M+H]⁺; LC/(-)-ESI-MS: *m*/*z* = 346 [M-H]⁻.
Example 80: **4-[(6-Phenylamino-[1,2,5]oxadiazolo(3,4-b]pyrazin-5-yl)-hydrazono-methyl]-phenol** was prepared from (6-hydrazino-[1,2,5]oxadiazolo[3,4-b]pyrazin-5-yl)-phenyl-amine hydrochloride and 4-hydroxy-benzaldehyde.
   ¹H NMR: δ = 6.87 (d, 2 H, *J* = 8.6 Hz), 7.18 (tt, 1 H, *J =* 7.5 Hz, *J =* 1.1 Hz), 7.42 ("t", 2 H, *J* ≈ 7.9 Hz), 7.93 (dd, 2 H, *J*= 8.5 Hz, J= 1.0 Hz), 7.95 (d, 2 H, *J*= 8.6 Hz), 8.59 (s, 1 H), 9.72 (s, 1 H), 10.11 (s, br., 1 H), 12.09 (s, br., 1 H); LC/(+)-ESI-MS: *m*/*z* = 348 [M+H]⁺; LC/(-)-ESI-MS: *m*/*z* = 346 [M-H]⁻.
Example 81: **4-[(6-Phenylamino-[1,2,5]oxadiazolo[3,4-b]pyrazin-5-yl)-hydrazono-methyl]-benzene-1,3-diol** was prepared from (6-hydrazino-[1,2,5]oxadiazolo[3,4-b]pyrazin-5-yl)-phenyl-amine hydrochloride and 2,4-dihydroxy-benzaldehyde.
   ¹H NMR: δ = 6.36 (t, 1 H, *J=* 2.1 Hz), 6.38 (dd, 1 H, *J=* 8.3 Hz, *J*= 2.2 Hz), 7.17 (t, 1 H, *J*= 7.5 Hz), 7.42 (dd, 2 H, *J =* 8.5 Hz, *J =* 7.5 Hz), 7.79 (d, br., 1 H, *J* = 8.3 Hz), 7.94 ("d", 2 H, J= 8.5 Hz), 8.82 (s, br., 1 H), 9.75 (s, br., 1 H), 10.09 (s, br., 1 H), 10.18 (s, br., 1 H), 12.21 (s, br., 1 H); LC/(+)-ESI-MS: *m*/*z* = 364 [M+H]⁺; LC/(-)-ESI-MS: *m*/*z* = 362 [M-H]-.
Example 82: **5-Methoxy-2-[(6-phenylamino-[1,2,5]oxadiazolo[3,4-b]pyrazin-5-yl)-hydrazoriomethyl]-phenol** was prepared from (6-hydrazino-[1,2,5]oxadiazolo[3,4-b]pyrazin-5-yl)-phenyl-amine hydrochloride and 2-hydroxy-4-methoxy-benzaldehyde.
   ¹H NMR: δ = 3.80 (s, 3 H), 6.50 (d, 1 H, *J=* 2.4 Hz), 6.56 (dd, 1 H, *J=* 8.7 Hz, *J=* 2.4 Hz), 7.17 (tt, 1 H, *J* = 7.5 *Hz, J =* 1.1 Hz), 7.42 (dd, 2 H, *J=* 8.6 *Hz, J =* 7.5 Hz), 7.90 (s, br., 1 H), 7.94 (dd, 2 H, *J=* 8.6 Hz, *J=* 1.2 Hz), 8.87 (s, 1 H), 9.77 (s, 1 H), 10.34 (s, br., 1 H), 12.27 (s, br., 1 H); LC/(+)-ESI-MS: *m*/*z* = 378 [M+H]⁺; LC/(-)-ESI-MS: *m*/*z* = 376 [M-H]⁻.
Example 83: **4-Methoxy-2-[(6-phenylamino-[1,2,5]oxadiazolo[3,4-blpyrazin-5-yl)-hydrazonomethyl]-phenol** was prepared from (6-hydrazino-[1,2,5]oxadiazolo[3,4-b]pyrazin-5-yl)-phenyl-amine hydrochloride and 2-hydroxy-5-methoxy-benzaldehyde.
   ¹H NMR: δ = 3.78 (s, 3 H), 6.88 (d, 1 H, *J=* 8.9 Hz), 6.98 (dd, 1 H, *J =* 8.9 Hz, *J* = 3.0 Hz), 7.18 (tt, 1 H, *J =* 7.4 Hz, *J =* 1.1 Hz), 7.42 (dd, 2 H, *J* = 8.6 Hz, *J =* 7.4 Hz), 7.71 (s, br., 1 H), 7.96 (dd, 2 H, J= 8.6 Hz, J= 1.1 Hz), 8.95 (s, br., 1 H), 9.81 (s, br., 2 H), 12.23 (s, br., 1 H); LC/(+)-ESI-MS: *m*/*z* = 378 [M+H]⁺; LC/(-)-ESI-MS: *m*/*z* = 376 [M-H]⁻.
Example 84: **2-Methoxy-6-[(6-phenylamino-[1,2,5]oxadiazolo[3,4-b]pyrazin-5-yl)-hydrazonomethyl]-phenol** was prepared from (6-hydrazino-[1,2,5]oxadiazolo[3,4-b]pyrazin-5-yl)-phenyl-amine hydrochloride and 2-hydroxy-3-methoxy-benzaldehyde.
   ¹H NMR: δ = 3.84 (s, 3 H), 6.89 (t, 1 H, *J=* 7.9 Hz), 7.10 (dd, 1 H, *J=* 7.9 Hz, *J =* 1.3 Hz), 7.18 (tt, 1 H, 1 H, *J* = 7.4 Hz, *J* = 1.1 Hz), 7.42 (dd, 2 H, *J* = 8.5 Hz, *J =* 7.4 Hz), 7.69 (d, br., 1 H, *J=* 7.9 Hz), 7.96 (dd, 2 H, *J=* 8.5 Hz, *J=* 1.1 Hz), 8.99 (s, 1 H), 9.74 (s, br., 1 H), 9.81 (s, br., 1 H), 12.31 (s, br., 1 H); LC/(+)-ESI-MS: *m*/*z* = 378 [M+H]⁺; LC/(-)-ESI-MS: *m*/*z* = 376 [M-H]⁻.
Example 85: **2,4-Dibromo-6-[(6-phenylamino-[1,2,5]oxadiazolo[3,4-blpyrazin-5-yl)-hydrazonomethyl]-phenol** was prepared from (6-hydrazino-[1,2,5]oxadiazolo[3,4-b]pyrazin-5-yl)-phenyl-amine hydrochloride and 3,5-dibromo-2-hydroxy-benzaldehyde.
   ¹H NMR: δ = 7.19 (tt, 1 H, *J =* 7.4 Hz, *J =* 1.1 Hz), 7.43 (dd, 2 H, *J* = 8.5 Hz, *J* = 7.4 Hz), 7.91 (d, 1 H, *J=* 2.4 Hz), 7.92 (dd, 2 H, *J=* 8.5 Hz, *J=* 1.1 Hz), 8.15 (d, 1 H, *J=* 2.4 Hz), 8.89 (s, 1 H), 9.83 (s, 1 H), 10.83 (s, br., 1 H), 12.56 (s, br., 1 H); LC/(+)-ESI-MS: *m*/*z* = 506 [M+H]⁺; LC/(-)-ESI-MS: *m*/*z* = 504 [M-H]⁻.
Example 86: **4-Bromo-2-methoxy-6-[(6-phenylamino-[1,2,5]oxadiazolo[3,4-b]pyrazin-5-yl)-hydrazonomethyl]-phenol** was prepared from (6-hydrazino-[1,2,5]oxadiazolo[3,4-b]pyrazin-5-yl)-phenyl-amine hydrochloride and 5-bromo-2-hydroxy-3-methoxy-benzaldehyde.
   ¹H NMR: δ = 3.87 (s, 3 H), 7.18 (tt, 1 H, *J* = 7.4 Hz, *J* = 1.1 Hz), 7.22 (d, 1 H, *J* = 2.3 Hz), 7.42 (dd, 2 H, *J=* 8.5 Hz, *J=* 7.4 Hz), 7.95 (dd, 2 H, *J=* 8.5 Hz, *J=* 1.1 Hz), 8.06 (s, br., 1 H), 8.96 (s, br., 1 H), 9.81 (s, br., 1 H), 9.88 (s, br., 1 H), 12.29 (s, br., 1 H); LC/(+)-ESI-MS: *m*/*z* = 456 [M+H]⁺; LC/(-)-ESI-MS: *m*/*z* = 454 [M-H]⁻.
Example 87: **4-Chloro-2-[(6-phenylamino-[1,2,5]oxadiazolo[3,4-b]pyrazin-5-yl)-hydrazonomethyl]-phenol** was prepared from (6-hydrazino-[1,2,5]oxadiazolo[3,4-b]pyrazin-5-yl)-phenyl-amine hydrochloride and 5-chloro-2-hydroxy-benzaldehyde.
   ¹H NMR: δ = 6.96 (d, 1 H, *J=* 8.8 Hz), 7.18 (tt, 1 H, *J =* 7.4 Hz, *J =* 1.1 Hz); 7.36 (dd, 1 H, *J =* 8.8 Hz, *J=* 2.8 Hz), 7.42 (dd, 2 H, *J =* 8.5 Hz, *J=* 7.4 Hz), 7.95 (dd, 2 H, *J=* 8.5 Hz, *J* = 1.1 Hz), 8.34 (s, br., 1 H), 8.93 (s, 1 H), 9.81 (s, 1 H), 10.51 (s, br., 1 H), 12.30 (s, br., 1 H); LC/(+)-ESI-MS: *m*/*z* = 382 [M+H]⁺; LC/(-)-ESI-MS: *m*/*z* = 380 [M-H]-.
Example 88: **[6-(N-Benzofuran-2-ylmethylene-hydrazino)-[1,2,5]oxadiazolo[3,4-b]pyrazin-5-yl]-phenyl-amine** was prepared from (6-hydrazino-[1,2,5]oxadiazolo[3,4-b]pyrazin-5-yl)-phenyl-amine hydrochloride and benzofuran-2-carbaldehyde.
   ¹H NMR: δ = 7.19 (tt, 1 H, *J =* 7.4 Hz, *J =* 1.1 Hz), 7.35 (ddd, 1 H, *J = J =* 7.5 Hz, *J=* 1.0 Hz), 7.40-7.50 (m, 4 H), 7.68 ("dq", 1 H, J= 8.3 Hz, *J*= 1.0 Hz), 7.71 (s, br., 1 H), 7.80 ("dq", 1 H, *J =* 7.7 Hz, *J =* 0.6 Hz), 7.94 (dd, 2 H, J = 8.6 Hz, *J =* 1.1 Hz), 8.69 (s, 1 H), 9.83 (s, br., 1 H), 12.32 (s, br., 1 H); LC/(+)-ESI-MS: *m*/*z* = 372 [M+H]⁺; LC/(-)-ESI-MS: *m*/*z=* 370 [M-H]⁻.
Example 89: **3-{5-[(6-Phenylamino-[1,2,5]oxadiazolo[3,4-b]pyrazin-5-yl)-hydrazonomethyl]-furan-2-yl)-benzoic** acid was prepared from (6-hydrazino-[1,2,5]oxadiazolo[3,4-b]pyrazin-5-yl)-phenyl-amine hydrochloride and 3-(5-formyl-furan-2-yl)-benzoic acid.
   ¹H NMR: δ = 7.19 (tt, 1 H, *J* = 7.4 *Hz, J =* 1.1 Hz), 7.37 (d, 1 H, *J* = 3.6 Hz), 7.42 (d, 1 H, *J =* 3.6 Hz), 7.43 ("t", 2 H, *J* ≈ 8 Hz), 7.64 (t, 1 *H, J =* 7.8 Hz), 7.91-7.97 (m, 3 H), 8.15 (dt, 1 H, *J*= 8.4 Hz, *J*= 1.8 Hz), 8.41 (t, 1 H, *J*= 1.8 Hz), 8.58 (s, 1 H), 9.79 (s, 1 H), 12.19 (s, br., 1 H), 13.17 (s, br., 1 H); LC/(+)-ESI-MS: *m*/*z* = 442 [M+H]⁺; LC/(-)-ESI-MS: *m*/*z* = 440 [M-H]⁻.
Example 90: **(6-{*N*'-[5-(3-Nitro-phenyl)-furan-2-ylmethylene]-hydrazino}-[1,2,5]ozadiazolo[3,4-b]pyrazin-5-yl)-phenyl-amine** was prepared from (6-hydrazino-[1,2,5]oxadiazolo[3,4-b]pyrazin-5-yl)-phenyl-amine hydrochloride and 5-(3-nitro-phenyl)-furan-2-carbaldehyde.
   ¹H NMR: δ = 7.20 (tt, 1 H, *J =* 7.4 Hz, *J =* 1.1 Hz), 7.44 ("t", 2 H, *J ≈* 8 Hz), 7.46 (d, 1 H, *J=* 3.6 Hz), 7.54 (d, 1 H, *J*= 3.6 Hz), 7.82 (t, 1 H, *J*= 8.0 Hz), 7.94 ("d", 2 H, *J ≈* 8 Hz), 8.23 (ddd, 1 H, *J =* 8.2 Hz, *J =* 2.3 Hz, *J* = 0.9 Hz), 8.3 5 ("d", 1 H, *J ≈* 8 Hz), 8.60 (s, 1 H), 8.66 (t, 1 H, J= 1.9 Hz), 9.80 (s, br., 1 H), 12.21 (s, br., 1 H); LC/(+)-ESI-MS: *m*/*z* = 443 [M+H]⁺; LC/(-)-ESI-MS: *m*/*z* = 441 [M-H]⁻.
Example 91: **Phenyl-{6-[N'-(5-phenyl-furan-2-ylmethylene)-hydrazino]-[1,2,5]oxa-diazolo[3,4-b]pyrazin-5-yl}-amine** was prepared from (6-hydrazino-[1,2,5]oxadiazolo[3,4-b]pyrazin-5-yl)-phenyl-amine hydrochloride and 5-phenyl-furan-2-carbaldehyde.
   ¹H NMR: δ = 7.19 (tt, 1 H, *J* = 7.4 Hz, *J* = 1.1 Hz), 7.24 (d, 1 H, *J* = 3.6 Hz), 7.36-7.43 (m, 1 H), 7.39 (d, 1 H, *J*= 3.6 Hz), 7.43 ("t", 2 H, *J ≈* 8 Hz), 7.51 (t, 2 H, *J=* 7.3 Hz), 7.89-7.97 (m, 4 H), 8.57 (s, 1 H), 9.78 (s, br., 1 H), 12.11 (s, br., 1 H); LC/(+)-ESI-MS: *m*/*z* = 398 [M+H]⁺.
Example 92: **2-Chloro-5-{5-[(6-phenylamino-[1,2,5]ozadiazolo[3,4-b]pyrazin-5-yl)-hydrazonomethyl]-furan-2-yl}-benzoic acid** was prepared from (6-hydrazino-[1,2,5]oxadiazolo[3,4-b]pyrazin-5-yl)-phenyl-amine hydrochloride and 2-chloro-5-(5-formyl-furan-2-yl)-benzoic acid.
   ¹H NMR: δ = 7.02 (d, 1 H, *J* =3.7 Hz), 7.18 (tt, 1 H, *J* = 7.4 Hz, *J* = 1.1 Hz), 7.41 (d, 1 H, *J =* 3.7 Hz), 7.43 ("t", 2 H, *J* ≈ 8 Hz), 7.71 (dd, 1 H, *J ≈* 8 Hz, *J =* 2.3 Hz), 7.72 (m, 1 H), 7.87 (d, 1 H, *J=* 8.0 Hz), 7.94 ("d", 2 H, *J ≈* 8.5 Hz), 8.54 (s, 1 H), 9.78 (s, br., 1 H), 12.07 (s, br., 1 H), 13.51 (s, br., 1 H); LC/(+)-ESI-MS: *m*/*z* = 476 [M+H]⁺.
Example 93: **[6-(*N*'-Furan-2-ylmethylene-hydrazino)-[1,2,5]oxadiazolo[3,4-b]pyrazin-5-yl]-phenyl-amine** was prepared from (6-hydrazino-[1,2,5]oxadiazolo[3,4-b]pyrazin-5-yl)-phenyl-amine hydrochloride and furan-2-carbaldehyde.
   ¹H NMR: δ = 6.75 (dd, 1 H, *J =* 3.5 Hz, *J* = 1.7 Hz), 7.18 (tt, 1 H, *J*= 7.4 Hz, *J =* 1.1 Hz), 7.30 (d, 1 H, *J* = 3.5 Hz), 7.42 (dd, 2 H, *J =* 8.5 Hz, *J* = 7.4 Hz), 7.92 (dd, 2 H, *J* = 8.5 Hz, *J=* 1.1 Hz), 7.99 (dd, 1 *H, J=* 1.7 *Hz, J=* 0.7 Hz), 8.54 (s, 1 H), 9.76 (s, br., 1 H), 12.14 (s, br., 1 H); LC/(+)-ESI-MS: *m*/*z =* [M+H]⁺; LC/(-)-ESI-MS: *m*/*z =* [M-H]⁻.
Example 94: **3-{5-[(6-Phenylamino-[1,2,5]oxadiazolo[3,4-b]pyrazin-5-yl)-hydrazono-methyl]-furan-2-yl}-benzamide** was prepared from (6-hydrazino-[1,2,5]oxadiazolo[3,4-b]pyrazin-5-yl)-phenyl-amine hydrochloride and 3-(5-formyl-furan-2-yl)-benzamide.
   ¹H NMR: δ = 7.19 (tt, 1 H, *J* = 7.4 Hz, *J* =1.1 Hz), 7.31 (d, 1 H, *J* = 3.6 Hz), 7.40-7.49 (m, 4 H), 7.59 (t, 1 H, *J*= 7.8 Hz), 7.87 (dt, 1 H, *J*= 8.1 Hz, *J*= 1.3 Hz), 7.94 (dd, 2 H, *J*= 8.5 Hz, *J*= 1.1 Hz), 8.05 ("d", 1 H, *J* ≈ 8 Hz), 8.36 (t, 1 H, *J=* 1.5 Hz), 8.58 (s, 1 H), 9.79 (s, br., 1 H), 12.14 (s, br., 1 H); LC/(+)-ESI-MS: *m*/*z* = 441 [N4+H]⁺.
Example 95: **3-[(6-Phenylamino-[1,2,5]oxadiazolo[3,4-b]pyrazin-5-yl)-hydrazono]-1,3-dihydro-indol-2-one** was prepared from (6-hydrazino-[1,2,5]oxadiazolo[3,4-b]pyrazin-5-yl)-phenyl-amine hydrochloride and isatin.
   ¹H NMR: δ = 6.90 (d, 1 H, 7.7 Hz), 7.03 (td, 1 H, *J*= 7.7 Hz, *J*= 0.9 Hz), 7.21 (tt, 1 H, *J*= 7.4 Hz, *J =* 1.1 Hz), 7.39 (td, 1 H, *J =* 7.7 Hz, *J =* 1.3 Hz), 7.44 (dd, 2 H, *J* = 8.5 Hz, *J =* 7.4 Hz), 7.94 (dd, 2 H, *J* = 8.5 Hz, *J* = 1.1 Hz), 8.00 (d, 1 H, *J* = 7.7 Hz), 9.93 (s, br., 1 H), 10.80 (s, br, 1 H), 12.29 (s, br., 1 H); LC/(+)-ESI-MS: *m*/*z* = 373 [M+H]⁺.
Example 96: **{6-[*N*'-(1*H*-Indol-3-ylmethylene)-hydrazino]-[1,2,5]oxadiazolo[3,4-b]pyrazin-5-yl}-phenyl-amine** was prepared from (6-hydrazino-[1,2,5]oxadiazolo[3,4-b]pyrazin-5-yl)-phenyl-amine hydrochloride and 1*H*-indole-3-carbaldehyde.
   ¹H NMR: δ = 7.18 (tt, 1 H, *J =* 7.4 Hz, *J =* 1.1 Hz), 7.21 (ddd, 1 H, *J =* 7.3 Hz, *J =* 7.3 Hz, *J =* 1.7 Hz), 7.26 (ddd, 1 H, *J =* 7.3 Hz, *J =* 7.3 Hz, *J* = 1.7 Hz), 7.43 (dd, 2 H, *J =* 8.5 Hz, *J* = 7.4 Hz), 7.46-7.50 (m, 1 H), 7.95 (dd, 2 H, *J*= 8.5 Hz, J= 1.1 Hz), 8.08 (d, 1 H, *J=* 2.9 Hz), 8.63 (dd, 1 H, *J*= 6.5 Hz, *J*= 2.0 Hz), 8.92 (s, 1 H), 9.76 (s, br., 1 H), 11.89 (s, br., 1 H), 11.74 (s, br., 1 H), 11.89 (s, br., 1 H); LC/(+)-ESI-MS: *m*/*z* = 371 [M+H]⁺; LC/(-)-ESI-MS: *m*/*z* = 369 [M-H]-.
Example 97: **2-[(6-Benzylamino-[1,2,5]oxadiazolo[3,4-b]pyrazin-5-yl)-hydrazono-methyl]-benzoic acid** was prepared from benzyl-(6-hydrazino-[1,2,5]oxadiazolo[3,4-b]pyrazin-5-yl)-amine hydrochloride and 2-formyl-benzoic acid.
   ¹H NMR: δ = 4.68 (d, 2 H, *J.=* 6.3 Hz), 7.24 (tt, 1 H, *J=* 7.0 Hz, *J* ≈ 1.6 Hz), 7.32 ("t", 2 H, *J* ≈ 7.3 Hz), 7.38 ("d", 2 H, *J ≈* 7.3 Hz), 7.60 ("td", 1 H, *J =* 7.5 Hz, *J =* 1.5 Hz), 7.67 ("td", 1 H, *J* = 7.5 Hz, *J =* 1.1 Hz), 7.92 (dd, 1 H, *J* = 7.6 Hz, *J =* 1.1 Hz), 8.69 (d, br., 1 H, *J=* 7.6 Hz), 8.96 (t, br., 1 H, *J=* 6.3 Hz), 9.32 (s, 1 H), 12.17 (s, br., 1 H), 13.38 (s, br., 1 H).
Example 98: **3-[(6-Benzylamino-[1,2,5]oxadiazolo[3,4-b]pyrazin-5-yl)-hydrazono-methyl]-benzoic** acid was prepared from benzyl-(6-hydrazino-[1,2,5]oxadiazolo[3,4-b]pyrazin-5-yl)-amine hydrochloride and 3-formyl-benzoic acid.
   ¹H NMR: δ = 4.69 (d, 2 H, *J=* 6.3 Hz), 7.25 (tt, 1 H, *J=* 7.0 Hz, *J ≈* 1.6 Hz), 7.33 ("t", 2 H, *J* ≈ 7.3 Hz), 7.38 ("d", 2 H, *J* ≈ 7.3 Hz), 7.62 (dd, 1 H, *J = J =* 7.8 Hz), 8.05 (ddd, 1 H, *J* = 7.8 Hz, *J= J=* 1.6 Hz), 8.38 ("d", 1 H, *J=* 7.8 Hz), 8.54 ("s", 1 H), 8.65 (s, 1 H), 8.82 (t, br., 1 H, *J*= 6.3 Hz), 12.22 (s, br., 1 H), 13.14 (s, br., 1 H).
Example 99: **4-[(6-Benzylamino-[1,2,5]ozadiazolo[3,4-b]pyrazin-5-yl)-hydrazono-methyl]-benzoic acid** was prepared from benzyl-(6-hydrazino-[1,2,5]oxadiazolo[3,4-b]pyrazin-5-yl)-amine hydrochloride and 4-formyl-benzoic acid.
   ¹H NMR: δ = 4.69 (d, 2 H, *J*= 6.3 Hz), 7.25 (tt, 1 H, *J=* 7.0 Hz, *J ≈* 1.6 Hz), 7.33 ("t", 2 H, *J* ≈ 7.3 Hz), 7.39 ("d", 2 H, *J* ≈ 7.3 Hz), 8.02 (d, 2 H, *J* = 8.4 Hz), 8.20 (d, 2 H, *J* = 8.4 Hz), 8.64 (s, 1 H), 8.84 (t, br., 1 H, *J*= 6.3 Hz), 12.27 (s, br., 1 H), 13.08 (s, br., 1 H). LC/(+)-ESI-MS: *m*/*z* = 390 [M+H]⁺.
   LC/(-)-ESI-MS: *m*/*z* = 388 [M-H]⁻.
Example 100: **2-[(6-Benzylamino-[1,2,5]oxadiazolo[3,4-b]pyrazin-5-yl)-hydrazono-methyl]-phenol** was prepared from benzyl-(6-hydrazino-[1,2,5]oxadiazolo[3,4-b]pyrazin-5-yl)-amine hydrochloride and 2-hydroxy-benzaldehyde.
   ¹H NMR: δ = 4.68 (d, 2 H, J= 6.3 Hz), 6.87-6.96 (m, 2 H), 7.24 (tt, 1 H, J= 7.0 Hz, *J* ≈ 1.6 Hz), 7.33 ("t", 2 H, *J* ≈ 7.3 Hz), ~ 7.34 (m, 1 H), 7.38 ("d", 2 H, *J* ≈ 7.3 Hz), 8.02 (d, br., 1 H, *J* ≈ 8 Hz), 8.83 (s, br., 2 H), 10.17 (s, br., 1 H), 12.21 (s, br., 1 H); LC/(+)-ESI-MS: *m*/*z* = 362 [M+H]⁺; LC/(-)-ESI-MS: *m*/*z* = 360 [M-H]⁻.
Example 101: **3-[(6-Benzylamino-[1,2,5]oxadiazolo[3,4-b]pyrazin-5-yl)-hydrazono-methyl]-phenol** was prepared from benzyl-(6-hydrazino-[1,2,5]oxadiazolo[3,4-b]pyrazin-5-yl)-amine hydrochloride and 3-hydroxy-benzaldehyde.
   ¹H NMR: δ = 4.68 (d, 2 H, *J* = 6.3 Hz), 6.91 ("d", 1 H, *J* ≈ 7.9 Hz), 7.21-7.41 (m, 6 H), 7.44-7.50 (m, 2 H), 8.48 (s, 1 H), 8.78 (t, br., 1 H, J= 6.3 Hz), 9.58 (s, 1 H), 12.09 (s, br., 1 H); LC/(+)-ESI-MS: *m*/*z* = 362 [M+H]⁺; LC/(-)-ESI-MS: *m*/*z* = 360 [M-H]⁻.
Example 102: **4-[(6-Benzylamino-[1,2,5]oxadiazolo[3,4-b]pyrazin-5-yl)-hydrazono-methyl]-phenol** was prepared from benzyl-(6-hydrazino-[1,2,5]oxadiazolo[3,4-b]pyrazin-5-yl)-amine hydrochloride and 4-hydroxy-benzaldehyde.
   ¹H NMR: δ = 4.67 (d, 2 H, *J*= 6.3 Hz), 6.85 (d, 2 H, *J*= 8.6 Hz), 7.24 (tt, 1 H, *J=* 7.0 Hz, *J* ≈ 1.6 Hz), 7.32 ("t", 2 H, *J* ≈ 7.3 Hz), 7.38 ("d", 2 H, *J* ≈ 7.3 Hz), 7.91 (d, 2 H, *J*= 8.6 Hz), 8.47 (s, 1 H), 8.74 (t, br., 1 H, J= 6.3 Hz), 10.06 (s, br., 1 H), 11.96 (s, br., 1 H); LC/(+)-ESI-MS: *m*/*z* = 362 [M+H]⁺; LC/(-)-ESI-MS: *m*/*z* = 360 [M-H]⁻.
Example 103: **4-[(6-Benzylamino-[1,2,5]oxadiazolo[3,4-b]pyrazin-5-yl)-hydrazono-methyl]-benzene-1,3-diol** was prepared from benzyl-(6-hydrazino-[1,2,5]oxadiazolo[3,4-b]pyrazin-5-yl)-amine hydrochloride and 2,4-dihydroxy-benzaldehyde.
   ¹H NMR: δ = 4.67 (d, 2 H, *J =* 6.3 Hz), 6.32-6.35 (m, 1 H), 6.36 (dd, 1 H, *J =* 8.4 Hz, *J* = 2.2 Hz), 7.24 (tt, 1 H, *J* = 7.0 Hz, *J ≈* 1.6 Hz), 7.32 ("t", 2 H, *J* ≈ 7.3 Hz), 7.37 ("d", 2 H, *J* ≈ 7.3 Hz), 7.76 (d, 1 H, *J =* 8.4 Hz), 8.71 (s, 1 H), 8.78 (t, br., 1 H, *J=* 6.3 Hz), 10.08 (s, 1 H), 10.13 (s, br., 1 H), 12.09 (s, br., 1 H); LC/(+)-ESI-MS: *m*/*z* = 378 [M+H]⁺; LC/(-)-ESI-MS: *m*/*z* = 376 [M-H]⁻.
Example **104: 2-[(6-Benzylamino-[1,2,5]oxadiazolo[3,4-b]pyrazin-5-yl)-hydrazono-methyl)-5-methoxy-phenol** was prepared from benzyl-(6-hydrazino-[1,2,5]oxadiazolo[3,4-b]pyrazin-5-yl)-amine hydrochloride and 2-hydroxy-4-methoxy-benzaldehyde.
   ¹H NMR: δ = 3.79 (s, 3 H), 4.67 (d, 2 H, J= 6.3 Hz), 6.49 (d, 1 H, *J*= 2.4 Hz); 6.54 (dd, 1 H, *J* = 8.6 Hz, *J* = 2.4 Hz), 7.24 (tt, 1 H, *J =* 7.0 Hz, *J* ≈ 1.6 Hz), 7.32 ("t", 2 H, *J* ≈ 7.3 Hz), 7.37 ("d", 2 H, *J ≈* 7.3 Hz), 7.87 (d, 1 H, *J=* 8.6 Hz), 8.74 (s, 1 H), 8.79 (t, br., 1 H, *J* = 6.3 Hz), 10.29 (s, br., 1 H), 12.15 (s, br., 1 H); LC/(+)-ESI-MS: *m*/*z* = 392 [M+H]⁺; LC/(-)-ESI-MS: *m*/*z* = 390 [M-H]⁻.
Example 105: **2-[(6-Benzylamino-[1,2,5]ozadiazolo[3,4-b]pyrazin-5-yl)-hydrazono-methyl]-4-methoxy-phenol** was prepared from benzyl-(6-hydrazino-[1,2,5]oxadiazolo[3,4-b]pyrazin-5-yl)-amine hydrochloride and 2-hydroxy-5-methoxy-benzaldehyde.
   ¹H NMR: δ = 3.76 (s, 3 H), 4.68 (d, 2 H, *J*= 6.3 Hz), 6.86 (d, 2 H, *J*= 8.9 Hz), 6.96 (dd, 1 H, *J=* 8.9 Hz, *J=* 3.1 Hz), 7.24 (tt, 1 H, *J=* 7.0 Hz, *J ≈* 1.6 Hz), 7.33 ("t", 2 H, *J* ≈ 7.3 Hz), 7.38 ("d", 2 H, *J ≈* 7.3 Hz), 7.67 ("s", br., 1 H), 8.83 (s, br., 1 H), 8.85 (t, br., 1 H, *J* ≈ 6 Hz), 9.73 (s, br., 1 H), 12.12 (s, br., 1 H); LC/(+)-ESI-MS: *m*/*z* = 392 [M+H]⁺; LC/(-)-ESI-MS: *m*/*z* = 390 [M-H]⁻.
Example 106: **2-[(6-Benzylamino-[1,2,5]oxadiazolo[3,4-b]pyrazin-5-yl)-hydrazono-methyl]-6-methoxy-phenol** was prepared from benzyl-(6-hydrazino-[1,2,5]oxadiazolo[3,4-b]pyrazin-5-yl)-amine hydrochloride and 2-hydroxy-3-methoxy-benzaldehyde.
   ¹H NMR: δ = 3.83 (s, 3 H), 4.68 (d, 2 H, *J=* 6.3 Hz), 6.87 (8.0 Hz), 7.09 (dd, 1 H, *J=* 8.0 Hz, *J =* 1.3 Hz), 7.24 (tt, 1 H, *J =* 7.0 Hz, *J* ≈ 1.6 Hz), 7.33 ("t", 2 H, *J ≈* 7.3 Hz), 7.38 ("d", 2 H, *J ≈* 7.3 Hz), 7.64 (d, 1 H, *J=* 8.0 Hz), 8.86 (s, br., 1 H), 8.86 (t, br., 1 H, *J ≈* 6 Hz), 9.71 (s, br., 1 H), 12.20 (s, br., 1 H); LC/(+)-ESI-MS: *mz*/*z =* 392 [M+H]⁺; LC/(-)-ESI-MS: *m*/*z* = 390 [M-H]-.
Example 107: **2-[(6-Benzylamino-[1,2,5]ozadiazolo[3,4-b]pyrazin-5-yl)-hydrazono-methyl]-4,6-dibromo-phenol** was prepared from benzyl-(6-hydrazino-[1,2,5]oxadiazolo[3,4-b]pyrazin-5-yl)-amine hydrochloride and 3,5-dibromo-2-hydroxy-benzaldehyde.
   ¹H NMR: δ = 4.69 (d, 2 H, *J*= 6.3 Hz), 7.25 (tt, 1 H, *J*= 7.0 Hz, *J* ≈ 1.6 Hz), 7.33 ("t", 2 H, *J* ≈ 7.3 Hz), 7.38 ("d", 2 H, *J* ≈ 7.3 Hz), 7.90 (d, 1 H, *J =* 2.4 Hz), 8.12 (d, 1 H, *J =* 2.4 Hz), 8.77 (s, 1 H), 8.86 (t, br., 1 H, *J*= 6.3 Hz), 10.77 (s, br., 1 H), 12.46 (s, br., 1 H); LC/(+)-ESI-MS: *m*/*z* = 520 [M+H]⁺; LC/(-)-ESI-MS: *m*/*z* = 518 [M-H]⁻.
Example 108: **2-[(6-Benzylamino-[1,2,5]oxadiazolo[3,4-b]pyrazin-5-yl)-hydrazono-methyl]-4-bromo-6-methoxy-phenol** was prepared from benzyl-(6-hydrazino-[1,2,5]oxadiazolo[3,4-b]pyrazin-5-yl)-amine hydrochloride and 5-bromo-2-hydroxy-3-methoxy-benzaldehyde.
   ¹H NMR: δ = 3.85 (s, 3 H), 4.67 (d, 2 H, *J*= 6.3 Hz), 7.19 ("s", br., 1 H), 7.24 (tt, 1 H, *J =* 7.0 Hz, *J ≈* 1.6 Hz), 7.32 ("t", 2 H, *J* ≈ 7.3 Hz), 7.37 ("d", 2 H, *J ≈* 7.3 Hz), 7.98 ("s", br., 1 H), 8.74-8.91 (m, 2 H), 9.87 (s, br., 1 H), 12.19 (s, br., 1 H); LC/(+)-ESI-MS: *m*/*z* = 470 [M+H]⁺; LC/(-)-ESI-MS: *m*/*z* = 468 [M-H]⁻.
Example 109: **2-[(6-Benzylamino-[1,2,5]oxadiazolo[3,4-b]pyrazin-5-yl)-hydrazono-methyl]-4-chloro-phenol** was prepared from benzyl-(6-hydrazino-[1,2,5]oxadiazolo[3,4-b]pyrazin-5-yl)-amine hydrochloride and 5-chloro-2-hydroxy-benzaldehyde.
   ¹H NMR: δ = 4.68 (d, 2 H, *J*= 6.3 Hz), 6.94 (d, 1 H, *J*= 8.8 Hz), 7.24 (tt, 1 H, *J*= 7.0 Hz, *J ≈* 1.6 Hz), 7.33 ("t", 2 H, *J ≈* 7.3 Hz), 7.38 ("d", 2 H, *J ≈* 7.3 Hz), 8.29 ("s", br., 1 H), 8.81 (s, 1 H), 8.87 (t, br., 1 H, *J*= 6.3 Hz), 10.47 (s, br., 1 H), 12.19 (s, br., 1 H); LC/(+)-ESI-MS: *m*/*z* = 396 [M+H]⁺; LC/(-)-ESI-MS: *m*/*z* = 394 [M-H]⁻.
Example 110: **[6-(*N*'-Benzofuran-2-ylmethylene-hydrazino)-[1,2,5]oxadiazolo[3,4-b]pyrazin-5-yl]-benzyl-amine** was prepared from benzyl-(6-hydrazino-[1,2,5]oxadiazolo[3,4-b]pyrazin-5-yl)-amine hydrochloride and benzofuran-2-carbaldehyde.
   ¹H NMR: δ = 4.69 (d, 2 H, *J*= 6.3 Hz), 7.25 (tt, 1 H, *J*= 7.0 Hz, *J* ≈ 1.6 Hz), 7.33 ("t", 2 H, *J* ≈ 7.3 Hz), 7.39 ("d", 2 H, *J* ≈ 7.3 Hz), 7.46 (ddd, 1 H, *J* = 8.3 Hz, *J* = 7.3 Hz, *J* = 1.3 Hz), 7.66 ("d", 1 H, *J* ≈ 8.3 Hz), 7.69 ("s", 1 H), 7.78 ("d", *J* ≈ 7.3 Hz), 8.56 (s, 1 H), 8.86 (t, br., 1 H, *J*= 6.3 Hz), 12.18 (s, br., 1 H); LC/(+)-ESI-MS: *m*/*z* = 386 [M+H]⁺; LC/(-)-ESI-MS: *m*/*z* = 384 [M-H]⁻.
Example 111: **3-{5-[(6-Benzylamino-[1,2,5]ozadiazolo[3,4-b]pyrazin-5-yl)-hydrazonomethyl]-furan-2-yl}-benzoic acid** was prepared from benzyl-(6-hydrazino-[1,2,5]oxadiazolo[3,4-b]pyrazin-5-yl)-amine hydrochloride and 3-(5-formyl-furan-2-yl)-benzoic acid.
   ¹H NMR: δ = 4.69 (d, 2 H, *J=* 6.3 Hz), 7.25 (tt, 1 H, *J=* 7.0 Hz, *J* ≈ 1.6 Hz), 7.29-7.42 (m, 6 H), 7.62 (dd, 1 H, *J= J=* 7.8 Hz), 7.93 (ddd, 1 H, *J=* 7.9 Hz, *J= J=* 1.3 Hz), 8.13 (ddd, 1 H, *J* = *7.9 Hz, J= J=* 1.3 Hz), 8.39 (dd, *J= J=* 1.5 Hz), 8.45 (s, 1 H), 8.81 (t, br., 1 *H, J* = 6.3 Hz), 12.07 (s, br, 1H), 13.16 (s, br., 1 H); LC/(+)-ESI-MS: *m*/*z* = 456 [M+H]⁺.
Example 112: **Benzyl-(6-{*N*'-[5-(3-nitro-phenyl)-furan-2-ylmethylene]-hydrazino}-[1,2,5]ozadiazolo[3,4-b]pyrazin-5-yl)-amine** was prepared from benzyl-(6-hydrazino-[1,2,5]oxadiazolo[3,4-b]pyrazin-5-yl)-amine hydrochloride and 5-(3-nitro-phenyl)-furan-2-carbaldehyde.
   ¹H NMR: δ = 4.69 (d, 2 H, *J=* 6.3 Hz), 7.25 (tt, 1 H, *J=* 7.0 Hz, *J* ≈ 1.6 Hz), 7.33 ("t", 2 H, *J* ≈ 7.3 Hz), 7.36-7.43 (m, 3 H), 7.51 (d, 1 H, *J=* 3.6 Hz), 7.79 (dd, 1 H, *J= J=* 8.0 Hz), 8.21 (ddd, 1 H, *J=* 8.2 Hz, *J=* 2.3 Hz, *J=* 0.9 Hz), 8.32 ("d", 1 H, *J=* 7.9 Hz), 8.46 (s, 1 H), 8.63 (dd, 1 H, *J= J=* 1.8 Hz), 8.81 (t, br., 1 H, *J=* 6.3 Hz), 12.07 (s, br., 1 H); LC/(+)-ESI-MS: *m*/*z* = 457 [M+H]⁺.
Example 113: **Benzyl-{6-[*N*'-(5-phenyl-furan-2-ylmethylene)-hydrazino]-[1,2,5]oza-diazolo[3,4-b]pyrazin-5-yl}-amine** was prepared from benzyl-(6-hydrazino-[1,2,5]oxadiazolo[3,4-b]pyrazin-5-yl)-amine hydrochloride and 5-phenyl-furan-2-carbaldehyde.
   ¹H NMR: δ = 4.69 (d, 2 H, *J=* 6.3 Hz), 7.22 (d, 1 H, *J=* 3.6 Hz), 7.25 (tt, 1 H, *J*= 7.0 Hz, *J ≈* 1.6 Hz), 7.29-7.42 (m, 6 H), 7.49 ("t", 2 H, *J ≈* 7.3 Hz), 7.90 ("d", 2 H, *J=* 7.3 Hz), 8.43 (s, 1 H), 8.80 (t, br., 1 H, *J=* 6.3 Hz), 11.98 (s, br., 1 H); LC/(+)-ESI-MS: *m*/*z* = 412 [M+H]⁺.
Example 114: **5-{5-[(6-Benzylamino-[1,2,5]oxadiazolo[3,4-b]pyrazin-5-yl)-hydrazono-methyl]-furan-2-yl}-2-chloro-benzoic acid** was prepared from benzyl-(6-hydrazino-[1,2,5]oxadiazolo[3,4-b]pyrazin-5-yl)-amine hydrochloride and 2-chloro-5-(5-formyl-furan-2-yl)-benzoic acid.
   ¹H NMR: δ = 4.68 (d, 2 H, *J =* 6.3 Hz), 6.99 (d, 1 H, *J=* 3.6 Hz), 7.24 (tt, 1 H, *J =* 7.0 Hz, *J≈* 1.6 Hz), 7.33 ("t", 2 H, *J≈* 7.3 Hz), 7.35-7.41 (m, 3H), 7.66-7.72 (m, 1 H), 7.71 (s, 1 H), 7.86 ("d", 1 H, J= 7.9 Hz), 8.40 (s, 1 H), 8.82 (t, br., 1 H, J= 6.3 Hz), 11.95 (s, br., 1 H), 13.52 (s, br., 1 H); LC/(+)-ESI-MS: *m*/*z* = 490 [M+H]⁺; LC/(-)-ESI-MS: *m*/*z* = 488 [M-H]⁻.
Example 115: **Benzyl-[6-(*N*'-furan-2-ylmethylene-hydrazino)-[1,2,5]oxadiazolo[3,4-b]pyrazin-5-yl]-amine** was prepared from benzyl-(6-hydrazino-[1,2,5]oxadiazolo[3,4-b]pyrazin-5-yl)-amine hydrochloride and furan-2-carbaldehyde.
   ¹H NMR: δ = 4.67 (d, 2 H, *J*= 6.3 Hz), 6.72 (dd, 1 H, *J*= 3.4 Hz, *J=* 1.7 Hz), 7.20-7.41 (m, 7 H), 7.95 (d, 1 H, J= 1.7 Hz), 8.41 (s, 1 H), 8.79 (t, br., 1 H, J= 6.3 Hz), 12.00 (s, br., 1 H); LC/(+)-ESI-MS: *m*/*z* = 336 [M+H]⁺; LC/(-)-ESI-MS: *m*/*z* = 334 [M-H]⁻.
Example 116: **3-{5-[(6-Benzylamino-[1,2,5]ozadiazolo[3,4-b]pyrazin-5-yl)-hydrazono-methyl]-furan-2-yl}-benzamide** was prepared from benzyl-(6-hydrazino-[1,2,5]oxadiazolo[3,4-b]pyrazin-5-yl)-amine hydrochloride and 3-(5-formyl-furan-2-yl)-benzamide.
   ¹H NMR: δ = 4.69 (d, 2 H, J= 6.3 Hz), 7.21-7.42 (m, 7 H), 7.47 (s, br., 1 H), 7.57 (dd, 1 *H, J = J =* 7.8 Hz), 7.86 (ddd, 1 H, *J*= 7.9 Hz, *J*= *J*= 1.3 Hz), 8.03 ("d", 1 H, *J*≈ 8 Hz), -8.05 (s, br., 1 H), 8.34 (dd, 1 H, *J= J=* 1.3 Hz), 8.45 (s, 1 H), 8.81 (t, br., 1 H, J= 6.3 Hz), 12.01 (s, br., 1 H); LC/(+)-ESI-MS: *m*/*z* = 455 [M+H]⁺.
Example 117: **4-{5-[(6-Benzylamino-[1,2,5]oxadiazolo[3,4-b]pyrazin-5-yl)-hydrazono-methyl]-furan-2-yl}-benzoic acid** was prepared from benzyl-(6-hydrazino-[1,2,5]oxadiazolo[3,4-b]pyrazin-5-yl)-amine hydrochloride and 4-(5-formyl-furan-2-yl)-benzoic acid.
   ¹H NMR: δ = 4.69 (d, 2 H, *J*= 6.3 Hz), 7.25 (tt, 1 H, *J* = 7.0 Hz, *J* ≈ 1.6 Hz), 7.33 ("t", 2 H, *J* ≈ 7.3 Hz), 7.36-7.42 (m, 4 H), 8.00 (d, 2 H, J = 8.8 Hz), 8.03 (d, 2 H, *J* = 8.8 Hz), 8.45 (s, 1 H), 8.82 (t, br., 1 H, J= 6.3 Hz), 12.02 (s, br., 1 H), 13.01 (s, br., 1 H); LC/(+)-ESI-MS: *m*/*z* = 456 [M+H]⁺.
Example 118: **Benzyl-(6-{*N*'-[5-(4-chloro-phenyl)-furan-2-ylmethylene]-hydrazino}-[1,2,5]oxadiazolo[3,4-b]pyrazin-5-yl)-amine** was prepared from benzyl-(6-hydrazino-[1,2,5]oxadiazolo[3,4-b)pyrazin-5-yl)-amine hydrochloride and 5-(4-chloro-phenyl)-furan-2-carbaldehyde.
   ¹H NMR: δ = 4.69 (d, 2 H, *J* = 6.3 Hz), 7.21-7.41 (m, 7 H), 7.56 (d, 2 H, *J* = 8.7 Hz), 7.91 (d, 2 H, *J=* 8.7 Hz), 8.42 (s, 1 H), 8.80 (t, br., 1 H, *J=* 6.3 Hz), 11.98 (s, br., 1 H); LC/(+)-ESI-MS: *m*/*z* = 446 [M+H]⁺:
Example 119: **5-[(6-Benzylamino-[1,2,5]oxadiazolo[3,4-b]pyrazin-5-yl)-hydrazono-methyl]-furan-2-sulfonic acid** was prepared from benzyl-(6-hydrazino-[1,2,5]oxadiazolo[3,4-b]pyrazin-5-yl)-amine hydrochloride and 5-formyl-furan-2-sulfonic acid.
   ¹H NMR: δ = 4.67 (d, 2 H, J= 6.3 Hz), 6.62 (d, 1 H, *J*= 3.4 Hz), 7.21 (d, 1 H, *J*= 3.4 Hz), 7.24 (tt, 1 H, *J=* 7.0 Hz, *J ≈* 1.6 Hz), 7.32 ("t", 2 H, *J ≈* 7.3 Hz), 7.37 ("d", 2 H, *J* ≈ 7.3 Hz), 8.39 (s, 1 H), 8.85 (t, br., 1 H, J= 6.3 Hz), 12.17 (s, br., 1 H); LC/(+)-ESI-MS: *m*/*z* = 416 [M+H]⁺; LC/(-)-ESI-MS: *m*/*z* = 414 [M-H]⁻.
Example 120: **3-[(6-Benzylamino-[1,2,5]oxadiazolo[3,4-b]pyrazin-5-yl)-hydrazono]-1,3-dihydro-indol-2-one** was prepared from benzyl-(6-hydrazino-[1,2,5]oxadiazolo[3,4-b]pyrazin-5-yl)-amine hydrochloride and isatin.
   ¹H NMR: δ = 4.76 (d, 2 H, *J =* 6.3 Hz), 6.89 (d, 1 H, *J* = 7.7 Hz), 6.97 (td, 1 H, *J =* 7.6 Hz, *J =* 0.9 Hz), 7.26 (tt, 1 H, *J* = 7.0 Hz, *J* ≈ 1.6 Hz), 7.35 ("t", 2 H, *J ≈* 7.3 Hz), 7.43 ("d", 2 H, *J* ≈ 7.3 Hz), 8.01 (d, 1 H, *J*= 7.6 Hz), 8.95 (t, br., 1 H, *J*= 6.3 Hz), 10.78 (s, 1 H), 12.22 (s, br., 1 H); LC/(+)-ESI-MS: *m*/*z* = 387 [M+H]⁺; LC/(-)-ESI-MS: *m*/z = 385 [M-H]⁻.
Example 121: **Benzyl-{6-[*N*'-(1*H*-indol-3-ylmethylene)-hydrazino]-[1,2,5]oxa-diazolo[3,4-b]pyrazin-5-yl}-amine** was prepared from benzyl-(6-hydrazino-[1,2,5]oxadiazolo[3,4-b]pyrazin-5-yl)-amine hydrochloride and 1*H*-indole-3-carbaldehyde.
   ¹H NMR: δ = 4.69 (d, 2 H, *J*= 6.3 Hz), 7.16-7.29 (m, 3 H), 7.34 ("t", 2 H, *J* ≈ 7.3 Hz), 7.39 ("d", 2 H, *J* ≈ 7.3 Hz), 7.44-7.48 (m, 1 H), 8.04 (d, 1 H, J= 2.9 Hz), 8.59 ("d", 1 H, *J* = 7.3 Hz), 8.73 (t, br., 1 H, *J =* 6.3 Hz), 8.79 (s, 1 H), 11.59 (s, br., 1 H), 11.83 (s, br., 1 H); LC/(+)-ESI-MS: *m*/*z* = 385 [M+H]⁺; LC/(-)-ESI-MS: *m*/*z* = 383 [M-H]⁻.
Example 122: **2-{[6-(4-Fluoro-phenylamino)-[1,2,5]oxadiazolo[3,4-b]pyrazin-5-yl]-hydrazonomethyl}-benzoic** acid ethyl ester was prepared from (4-fluoro-phenyl)-(6-hydrazino-[1,2,5]oxadiazolo[3,4-b]pyrazin-5-yl)-amine hydrochloride and 2-formyl-benzoic acid. Due to esterification, a mixture of the ethyl ester and the free acid (ratio, 80 : 20) was obtained.
   ¹H NMR: δ = 1.36 (t, 3 H, *J*= 7.1 Hz), 4.36 (q, 2 H, *J*= 7.1 Hz), 7.26 (dd, 2 H, ³*J*_{HH} = ³*J*_{HF} = 9.1 Hz), 7.63 (ddd, 1 *H, J= J=* 7.2 Hz, *J=* 1.6 Hz), 7.72 ("t", 1 H, *J ≈* 7.3 Hz), 7.91-7.98 (m, 3 H), 8.71 (d, 1 H, *J=* 7.7 Hz), 9.29 (s, 1 H), 9.93 (s, br., 1 H), 12.26 (s, br., 1 H).
Example 123: **2-{[6-(4-Fluoro-phenylamino)-[1,2,5]oxadiazolo[3,4-b]pyrazin-5-yl]-hydrazonomethyl}-phenol** was prepared from (4-fluoro-phenyl)-(6-hydrazino-[1,2,5]oxadiazolo[3,4-b]pyrazin-5-yl)-amine hydrochloride and 2-hydroxy-benzaldehyde.
   ¹H NMR: δ = 6.91-6.97 (m, 2 H), 7.26 (dd, 2 H, ³*J*_{HH} = ³*J*_{HF} = 9.1 Hz), 7.36 (ddd, 1 H, *J=* 8.3 Hz, *J=* 7.3 Hz, *J*= 1.7 Hz), 7.97 (dd, 2 H, ³*J*_{HH} = 9.1 Hz, ⁴*J*_{HF} = 5.1 Hz), 8.09 (d, 1 H, *J* = 7.3 Hz), 8.96 (s, 1 H), 9.89 (s, br., 1 H), 10.21 (s, br., 1 H), 12.30 (s, br., 1 H); LC/(+)-ESI-MS: *m*/*z* = 366 [M+H]⁺; LC/(-)-ESI-MS: *m*/*z* = 364 [M-H]⁻.
Example 124: **3-{[6-(4-Fluoro-phenylamino)-[1,2,5]ozadiazolo[3,4-b]pyrazin-5-yl]-hydrazonoinethyl}-phenol** was prepared from (4-fluoro-phenyl)-(6-hydrazino-[1,2,5]oxadiazolo[3,4-b]pyrazin-5-yl)-amine hydrochloride and 3-hydroxy-benzaldehyde.
   ¹H NMR: δ = 6.94 ("d", 1 H, J ≈ 8.0 Hz), 7.22-7.33 (m, 3 H), 7.46-7.52 (m, 2 H), 7.95 (dd, 2 H, ³*J*_{HH} = 9.1 Hz, ⁴*J*_{HF} = 5.1 Hz), 8.59 (s, 1 H), 9.61 (s, br., 1 H), 9.86 (s, br., 1 H), 12.20 (s, br., 1 H); LC/(+)-ESI-MS: *m*/*z* = 366 [M+H]⁺; LC/(-)-ESI-MS: *m*/*z* = 364 [M-H]-.
Example 125: **4-{[6-(4-Fluoro-phenylamino)-[1,2,5]ozadiazolo[3,4-b]pyrazin-5-yl]-hydrazonomethyl}-phenol** was prepared from (4-fluoro-phenyl)-(6-hydrazino-[1,2,5]oxadiazolo[3,4-b]pyrazin-5-yl)-amine hydrochloride and 4-hydroxy-benzaldehyde.
   ¹H NMR: δ = 6.87 (d, 2 H, *J=* 8.6 Hz), 7.26 (dd, 2 H, ³*J*_{HH} = ³*J*_{HF} = 9.1 Hz), 7.95 (dd, 2 H, ^{*3*}*J*_{HH} = 9.1 Hz, ⁴*J*_{HF} = 5.1 Hz), 7.95 (d, 2 H, *J=* 8.6 Hz), 8.57 (s, 1 H), 9.78 (s, br., 1 H), 10.09 (s, br., 1 H), 12.07 (s, br., 1 H); LC/(+)-ESI-MS: *m*/*z* = 366 [M+H]⁺; LC/(-)-ESI-MS: *m*/*z* = 364 [M-H]⁻.
Example 126: **4-{[6-(4-Fluoro-phenylamino)-[1,2,5]oxadiazolo[3,4-b]pyrazin-5-yl]-hydrazonomethyl}-benzene-1,3-diol** was prepared from (4-fluoro-phenyl)-(6-hydrazino-[1,2,5]oxadiazolo[3,4-b]pyrazin-5-yl)-amine hydrochloride and 2,4-dihydroxy-benzaldehyde.
   ¹H NMR: δ = 6.33-6.40 (m, 2 H), 7.25 (dd, 2 H, ³*J*_{HH} = ³*J*_{HF} = 9.1 Hz), 7.76 (m, br., 1 H), 7.96 (dd, 2 H, ³*J*_{HH} = 9.1 Hz; ⁴*J*_{HF} = 5.1 Hz), 8.80 (s, br., 1 H), 9.80 (s, br., 1 H), 10.04 (s, br., 1 H), 10.20 (s, br., 1 H), 12.17 (s, br., 1 H); LC/(+)-ESI-MS: *m*/*z* = 382 [M+H]⁺; LC/(-)-ESI-MS: *m*/*z* = 380 [M-H]⁻.
Example 127: **2-{[6-(4-Fluoro-phenylamino)-[1,2,5]oxadiazolo[3,4-b]pyrazin-5-yl)-hydrazonomethyl}-5-methozy-phenol** was prepared from (4-fluoro-phenyl)-(6-hydrazino-[1,2,5]oxadiazolo[3,4-b]pyrazin-5-yl)-amine hydrochloride and 2-hydroxy-4-methoxy-benzaldehyde.
   ¹H NMR: δ = 3.80 (s, 3 H), 6.51 (d, 1 H, *J=* 2.3 Hz), 6.56 (dd, 1 H, *J=* 8.6 Hz, *J=* 2.3 Hz), 7.26 (dd, 2 H, ³*J*_{HH} = ³*J*_{HF} = 9.1 Hz), 7.92 (d, 1 H, *J*= 8.6 Hz), 7.96 (dd, 2 H, ³*J*_{*HH*} *=* 9.1 Hz, ⁴*J*_{HF} = 5.1 Hz), 8.87 (s, 1 H), 9.85 (s, br., 1 H), 10.32 (s, br., 1 H), 11.47 (s, br., 1 H), 12.25 (s, br., 1 H); LC/(+)-ESI-MS: *m*/*z* = 396 [M+H]⁺; LC/(-)-ESI-MS: *m*/*z* = 394 [M-H]⁻.
Example 128: **2-{[6-(4-Fluoro-phenylamino)-[1,2,5]ozadiazolo[3,4-b]pyrazin-5-yl]-hydrazonomethyl}-4-methoxy-phenol** was prepared from (4-fluoro-phenyl)-(6-hydrazino-[1,2,5]oxadiazolo[3,4-b]pyrazin-5-yl)-amine hydrochloride and 2-hydroxy-5-methoxy-benzaldehyde.
   ¹H NMR: δ = 3.75 (s, 3 H), 6.88 (d, 1 H, *J=* 8.9 Hz), 6.98 (dd, 1 H, *J=* 8.9 Hz, *J=* 3.0 Hz), 7.26 (dd, 2 H, ³*J*_{HH} = ³*J*_{HF} = 9.1 Hz), 7.72 (d, br., 1 H, *J* = 3.0 Hz), 7.97 (dd, 2 H, ³*J*_{HH} = 9.1 Hz, ⁴*J*_{HF} = 5.1 Hz), 8.95 (s, 1 H), 9.78 (s, br., 1 H), 9.89 (s, br., 1 H), 12.21 (s, br., 1 H); LC/(+)-ESI-MS: *m*/*z* = 396 [M+H]⁺; LC/(-)-ESI-MS: *m*/*z* = 394 [M-H]⁻.
Example 129: **2-{[6-(4-Fluoro-phenylamino)-[1,2,5]ozadiazolo[3,4-b]pyrazin-5-yl]-hydrazonomethyl)-6-methoxy-phenol** was prepared from (4-fluoro-phenyl)-(6-hydrazino-[1,2,5]oxadiazolo[3,4-b]pyrazin-5-yl)-amine hydrochloride and 2-hydroxy-3-methoxy-benzaldehyde.
   ¹H NMR: δ = 3.84 (s, 3 H), 6.89 (dd, 1 H, *J = J =* 7.9 Hz), 7.10 (dd, 1 *H, J =* 8:0 Hz, *J* = 1.3 Hz), 7.26 (dd, 2 H, ³*J*_{HH} = ³*J*_{HF} = 9.1 Hz), 6.89 (d, br., 1 H, J= 7.8 Hz), 7.97 (dd, 2 H, ^{*3*}*J*_{HH} = 9.1 Hz, ⁴*J*_{HF} = 5.1 Hz), 8.98 (s, 1 H), 9.73 (s, br., 1 H), 9.89 (s, br., 1 H), 12.30 (s, br., 1 H).
   LC/(+)-ESI-MS: *m*/*z* = 396 EM+H]⁺.
Example 130: **2,4-Dibromo-6-{[6-(4-fluoro-phenylamino)-[1,2,5]oxadiazolo[3,4-b]pyrazin-5-yl]-hydrazonomethyl}-phenol** was prepared from (4-fluoro-phenyl)-(6-hydrazino-[1,2,5]oxadiazolo[3,4-b]pyrazin-5-yl)-amine hydrochloride and 3,5-dibromo-2-hydroxy-benzaldehyde.
   ¹H NMR: δ = 7.26 (dd, 2 H, ³*J*_{HH} = ³*J*_{HF} = 9.1 Hz), 7.91 (d, 1 H, *J*= 2.4 Hz), 7.94 (dd, 2 H, ³*J*_{HH} = 9.1 Hz, 4*J*_{HF} = 5.1 Hz), 8.15 (d, 1 H, *J=* 2.4 Hz), 8.88 (s, 1 H), 9.92 (s, br., 1 H), 10.78 (s, br., 1 H), 12.55 (s, br., 1 H); LC/(+)-ESI-MS: *m*/*z* = 524 [M+H]⁺.
Example 131: **4-Bromo-2-{[6-(4-fluoro-phenylamino)-[1,2,5]oxadiazolo[3,4-b]pyrazin-5-yl]-hydrazonomethyl}-6-methoxy-phenol** was prepared from (4-fluoro-phenyl)-(6-hydrazino-[1,2,5]oxadiazolo[3,4-b]pyrazin-5-yl)-amine hydrochloride and 5-bromo-2-hydroxy-3-methoxy-benzaldehyde.
   ¹H NMR: δ = 3.87 (s, 3 H), 7.22 (d, 1 H, *J*= 2.3 Hz), 7.26 (dd, 2 H, ³*J*_{HH} = 9.1 Hz, ⁴*J*_{HF}= 5.1 Hz), 7.97 (dd, 2 H, ³*J*_{HH} = 9.1 Hz, ⁴*J*_{HF} = 5.1 Hz), 8.06 (d, br., 1 H, *J* ≈ 2 Hz), 8.96 (s, 1 H), 9.89 (s, br., 1 H), 12.28 (s, br., 1 H); LC/(+)-ESI-MS: *m*/*z* = 474 [M+H]⁺.
Example 132: **4-Chloro-2-{[6-(4-fluoro-phenylamino)-[1,2,5]oxadiazolo[3,4-b]pyrazin-5-yl]-hydrazonomethyl}-phenol** was prepared from (4-fluoro-phenyl)-(6-hydrazino-[1,2,5]oxadiazolo[3,4-b]pyrazin-5-yl)-amine hydrochloride and 5-chloro-2-hydroxy-benzaldehyde.
   ¹H NMR: δ = 6.96 (d, 1 H, *J*= 8.8 Hz), 7.26 (dd, 2 H, ³*J*_{HH} = ³*J*_{HF} = 9.1 Hz), 7.36 (dd, 1 H, *J=* 8.8 Hz, *J*= 2.8 Hz), 7.97 (dd, 2 H, ³*J*_{HH} = 9.1 Hz, ⁴*J*_{HF} = 5.1 Hz), 8.33 (s, br., 1 H), 8.92 (s, 1 H), 9.89 (s, br., 1 H), 10.52 (s, br., 1 H), 12.28 (s, br., 1 H); LC/(+)-ESI-MS: *m*/*z* = 400 [M+H]⁺; LC/(-)-ESI-MS: *m*/*z* = 398 [M-H]⁻.
Example 133: **[6-(*N*'-Benzofuran-2-ylmethylene-hydrazino)-[1,2,5]ozadiazolo[3,4-b]pyrazin-5-yl]-(4-fluoro-phenyl)-amine** was prepared from (4-fluoro-phenyl)-(6-hydrazino-[1,2,5]oxadiazolo[3,4-b]pyrazin-5-yl)-amine hydrochloride and benzofuran-2-carbaldehyde.
   ¹H NMR: δ = 7.27 (dd, 2 H, ³*J*_{HH} = ³*J*_{HF} = 9.0 Hz), 7.34 ("t", 1 H, *J=* 7.7 Hz), 7.47 ("t", 1 H, *J* ≈ 7.5 Hz), 7.68 ("d", 1 H, *J* ≈ 8.5 Hz), 7.72 (s, 1 H), 7.79 ("d", 1 H, *J* ≈ 7.7 Hz), 7.95 (dd, 2 H, ³*J*_{HH} = 9.0 Hz, ⁴*J*_{HF}, = 5.1 Hz), 8.67 (s, 1 H), 9.92 (s, br., 1 H), 12.30 (s, br., 1 H); LC/(+)-ESI-MS: *m*/*z* = 390 [M+H]⁺; LC/(-)-ESI-MS: *m*/*z* = 388 [M-H]⁻.
Example 134: **3-(5-{[6-(4-Fluoro-phenylamino)-[1,2,5]ozadiazolo[3,4-b]pyrazin-5-yl]-hydrazonomethyl}-furan-2-yl)-benzoic acid** was prepared from (4-fluoro-phenyl)-(6-hydrazino-[1,2,5]oxadiazolo[3,4-b]pyrazin-5-yl)-ainine hydrochloride and 3-(5-formyl-furan-2-yl)-benzoic acid.
   ¹H NMR: δ = 7.24-7.28 (m, 1 H), 7.25 (dd, 2 H, ³*J*_{HH} = ³*J*_{HF} = 9.0 Hz), 7.30 (d, 1 H, *J=* 3.4 Hz), 7.60 (dd, 1 H, *J= J=* 7.8 Hz), 7.92 ("d", 1 H, *J=* 7.5 Hz), 7.96 (dd, 2 H, ³*J*_{HH} = 9.0 Hz, ⁴*J*_{HF} = 5.1 Hz), 8.09 ("d", 1 H, *J* = 7.9 Hz), 8.38 ("t", 1 H, *J* ≈ 1.5 Hz), 8.48 (s, 1 H), 9.90 (s, br., 1 H); LC/(+)-ESI-MS: *m*/*z* = 460 [M+H]⁺.
Example 135: **(4-Fluoro-phenyl)-(6-{*N*'-[5-(3-nitro-phenyl)-furan-2-ylmethylene]-hydrazino)-[1,2,5]oxadiazolo[3,4-blpyrazin-5-yl)-amine** was prepared from (4-fluoro-phenyl)-(6-hydrazino-[1,2,5]oxadiazolo[3,4-b]pyrazin-5-yl)-amine hydrochloride and 5-(3-nitro-phenyl)-furan-2-carbaldehyde.
   ¹H NMR: δ = 7.27 (dd, 2 H, ³*J*_{HH} = ³*J*_{HF} = 8.9 Hz), 7.44 (d, 1 H, *J=* 3.6 Hz), 7.53 (d, 1 H, *J* = 3.6 Hz), 7.80 (dd, 1 H, *J = J =* 7.9 Hz), 7.95 (dd, 2 H, ³*J*_{HH} = 9.0 Hz, ⁴*J*_{HF} = 5.1 Hz), 8.21 ("dd", 1 H, *J=* 7.9 Hz, *J* ≈ 2.3 Hz), 8.33 ("d", 1 H, *J =* 7.8 Hz), 8.57 (s, 1H), 8.64 ("t", 1 H, *J* ≈ 2.0 Hz), 9.88 (s, br., 1 H), 12.18 (s, br., 1 H); LC/(+)-ESI-MS: *m*/*z* = 461 [M+H]⁺.
Example 136: **(4-Fluoro-phenyl)-{6-[*N'*-(5-phenyl-furan-2-ylmethylene)-hydrazinol-[1,2,5]ozadiazolo[3,4-b]pyrazin-5-yl}-amine** was prepared from (4-fluoro-phenyl)-(6-hydrazino-[1,2,5]oxadiazolo[3,4-b]pyrazin-5-yl)-amine hydrochloride and 5-phenyl-furan-2-carbaldehyde.
   ¹H NMR: δ = 7.23 (d, 1 H, *J*= 3.4 Hz), 7.27 (dd, 2 H, ³*J*_{HH} = ³*J*_{HF} = 8.9 Hz), 7.34-7.43 (m, 2 H), 7.50 (dd, 2 H, *J ≈ J ≈* 7.7 Hz), 7.91 (d, 2 H, *J ≈* 7.2 Hz), 7.95 (dd, 2 H, ³*J*_{HH} = 8.9 Hz, ⁴*J*_{HF} = 5.1 Hz), 8.53 (s, 1 H), 9.87 (s, br., 1 H), 12.04 (s, br., 1 H); LC/(+)-ESI-MS: *m*/*z* = 416 [M+H]⁺.
Example 137: **(4-Fluoro-phenyl)-[6-(*N*'-furan-2-ylmethylene-hydrazino)-[1,2,5]oxadiazolo[3,4-b]pyrazin-5-yl]-amine** was prepared from (4-fluoro-phenyl)-(6-hydrazino-[1,2,5]oxadiazolo[3,4-b]pyrazin-5-yl)-amine hydrochloride and furan-2-carbaldehyde.
   ¹H NMR: δ = 6.75 (dd, 1 H, *J* = 3 .4 Hz, *J* = 1.7 Hz), 7.26 (dd, 2 H, ³*J*_{HH} =³*J* _{HF} = 8.9 Hz), 7.28-7.33 (m, 1 H), 7.93 (dd, 2 H, ³*J*_{HH} = 8.9 Hz, ⁴*J*_{HF} = 5.1 Hz), 7.97-8.00 (m, 1 H), 8.52 (s, 1 H), 9.85 (s, br., 1 H), 12.12 (s, br., 1 H); LC/(+)-ESI-MS: *m*/*z* = 340 [M+H]⁺; LC/(-)-ESI-MS: *m*/*z* = 338 [M-H]⁻.
Example 138: **3-(5-{[6-(4-Fluoro-phenylamino)-[1,2,5]oxadiazolo[3,4-b]pyrazin-5-yl]-hydrazonomethyl}-furan-2-yl)-benzamide** was prepared from (4-fluoro-phenyl)-(6-hydrazino-[1,2,5]oxadiazolo[3,4-b]pyrazin-5-yl)-amine hydrochloride and 3-(5-formyl-furan-2-yl)-benzamide.
   ¹H NMR: δ = 7.27 (dd, 2 H, ³*J*_{HH} = ³*J*_{HF} = 8.9 Hz), 7.30 (d, 1 H, *J*= 3.6 Hz), 7.43 (d, 1 H, *J* = 3.6 Hz), 1.48 (s, br., 1 H), 7.59 (dd, 1 H, *J = J =* 7.8 Hz), 7.87 ("dt", 1 H, *J =* 7.8 Hz, *J* = 1.2 Hz), 7.95 (dd, 2 H, ³*J*_{HH} = 8.9 Hz, ⁴*J*_{HF} = 5.1 Hz), 8.05 ("d", 1 H, *J*= 7.8 Hz), -8.06 (s, br., 1 H), 8.35 ("t", 1 H, *J* ≈ 1.4 Hz), 8.56 (s, 1 H), 9.88 (s, br., 1 H), 12.13 (s, br., 1 H); LC/(+)-ESI-MS: *m*/*z* = 459 [M+H]⁺.
Example 139: **4-(5-{[6-(4-Fluoro-phenylamino)-[1,2,5]oxadiazolo[3,4-b]pyrazin-5-yl]-hydrazonomethyl}-furan-2-yl)-benzoic** acid was prepared from (4-fluoro-phenyl)-(6-hydrazino-[1,2,5]oxadiazolo[3,4-b]pyrazin-5-yl)-amine hydrochloride and 4-(5-formyl-furan-2-yl)-benzoic acid.
   ¹H NMR: δ = 7.27 (dd, 2 H, ³*J*_{HH} = ³*J*_{HF} = 8.9 Hz), 7.40 (d, 1 H, *J*= 3.6 Hz), 7.43 (d, 1 H, *J* = 3.6 Hz), 7.95 (dd, 2 H, ³*J*_{HH} *=* 8.9 Hz, ⁴*J*_{HF} = 5.1 Hz), 8.01 (d, 2 H, *J =* 8.8 Hz), 8.05 (d, 2 H, *J*= 8.8 Hz), 8.56 (s, 1 H), 9.88 (s, br., 1 H), 12.13 (s, br., 1 H); LC/(+)-ESI-MS: *m*/*z* = 460 [M+H]⁺.
Example 140: **(6-{*N*'-[5-(4-Chloro-phenyl)-furan-2-ylmethylene]-hydrazino}-[1,2,5]-ozadiazolo[3,4-b]pyrazin-5-yl)-(4-fluoro-phenyl)-amine** was prepared from-(4-fluoro-phenyl)-(6-hydrazino-[1,2,5]oxadiazolo[3,4-b]pyrazin-5-yl)-amine hydrochloride and 5-(4-chloro-phenyl)-furan-2-carbaldehyde.
   ¹H NMR: δ = 7.27 (dd, 2 H, ³*J*_{HH} *=* ³*J*_{HF} = 8.9 Hz), 7.28 (d, 1 H, *J=* 3.6 Hz), 7.39 (d, 1 H, *J* = 3.6 Hz), 7.57 (d, 2 H, *J* = 8.6 Hz), 7.93 (d, 2 H, *J =* 8.6 Hz), 7.94 (dd, 2 H, ³*J*_{HH} = 8.9 Hz, ⁴*J*_{HF} = 5.1 Hz), 8.53 (s, 1 H), 9.87 (s, 1 H), 12.12 (s, br., 1 H); LC/(+)-ESI-MS: *m*/*z* = 450 [M+H]⁺.
Example 141: **3-{[6-(4-Fluoro-phenylamino)-[1,2,5]oxadiazolo[3,4-b]pyrazin-5-yl]-hydrazono}-1,3-dihydro-indol-2-one** was prepared from (4-fluoro-phenyl)-(6-hydrazino-[1,2,5]oxadiazolo[3,4-b]pyrazin-5-yl)-amine hydrochloride and isatin.
   ¹H NMR: δ = 6.90 (d, 1 H, *J=* 7.8 Hz), 7.02 (t, 1 H, *J=* 7.6 Hz), 7.28 (dd, 2 H, ³*J*_{HH} = ³*J*_{HH} = 8.9 Hz), 7.39 (td, 1 H, *J=* 7.7 Hz, *J=* 1.0 Hz), 7.95 (dd, 2 H, ³*J*_{HH}. = 8.9 Hz, ⁴*J*_{HF} = 5.1 Hz), 7.99 (d, 2 H, *J=* 7.7 Hz), 10.03 (s, br., 1 H), 10.80 (s, br., 1 H), 12.22 (s, br., 1 H); LC/(+)-ESI-MS: *m*/*z* = 391 [M+H]⁺.
Example 142: **(4-Fluoro-phenyl)-{6-[*N*'-(2-methyl-1*H*-indol-3-ylmethylene)-hydrazino]-[1,2,5]ozadiazolo[3,4-b]pyrazin-5-yl}-amine** was prepared from (4-fluoro-phenyl)-(6-hydrazino-[1,2,5]oxadiazolo[3,4-b]pyrazin-5-yl)-amine hydrochloride and 2-methyl-1*H*-indole-3-carbaldehyde.
   ¹H NMR: δ = 2.62 (s, 3 H), 7.15-7.20 (m, 2 H), 7.27 (dd, 2 H, ³*J*_{HH} = ³*J*_{HF} = 8.9 Hz), 7.32-7.40 (m, 1 H), 7.98 (dd, 2 H, ³*J*_{HH} = 8.9 Hz, ⁴*J*_{HF} = 5.1 Hz), 8.57-8.62 (m, 1 H), 8.98 (s, 1 H), 9.85 (s, br., 1 H), 11.78 (s, br., 1 H); LC/(+)-ESI-MS: m/z = 403 [M+H]⁺.
Example 143: **4-{[6-(3-Chloro-phenylamino)-[1,2,5]ozadiazolo[3,4-b]pyrazin-5-yl]-hydrazonomethyl}-benzoic acid** was prepared from (3-chloro-phenyl)-(6-hydrazino-[1,2,5]oxadiazolo[3,4-b]pyrazin-5-yl)-amine hydrochloride and 4-formyl-benzoic acid.
   'H NMR: δ = 7.23 ("d", 1 H, *J*= 8.1 Hz), 7.45 (dd, 1 H, *J*= *J* = 8.1 Hz), 7.91 ("d", 1 H, *J* = 8.1 Hz), 8.05 (d, 2 H, *J =* 8.2 Hz), 8.17 ("t", 1 H, *J*= 1.8 Hz), 8.24 (d, 2 H, *J =* 8.2 Hz), 8.74 (s, 1 H), 9.96 (s, br., 1 H), 12.37 (s, br., 1 H), 13.13 (s, br., 1 H); LC/(+)-ESI-MS: *m*/*z* = 410 [M+H]⁺; LC/(-)-ESI-MS: *m*/*z* = 408 [M-H]⁻.
Example 144: **2-([6-(3-Chloro-phenylamino)-[1,2,5]oxadiazolo[3,4-b]pyrazin-5-yl]-hydrazonomethyl}-phenol** was prepared from (3-chloro-phenyl)-(6-hydrazino-[1,2,5]oxadiazolo[3,4-b]pyrazin-5-yl)-amine hydrochloride and 2-hydroxy-benzaldehyde.
   ¹H NMR: δ = 6.90-6.98 (m, 2 H), 7.23 ("d", 1 H, J= 8.1 Hz), 7.33-7.40 (m, 1 H), 7.45 (dd, 1 H, *J = J =* 8.1 Hz), 7.95 ("d", 1 H, *J* = 8.1 Hz), 8.10 ("d", 1 H, *J* = 7.6 Hz), 8.17 ("t", 1 H, J= 2.0 Hz), 8.98 (s, 1 H), 9.95 (s, br., 1 H), 10.21 (s, br., 1 H), 12.34 (s, br., 1 H); LC/(+)-ESI-MS: *m*/*z* = 380 [M+H]⁺; LC/(-)-ESI-MS: *m*/*z* = 382 [M-H]⁻.
Example 145: **3-{[6-(3-Chloro-phenylamino)-[1,2,5]ozadiazolo[3,4-b]pyrazin-5-yl]-hydrazonomethyl}-phenol** was prepared from (3-chloro-phenyl)-(6-hydrazino-[1,2,5]oxadiazolo[3,4-b]pyrazin-5-yl)-amine hydrochloride and 3-hydroxy-benzaldehyde.
   ¹H NMR: δ = 6.94 (ddd, 1 H, *J =* 8.1 Hz, *J =* 2.1 Hz, *J* = 1.0 Hz), 7.23 ("d", 1 H, *J =* 8.1 Hz), 7.30 (dd, 1 H, *J=* 8.0 Hz), 7.45 (dd, 1 H, *J= J=* 8.1 Hz), 7.47-7.51 (m, 2 H), 7.91 ("d", 1 H, *J =* 8.1 Hz), 8.17 ("t", 1 H, *J =* 2.0 Hz), 8.60 (s, 1 H), 9.62 (s, br., 1 H), 9.92 (s, br., 1 H), 12.24 (s, br., 1 H); LC/(+)-ESI-MS: *m*/*z* = [M+H]⁺; LC/(-)-ESI-MS: *m*/*z* = [M-H]⁻.
Example 146: **4-{[6-(3-Chloro-phenylamino)-[1,2,5]ozadiazolo[3,4-b]pyrazin-5-yl]-hydrazonomethyl}-phenol** was prepared from (3-chloro-phenyl)-(6-hydrazino-[1,2,5]oxadiazolo[3,4-b]pyrazin-5-yl)-amine hydrochloride and 4-hydroxy-benzaldehyde.
   ¹H NMR: δ = 6.87 (d, 2 *H, J =* 8.5 Hz), 7.22 ("d", 1 H, *J =* 8.1 Hz), 7.44 (dd, 1 H, *J = J =* 8.1 Hz), 7.90 ("d", 1 H, *J =* 8.1 Hz), 7.94 (d, 2 H, *J =* 8.5 Hz), 8.16 ("t", 1 H, *J =* 1.8 Hz), 8.58 (s, 1 H), 9.88 (s, br., 1 H), 10.12 (s, br., 1 H), 12.09 (s, br., 1 H); LC/(+)-ESI-MS: *m*/*z* = 382 [M+H]⁺; LC/(-)-ESI-MS: *m*/*z* = 380 [M-H]⁻.
Example 147: **4-([6-(3-Chloro-phenylamino)-[1,2,5]oxadiazolo[3,4-b]pyrazin-5-yl]-hydrazonomethyl}-benzene-1,3-diol** was prepared from (3-chloro-phenyl)-(6-hydrazino-[1,2,5]oxadiazolo[3,4-b]pyrazin-5-yl)-amine hydrochloride and 2,4-dihydroxy-benzaldehyde.
   ¹H NMR: δ = 6.36-6.41 (m, 2 H), 7.22 ("d", 1 H, *J =* 8.1 Hz), 7.44 (dd, 1 H, *J = J =* 8.1 Hz), 7.82 (d, 1 H, *J=* 8.4 Hz), 7.93 ("d", 1 H, *J=* 8.1 Hz), 8.16 ("t", 1 H, *J=* 1.9 Hz), 8.84 (s, 1 H), 9.89 (s, br., 1 H), 10.11 (s, br., 1 H), 10.18 (s, br., 1 H), 12.22 (s, br., 1 H); LC/(-)-ESI-MS: *m*/*z* = 396 [M-H]⁻.
Example 148: **2-{[6-(3-Chloro-phenylamino)-[1,2,5]ozadiazolo[3,4-b]pyrazin-5-yl]-hydrazonomethyl)-5-methoxy-phenol** was prepared from (3-chloro-phenyl)-(6-hydrazino-[1,2,5]oxadiazolo[3,4-b]pyrazin-5-yl)-amine hydrochloride and 2-hydroxy-4-methoxy-benzaldehyde.
   ¹H NMR: δ = 3.80 (s, 3 H), 6.51 (s, 1 H), 6.56 ("d", 1 H, *J*= 8.7 Hz), 7.22 ("d", 1 H, J= 8.1 Hz), 7.44 (dd, 1 H, *J = J =* 8.1 Hz), 7.91 (d, 1 H, *J ≈* 8 Hz), 7.91 (d, 1 H, *J* ≈ 8 Hz), 8.16 (s, 1 H), 8.88 (s, 1 H), 9.90 (s, br., 1 H), 10.34 (s, br., 1 H), 12.29 (s, br., 1 H); LC/(+)-ESI-MS: *m*/*z* = 412 [M+H]⁺; LC/(-)-ESI-MS: *m*/*z =* 410 [M-H]-.
Example 149: **2-{[6-(3-Chloro-phenylamino)-[1,2,5]ozadiazolo[3,4-b]pyrazin-5-yl]-hydrazonomethyl}-4-methozy-phenol** was prepared from (3-chloro-phenyl)-(6-hydrazino-[1,2,5]oxadiazolo[3,4-b]pyrazin-S-yl)-amine hydrochloride and 2-hydroxy-5-methoxy-benzaldehyde.
   ¹H NMR: δ = 3.78 (s, 3 H), 6.89 (d, 1 H, *J*= 8.9 Hz), 6.99 (dd, 1 H, *J*= 8.9 Hz, *J*= 3.1 Hz), 7.24 ("d", 1 H, *J =* 8.1 Hz), 7.45 (dd, 1 H, *J = J =* 8.1 Hz), 7.72 (d, 1 H, *J* = 2.9 Hz), 7.95 ("d", 1 H, J= 8.1 Hz), 8.18 (s, 1 H), 8.97 (s, 1 H), 9.78 (s, br., 1 H), 9.95 (s, br., 1 H), 12.24 (s, br., 1 H); LC/(+)-ESI-MS: *m*/*z* = 412 [M+H]⁺; LC/(-)-ESI-MS: *m*/*z* = 410 [M-H]⁻.
Example 150: **2-{[6-(3-Chloro-phenylamino)-[1,2,5]oxadiazolo[3,4-b]pyrazin-5-yl]-hydrazonomethyl}-6-methoxy-phenol** was prepared from (3-chloro-phenyl)-(6-hydrazino-[1,2,5]oxadiazolo[3,4-b]pyrazin-5-yl)-amine hydrochloride and 2-hydroxy-3-methoxy-benzaldehyde.
   ¹H NMR: δ = 6.89 (dd, 1 H, *J= J=* 7.9 Hz), 7.10 ("d", 1 H, *J=* 7.9 Hz), 7.22 ("d", 1 H, *J* = 8.1 Hz), 7.44 (dd, 1 H, *J = J =* 8.1 Hz), 7.70 ("d", 1 H, *J* = 7.9 Hz), 7.95 ("d", 1 H, *J=* 8.1 Hz), 8.18 ("t", 1 H, *J=* 1.7 Hz), 9.00 (s, 1 H), 9.73 (s, br., 1 H), 9.94 (s, br., 1 H), 12.33 (s, br., 1 H); LC/(+)-ESI-MS: *m*/*z* = 412 [M+H]⁺.
Example 151: **2,4-Dibromo-6-{[6-(3-chloro-phenylamino)-[1,2,5]oxadiazolo[3,4-b]pyrazin-5-yl]-hydrazonomethyl}-phenol** was prepared from (3-chloro-phenyl)-(6-hydrazino-[1,2,5]oxadiazolo[3,4-b]pyrazin-5-yl)-amine hydrochloride and 3,5-dibromo-2-hydroxy-benzaldehyde.
   ¹H NMR: δ = 7.23 ("d", 1 H, *J*= 8.1 Hz), 7.45 (dd, 1 H, *J=J=* 8.1 Hz), 7.91 ("d", 1 H, *J* = 8.1 Hz), 7.92 (d, 1 H, *J*= 2.4 Hz), 8.14-8.16 (m, 2 H), 8.89 (s, 1 H), 9.98 (s, 1 H), 9.98 (s, br., 1 H), 10.78 (s, br., 1 H), 12.58 (s, br., 1 H); LC/(+)-ESI-MS: *m*/*z* = 540 [M+H]⁺; LC/(-)-ESI-MS: *m*/*z* = 538 [M-H]⁻.
Example 152: **4-Bromo-2-{[6-(3-chloro-phenylamino)-[1,2,5]oxadiazolo[3,4-b]pyrazin-5-yl]-hydrazonomethyl}-6-methoxy-phenol** was prepared from (3-chloro-phenyl)-(6-hydrazino-[1,2,5]oxadiazolo[3,4-b]pyrazin-5-yl)-amine hydrochloride and 5-bromo-2-hydroxy-3-methoxy-benzaldehyde.
   ¹H NMR: δ = 3.87 (s, 3 H), 7.21-7.25 (m, 2 H), 7.44 (dd, 1 H, *J= J=* 8.1 Hz), 7.95 ("d", 1 H, *J*= 8.1 Hz), 8.05 (d, 1 H, *J*= 1.8 Hz), 8.17 ("t", 1 H, J= 2.0 Hz), 8.97 (s, 1 H), 9.89 (s, br., 1 H), 9.94 (s, br., 1 H), 12.30 (s, br., 1 H); LC/(+)-ESI-MS: *m*/*z* = 492 [M+H]⁺; LC/(-)-ESI-MS: *m*/*z* = 490 [M-H]⁻.
Example 153: **4-Chloro-2-{[6-(3-chloro-phenylamino)-[1,2,5]oxadiazolo[3,4-b]pyrazin-5-yl]-hydrazonomethyl}-phenol** was prepared from (3-chloro-phenyl)-(6-hydrazino-[1,2,5]oxadiazolo[3,4-b]pyrazin-5-yl)-amine hydrochloride and 5-chloro-2-hydroxy-benzaldehyde.
   ¹H NMR: δ = 6.96 (d, 1 H, *J*= 8.8 Hz), 7.22 (ddd, 1 H, *J*= 8.1 Hz, *J*= 1.8 Hz, *J*= 0.8 Hz), 7.36 (dd, 1 H, *J =* 8.8 Hz, *J =* 2.7 Hz), 7.44 (dd, 1 H, *J = J =* 8.1 Hz), 7.95 ("d", 1 H, *J* = 8.1 Hz), 8.17 ("t", 1 H, *J =* 2.0 Hz), 8.35 (d, 1 H, *J =* 2.7 Hz), 8.94 (s, 1 H), 9.95 (s, br., 1 H), 10.52 (s, br., 1 H), 12.31 (s, br., 1 H); LC/(+)-ESI-MS: *m*/*z* = 416 [M+H]⁺; LC/(-)-ESI-MS: *m*/z = 414 [M-H]⁻.
Example 154: **3-(5-{[6-(3-Chloro-phenylamino)-[1,2,5]ozadiazolo[3,4-b]pyrazin-5-yl]-hydrazonomethyl}-furan-2-yl)-benzoic** acid was prepared from (3-chloro-phenyl)-(6-hydrazino-[1,2,5]oxadiazolo[3,4-b]pyrazin-5-yl)-amine hydrochloride and 3-(5-formyl-furan-2-yl)-benzoic acid.
   ¹H NMR: δ = 7.23 ("d", 1 H, *J* = 8.1 Hz), 7.38 (d, 1 H, *J* = 3.6 Hz), 7.42 (d, 1 H, *J* = 3.6 Hz), 7.45 (dd, 1 H, *J= J=* 8.1 Hz), 7.64 (dd, 1 *H, J= J=* 7.8 Hz)*,* 7.88-7.97 (m, 2 H), 8.11-8.18 (m, 2 H), 8.41 ("t", 1 H, *J*= 1.5 Hz), 8.57 (s, 1 H), 9.94 (s, br., 1 H), 12.20 (s, br., 1 H), 13.14 (s, br., 1 H); LC/(+)-ESI-MS: *m*/*z* = 476 [M+H]⁺.
Example 155: **(3-Chloro-phenyl)-(6-{*N*'-[5-(3-nitro-phenyl)-furan-2-ylmethylene]-hydrazino}-[1,2,5]ozadiazolo[3,4-b]pyrazin-5-yl)-amine** was prepared from (3-chloro-phenyl)-(6-hydrazino-[1,2,5]oxadiazolo[3,4-b]pyrazin-5-yl)-amine hydrochloride and 5-(3-nitro-phenyl)-furan-2-carbaldehyde.
   ¹H NMR: δ = 7.23 (ddd, 1 H, *J=* 8.1 Hz, *J*= 2.0 Hz, *J=* 0.9 Hz), 7.45 (d, 1 H, *J*= 3.6 Hz), 7.46 (dd, 1 H, *J= J=* 8.1 Hz), 7.54 (d, 1 H, *J*= 3.6 Hz), 7.81 (dd, 1 H, *J= J=* 8.0 Hz), 7.91 ("d", 1 H, *J* = 8.1 Hz), 8.16 ("t", 1 H, *J* = 2.0 Hz), 8.22 (ddd, 1 H, *J* = 8.1 Hz, *J* = 2.1 Hz, *J*= 0.8 Hz), 8.34 ("d", 1 H, *J*= 7.7 Hz), 8.58 (s, 1 H), 8.65 ("t", 1 H, *J* ≈ 1.7 Hz), 9.94 (s, br., 1 H), 12.22 (s, br., 1 H); LC/(+)-ESI-MS: *m*/*z* = 477 [M+H]⁺; LC/(-)-ESI-MS: *m*/*z* = 475 [M-H]⁻.
Example 156: **(3-Chloro-phenyl)-{6-[*N*'-(5-phenyl-furan-2-ylmethylene)-hydrazino]-[1,2,5]oxadiazolo[3,4-b]pyrazin-5-yl}-amine** was prepared from (3-chloro-phenyl)-(6-hydrazino-[1,2,5]oxadiazolo[3,4-b]pyrazin-5-yl)-amine hydrochloride and 5-phenyl-furan-2-carbaldehyde.
   ¹H NMR: δ = 7.21-7.27 (m, 2 H), 7.35-7.55 (m, 6 H), 7.91 ("d", 1 H, *J* = 8.1 Hz), 8.16 ("t", 1 H, *J*= 1.9 Hz), 8.56 (s, 1 H), 9.93 (s, br., 1 H), 12.15 (s, br., 1 H); LC/(+)-ESI-MS: *m*/*z* = 432 [M+H]⁺; LC/(-)-ESI-MS: *m*/*z* = 430 [M-H]⁻.
Example 157: **2-Chloro-5-(5-{[6-(3-chloro-phenylamino)-[1,2,5]oxadiazolo[3,4-b]pyrazin-5-yl]-hydrazonomethyl}-furan-2-yl)-benzoic acid** was prepared from (3-chloro-phenyl)-(6-hydrazino-[1,2,5]oxadiazolo[3,4-b]pyrazin-5-yl)-amine hydrochloride and 2-chloro-5-(5-formyl-furan-2-yl)-benzoic acid.
   ¹H NMR: δ = 7.02 (d, 1 H, *J* = 3.6 Hz), 7.23 ("d", 1 H, *J* = 8.1 Hz), 7.41 (d, 1 H, *J* = 3.6 Hz), 7.45 (dd, 1 H, *J*= *J*= 8.1 Hz), 7.67-7.74 (m, 2 H), 7.86 (d, 1 H, *J*= 8.1 Hz), 7.92 ("d", 1 H, J= 8.2 Hz), 8.16 ("t", 1 H, J= 1.7 Hz), 8.53 (s, 1 H), 9.92 (s, br., 1 H), 12.09 (s, br., 1 H), 13.50 (s, br., 1 H); LC/(+)-ESI-MS: *m*/*z* = 510 [M+H]⁺.
Example 158: **(3-Chloro-phenyl)-[6-(*N*'-furan-2-ylmethylene-hydrazino)-[1,2,5]ozadiazolo[3,4-b]pyrazin-5-yl]-amine** was prepared from (3-chloro-phenyl)-(6-hydrazino-[1,2,5]oxadiazolo[3,4-b]pyrazin-5-yl)-amine hydrochloride and furan-2-carbaldehyde.
   ¹H NMR: δ = 6.75 (dd, 1 H, *J=* 3.5 Hz, *J=* 1.7 Hz), 7.22 ("d", 1 H, *J=* 8.1 Hz), 7.31 (d, 1 H, *J =* 3.5 Hz), 7.44 (dd, 1 H, *J = J =* 8.1 Hz), 7.90 ("d", 1 H, *J* = 8.1 Hz), 7.99 (d, 1 H, *J=* 1.7 Hz), 8.15 ("t", 1 H, *J =* 2.0 Hz), 8.53 (s, 1 H), 9.91 (s, br., 1 H), 12.16 (s, br., 1 H); LC/(+)-ESI-MS: *m*/*z* = 356 [M+H]⁺; LC/(-)-ESI-MS: *m*/*z* = 354 [M-H]⁻.
Example 159: **2-{[6-(3-Chloro-4-fluoro-phenylamino)-[1,2,5]ozadiazolo[3,4-b]pyrazin-5-yl]-hydrazonomethyl}-benzoic** acid ethyl ester was prepared from (3-chloro-4-fluoro-phenyl)-(6-hydrazino-[1,2,5]oxadiazolo[3,4-b]pyrazin-5-yl)-amine hydrochloride and 2-formyl-benzoic acid. Due to esterification, a mixture of the ethyl ester and the free acid (ratio, 70 : 30) was obtained.
   ¹H NMR: δ = 1.36 (t, 3 H, *J*= 7.1 Hz), 4.37 (q, 2 H, *J*= 7.1 Hz), 7.48 (dd, 1 H, ³*J*_{HH} = ³*J*_{HF} = 9.1 Hz), 7.64 (td, 1 H, *J* = 7.6 Hz, J = 1.5 Hz), 7.72 ("t", 1 H, *J ≈* 7.7 Hz), 7.92 ("d", 1 H, *J ≈* 7.5 Hz), 7.95 (ddd, 1 H, ³*J*_{HH} = 9.1 Hz, ⁴*J*_{HF} = 4.3 Hz, ⁴*J*_{HH} = 2.6 Hz), 8.24 (dd, 1 H, ⁴*J*_{HF} = 6.9 Hz, ⁴*J*_{HH} = 2.6 Hz), 8.72 (dd, 1 H, *J =* 7.9 Hz, *J* = 1.1 Hz), 9.30 (s, 1 H), 10.04 (s, br., 1 H), 12.29 (s, br., 1 H); LC/(+)-ESI-MS: *m*/*z* = 456 [M+H]⁺.
Example 160: **3-{[6-(3-Chloro-4-fluoro-phenylamino)-[1,2,5]oxadiazolo[3,4-b]pyrazin-5-yl]-hydrazonomethyl}-benzoic** acid was prepared from (3-chloro-4-fluoro-phenyl)-(6-hydrazino-[1,2,5]oxadiazolo[3,4-b]pyrazin-5-yl)-amine hydrochloride and 3-formyl-benzoic acid.
   ¹H NMR: δ = 7.48 (dd, 1 H, ³*J*_{HH} = ³*J*_{HF} = 9.1 Hz), 7.64 (dd, 1 H, *J= J=* 7.7 Hz), 7.95 (ddd, 1 H, ³*J*_{HH}=9.1 Hz, ⁴*J*_{HF} = 4.3 Hz, ⁴*J*_{HH} = 2.6 Hz), 8.07 (dt, 1 H, *J*= 7.7 Hz, *J*= 1.4 Hz), 8.26 (dd, 1 H, ⁴*J*_{HF} = 6.9 Hz, ⁴*J*_{HH} = 2.6 Hz), 8.38 ("t", 1 H, *J=* 7.7 Hz), 8.57 (t, 1 H, *J* = 1.4 Hz), 8.73 (s, 1 H), 10.02 (s, br., 1 H), 12.34 (s, br., 1 H), 13.16 (s, br., 1 H); LC/(+)-ESI-MS: *m*/*z* = 428 [M+H]⁺; LC/(-)-ESI-MS: *m*/*z* = 426 [M-H]⁻.
Example 161: **4-{[6-(3-Chloro-4-fluoro-phenylamino)-[1,2,5]oxadiazolo[3,4-b]pyrazin-5-yl]-hydrazonomethyl}-benzoic acid** was prepared from (3-chloro-4-fluoro-phenyl)-(6-hydrazino-[1,2,5]oxadiazolo[3,4-b]pyrazin-5-yl)-amine hydrochloride and 4-formyl-benzoic acid.
   ¹H NMR: δ = 7.48 (dd, 1 H, ³*J*_{HH} = ³*J*_{HF} = 9.1 Hz), 7.95 (ddd, 1 H, ³*J*_{HH} = 9.1 Hz, ⁴*J*_{HF} *=* 4.3 Hz, ⁴*J*_{HH} = 2.6 Hz), 8.04 (d, 2 H, *J* = 8.3 Hz), 8.22 (d, 2 H, *J* = 8.3 Hz), 8.25 (dd, 1 H, ⁴*J*_{HF} = 6.9 Hz, ⁴*J*_{HH} = 2.6 Hz), 8.72 (s, 1 H), 10.00 (s, br., 1 H), 12.36 (s, br., 1 H), 13.12 (s, br., 1 H); LC/(+)-ESI-MS: *m*/*z* = 428 [M+H]⁺; LC/(-)-ESI-MS: *m*/*z* = 426 [M-H]⁻.
Example 162: **2-{[6-(3-Chloro-4-fluoro-phenylamino)-[1,2,5]oxadiazolo[3,4-b]pyrazin-5-yl]-hydrazonomethyl}-phenol** was prepared from (3-chloro-4-fluoro-phenyl)-(6-hydrazino-[1,2,5]oxadiazolo[3,4-b]pyrazin-5-yl)-amine hydrochloride and 2-hydroxy-benzaldehyde.
   ¹H NMR: δ = 6.90-7.00 (m, 2 H), 7.36 ("t", 1 H, *J* ≈ 7.8 Hz), 7.48 (dd, 1 H, ³*J*_{HH} = ³*J*_{HF}= 9.1 Hz), 7.98 (ddd, 1 H, ³*J*_{HH} = 9.1 Hz, ⁴*J*_{HF} = 4.3 Hz, ⁴*J*_{HH} = 2.6 Hz), 8.10 ("d", 1 H, *J ≈* 8 Hz), 8.26 (dd, 1 H, ⁴*J*_{HF} = 6.8 Hz, ⁴*J*_{HH} = 2.6 Hz), 8.97 (s, 1 H), 9.99 (s, br., 1 H), 10.22 (s, br., 1 H), 12.33 (s, br., 1 H); LC/(+)-ESI-MS: *m*/*z* = 400 [M+H]⁺; LC/(-)-ESI-MS: *m*/*z* = 398 [M-H]⁻.
Example 163: **3- {[6-(3-Chloro-4-fluoro-phenylamino)- [1,2,5] oxadiazolo [3,4-b] pyrazin-5-yl]-hydraxonomethyl}-phenol** was prepared from (3-chloro-4-fluoro-phenyl)-(6-hydrazino-[1,2,5]oxadiazolo[3,4-b]pyrazin-5-yl)-amine hydrochloride and 3-hydroxy-benzaldehyde.
   ¹H NMR: δ = 6.94 ("d", 1 H, *J* ≈ 8.2 Hz), 7.30 (dd, 1 H, *J*= *J*= 8.2 Hz), 7.48 (dd, 1 H, ³*J*_{HH} = ³*J*_{HF} = 9.1 Hz), 7.47-7.54 (m, 2 H), 7.95 (ddd, 1 H, ³*J*_{HH} = 9.1 Hz, ⁴*J*_{HF} = 4.3 Hz, ⁴*J*_{HH} = 2.6 Hz), 8.26 (dd, 1 H, ⁴*J*_{HF} = 6.8 Hz, ⁴*J*_{HH} = 2.6 Hz), 8.58 (s, 1 H), 9.62 (s, br., 1 H), 9.98 (s, br., 1 H), 12.23 (s, br., 1 H); LC/(+)-ESI-MS: *m*/*z* = 400 [M+H]⁺; LC/(-)-ESI-MS: *m*/*z* = 398 [M-H]-.
Example 164: **4-{[6-(3-Chloro-4-fluoro-phenylamino)-[1,2,5]oxadiazolo[3,4-b]pyrazin-5-yl]-hydrazonomethyl}-phenol** was prepared from (3-chloro-4-fluoro-phenyl)-(6-hydrazino-[1,2,5]oxadiazolo[3,4-b]pyrazin-5-yl)-amine hydrochloride and 4-hydroxy-benzaldehyde.
   ¹H NMR: δ = 6.87 (d, 2 H, *J*= 8.7 Hz), 7.47 (dd, 1 H, ³*J*_{HH} = ³*J*_{HF} = 9.1 Hz), 7.94 (ddd, 1 H, ³*J*_{HH} = 9.1 Hz, ⁴*J*_{HF} = 4.3 Hz, ⁴*J*_{HH} = 2.6 Hz), 7.94 (d, 2 H, *J=* 8.7 Hz); 8.25 (dd, 1 H, ⁴*J*_{HF} = 6.8 Hz, ⁴*J*_{HH} = 2.6 Hz), 8.57 (s, 1 H), 9.93 (s, br., 1 H), 10.11 (s, br., 1 H), 12.09 (s, br., 1 H); LC/(+)-ESI-MS: *m*/*z* = 400 [M+H]⁺; LC/(-)-ESI-MS: *m*/*z* = 398 [M-H]⁻.
Example 165: **4-{[6-(3-Chloro-4-fluoro-phenylamino)-[1,2,5]oxadiazolo[3,4-b]pyrazin-5-yl]-hydrazonomethyl]-benzene-1,3-diol** was prepared from (3-chloro-4-fluoro-phenyl)-(6-hydrazino-[1,2,5]oxadiazolo[3,4-b]pyrazin-5-yl)-amine hydrochloride and 2,4-dihydroxy-benzaldehyde.
   ¹H NMR: δ = 6.36 ("t", 1 H, *J* = 2.3 Hz), 6.38 (dd, 1 H, *J* = 8.3 Hz, *J* = 2.3 Hz), 7.47 (dd, 1 H, ³*J*_{HH} = ³*J*_{HF} = 9.1 Hz), 7.82 (d, 1 H, *J*= 8.6 Hz), 7.96 (ddd, 1 H, ³*J*_{HH} = 9.1 Hz, ⁴*J*_{HF} = 4.3 Hz, ⁴*J*_{HH} = 2.6 Hz), 8.25 (dd, 1 H, ⁴*J*_{HF} = 6.8 Hz, ⁴*J*_{HH} = 2.6 Hz), 8.83 (s, br., 1 H), 9.93 (s, br., 1 H), 10.11 (s, br., 1 H), 10.17 (s, br., 1 H), 12.22 (s, br., 1 H); LC/(+)-ESI-MS: *m*/*z* = 416 [M+H]⁺; LC/(-)-ESI-MS: *m*/*z* = 414 [M-H]⁻.
Example 166:**2-{[6-(3-Chloro-4-fluoro-phenylamino)-[1,2,5]oxadiazolo[3,4-b]pyrazin-5-yl]-hydrazonomethyl}-5-methozy-phenol** was prepared from (3-chloro-4-fluoro-phenyl)-(6-hydrazino-[1,2,5]oxadiazolo[3,4-b]pyrazin-5-yl)-amine hydrochloride and 2-hydroxy-4-methoxy-benzaldehyde.
   ¹H NMR: δ = 3.80 (s, 3 H), 6.50 (d, 1 H, *J=* 2.4 Hz), 6.56 (dd, 1 H, *J=* 8.7 Hz, *J*= 2.4 Hz), 7.48 (dd, 1 H, ³*J*_{HH} = ³*J*_{HF} = 9.1 Hz), 7.93 (d, 1 H, J= 8.7 Hz), 7.97 (ddd, 1 H, ³*J*_{HH}= 9.1 Hz, ⁴*J*_{HF} = 4.3 Hz, ⁴*J*_{HH} = 2.6 Hz), 8.26 (dd, 1 H, ⁴*J*_{HF} 6.8 Hz, ⁴*J*_{HH} = 2.6 Hz), 8.87 (s, 1 H), 9.96 (s, br., 1 H), 10.35 (s, br., 1 H), 12.28 (s, br., 1 H); LC/(+)-ESI-MS: *m*/*z* = 430 [M+H]⁺; LC/(-)-ESI-MS: *m*/*z* = 428 [M-H]⁻.
Example 167: **2-{[6-(3-Chloro-4-fluoro-phenylamino)-[1,2,5]oxadiazolo [3,4-b]pyrazin-5-yl]-hydrazonomethyl}-4-methoxy-phenol** was prepared from (3-chloro-4-fluoro-phenyl)-(6-hydrazino-[1,2,5]oxadiazolo[3,4-b]pyrazin-5-yl)-amine hydrochloride and 2-hydroxy-5-methoxy-benzaldehyde.
   ¹H NMR: δ = 3.78 (s, 3 H), 6.87 (d, 1 H, *J*= 8.9 Hz), 6.98 (dd, 1 H, *J=* 8.9 Hz, *J=* 3.2 Hz), 7.48 (dd, 1 H, ³*J*_{HH} = ³*J*_{HF}= 9.1 Hz), 7.71 (d, br., 1 H, *J* ≈ 3 Hz), 7.99 (ddd, 1 H, ³*J*_{HH}, = 9.1 Hz, ⁴*J*_{HF} = 4.3 Hz, ⁴*J*_{HH} = 2.6 Hz), 8.27 (dd, 1 H, ⁴*J*_{HF} = 6.8 Hz, ⁴*J*_{HH} = 2.6 Hz), 8.95 (s, 1 H), 9.82 (s, br., 1 H), 9.99 (s, br., 1 H), 12.23 (s, br., 1 H); LC/(+)-ESI-MS: *m*/*z* = 430 [M+H]⁺; LC/(-)-ESI-MS: *m*/*z* = 428 [M-H]⁻.
Example 168: **2-{[6-(3-Chloro-4-fluoro-phenylamino)-[1,2,5]oxadiazolo[3,4-b]pyrazin-5-yl]-hydrazonomethyl}-6-methozy-phenol** was prepared from (3-chloro-4-fluoro-phenyl)-(6-hydrazino-[1,2,5]oxadiazolo[3,4-b]pyrazin-5-yl)-amine hydrochloride and 2-hydroxy-3-methoxy-benzaldehyde.
   ¹H NMR: δ = 3.84 (s, 3 H), 6.89 (dd, 1 H, *J = J =* 7.9 Hz), 7.10 (dd, 1 H, *J =* 7.9 Hz, *J =* 1.3 Hz), 7.48 (dd, 1 H, ³*J*_{HH} = ³*J*_{HF} = 9.1 Hz), 7.69 (d, br., 1 H, *J* = 7.9 Hz), 7.98 (ddd, 1 H, ³*J*_{HH} = 9.1 Hz, ⁴*J*_{HF} = 4.3 Hz, ⁴*J*_{HH} = 2.6 Hz), 8.27 (dd, 1 H, ⁴*J*_{HF} = 6.8 Hz, ⁴*J*_{HH} = 2.6 Hz), 8.99 (s, 1 H), 9.72 (s, br., 1 H), 9.99 (s, br., 1 H), 12.32 (s, br., 1 H); LC/(+)-ESI-MS: *m*/*z* = 430 [M+H]⁺; LC/(-)-ESI-MS: *m*/*z* = 428 [M-H]⁻.
Example 169: **2,4-Dibromo-6-{[6-(3-chloro-4-fluoro-phenylamino)-[1,2,5]oxadiazolo-[3,4-b]pyrazin-5-yl]-hydrazonomethyl}-phenol** was prepared from (3-chloro-4-fluoro-phenyl)-(6-hydrazino-[1,2,5]oxadiazolo[3,4-b]pyrazin-5-yl)-amine hydrochloride and 3,5-dibromo-2-hydroxy-benzaldehyde.
   ¹H NMR: δ = 7.48 (dd, 1 H, ³*J*_{HH} = ³*J*_{HF} = 9.1 Hz), 7.91 (d, 1 H, *J* = 2.4 Hz), 7.94 (ddd, 1 H, ³*J*_{HH} = 9.1 Hz, ⁴*J*_{HF} = 4.3 Hz, ⁴*J*_{HH} = 2.6 Hz), 8.16 (d, 1 H, *J =* 2.4 Hz), 8.24 (dd, 1 H, ⁴*J*_{HF} = 6.8 Hz, ⁴*J*_{HH} = 2.6 Hz), 8.87 (s, 1 H), 10.03 (s, br., 1 H), 10.80 (s, br., 1 H), 12.57 (s, br., 1 H); LC/(+)-ESI-MS: *m*/*z* = 558 [M+H]⁺; LC/(-)-ESI-MS: *m*/*z* = 556 [M-H]⁻.
Example 170: **4-Bromo-2-{[6-(3-chloro-4-fluoro-phenylamino)-[1,2,5]oxadiazolo[3,4-b]pyrazin-5-yl]-hydrazonomethyl}-6-methoxy-phenol** was prepared from (3-chloro-4-fluoro-phenyl)-(6-hydrazino-[1,2,5]oxadiazolo[3,4-b]pyrazin-5-yl)-amine hydrochloride and 5-bromo-2-hydroxy-3-methoxy-benzaldehyde.
   ¹H NMR: δ = 3.87 (s, 3 H), 7.22 (d, 1 H, *J*= 2.3 Hz), 7.48 (dd, 1 H, ³*J*_{HH} = ³*J*_{HF} = 9.1 Hz), 7.98 (ddd, 1 H, ³*J*_{HH} = 9.1 Hz, ⁴*J*_{HF} = 4.3 Hz, ⁴*J*_{HH} = 2.6 Hz), 8.05 (d, br., 1 H, *J ≈* 1.7 Hz), 8.27 (dd, 1 H, ⁴*J*_{HF} = 6.8 Hz, ⁴*J*_{HH} = 2.6 Hz), 8.96 (s, 1 H), 9.90 (s, br., 1 H), 9.99 (s, br., 1 H), 12.30 (s, br., 1 H); LC/(+)-ESI-MS: *m*/*z* = 510 [M+H]⁺; LC/(-)-ESI-MS: *m*/*z* = 508 [M-H]⁻.
Example 171: **4-Chloro-2-{[6-(3-chloro-4-fluoro-phenylamino)-[1,2,5]oxadiazolo[3,4-b]pyrazin-5-yl]-hydrazonomethyl}-phenol** was prepared from (3-chloro-4-fluoro-phenyl)-(6-hydrazino-[1,2,5]oxadiazolo[3,4-b]pyrazin-5-yl)-amine hydrochloride and 5-chloro-2-hydroxy-benzaldehyde.
   ¹H NMR: δ = 6.96 (d, 1 H, *J* = 8.7 Hz), 7.36 (dd, 1 H, *J =* 8.7 Hz, *J =* 2.7 Hz), 7.48 (dd, 1 H, ³*J*_{HH} =³*J*_{HF} = 9.1 Hz), 7.98 (ddd, 1 H, ³*J*_{HH} = 9.1 Hz, ⁴*J*_{HF} = 4.3 Hz, ⁴*J*_{HH} = 2.6 Hz), 8.26 (dd, 1 H, ⁴*J*_{HF} = 6.8 Hz, ⁴*J*_{HH} = 2.6 Hz), 8.34 (d, 1 H, *J*= 2.6 Hz), 8.93 (s, 1 H), 9.99 (s, br., 1 H), 10.54 (s, br., 1 H), 11.08 (s, br., 1 H), 12.30 (s, br., 1 H); LC/(+)-ESI-MS: *m*/*z* = 434 [M+H]⁺.
Example 172: **[6-(*N*'-Benzofuran-2-ylmethylene-hydrazino)-[1,2,5]oxadiazolo[3,4-b]pyrazin-5-yl]-(3-chloro-4-fluoro-phenyl)-amine** was prepared from (3-chloro-4-fluoro-phenyl)-(6-hydrazino-[1,2,5]oxadiazolo[3,4-b]pyrazin-5-yl)-amine hydrochloride and benzofuran-2-carbaldehyde.
   ¹H NMR: 8 = 7.34 ("t", 1 H, *J ≈* 7.5 Hz), 7.43-7.50 (m, 1 H), 7.48 (dd, 1 H, ³*J*_{HH} = ³*J*_{HF} = 9.1 Hz), 7.68 ("d", 1 H, *J* ≈ 8.3 Hz), 7.72 (s, 1 H), 7.79 ("d", 1 H, *J ≈* 7.9 Hz), 7.95 (ddd, 1 H, ³*J*_{*HH*} = 9.1 Hz, ⁴*J*_{HF} = 4.3 Hz, ⁴*J*_{HH} = 2.6 Hz), 8.25 (dd, 1 H, ⁴*J*_{HF} = 6.8 Hz, ⁴*J*_{HH} = 2.6 Hz), 8.66 (s, 1 H), 10.02 (s, br., 1 H), 12.33 (s, br., 1 H); LC/(+)-ESI-MS: *m*/*z* = 424 [M+H]⁺.
Example 173: **3-(5-{[6-(3-Chloro-4-fluoro-phenylamino)-[1,2,5]oxadiazolo[3,4-b]-pyrazin-5-yl]-hydrazonomethyl}-furan-2-yl)-benzoic** acid was prepared from (3-chloro-4-fluoro-phenyl)-(6-hydrazino-[1,2,5]oxadiazolo[3,4-b]pyrazin-5-yl)-amine hydrochloride and 3-(5-formyl-furan-2-yl)-benzoic acid.
   ¹H NMR: δ = 7.33-7.39 (m, 2 H), 7.48 (dd, 1 H, ³*J*_{HH} = ³*J*_{HF} = 9.1 Hz), 7.63 (dd, 1 H, *J = J* = 7.9 Hz), 7.91-7.98 (m, 2 H), 8.13 ("d", 1 H, *J=* 7.9 Hz), 8.27 (dd, 1 H, ⁴*J*_{HF} = 6.8 Hz, ⁴*J*_{HH} = 2.6 Hz), 8.40 (t, 1 H, *J=* 1.7 Hz), 8.53 (s, 1 H), 10.02 (s, br., 1 H); LC/(+)-ESI-MS: *m*/*z* = 494 [M+H]⁺; LC/(-)-ESI-MS: *m*/*z* = 492 [M-H]⁻.
Example 174: **(3-Chloro-4-fluoro-phenyl)-(6-{*N*'-[5-(3-nitro-phenyl)-furan-2-ylmethylene]-hydrazino}-[1,2,5]oxadiazolo[3,4-b]pyrazin-5-yl)-amine** was prepared from (3-chloro-4-fluoro-phenyl)-(6-hydrazino-[1,2,5]oxadiazolo[3,4-b]pyrazin-5-yl)-amine hydrochloride and 5-(3-nitro-phenyl)-furan-2-carbaldehyde.
Example 175: **(3-Chloro-4-fluoro-phenyl)-{6-[*N*'-(5-phenyl-furan-2-ylmethylene)-hydrazino]-[1,2,5]oxadiazolo[3,4-b]pyrazin-5-yl}-amine** was prepared from (3-chloro-4-fluoro-phenyl)-(6-hydrazino-[1,2,5]oxadiazolo[3,4-b]pyrazin-5-yl)-amine hydrochloride and 5-phenyl-furan-2-carbaldehyde.
   LC/(+)-ESI-MS: *m*/*z* = 450 [M+H]⁺.
Example 176: **2-Chloro-5-(5-1[6-(3-chloro-4-fluoro-phenylamino)-[1,2,5]oxadiazolo-[3,4-b]pyrazin-5-yl]-hydrazonomethyl}-furan-2-yl)-benzoic** acid was prepared from (3-chloro-4-fluoro-phenyl)-(6-hydrazino-[1,2,5]oxadiazolo[3,4-b]pyrazin-5-yl)-amine hydrochloride and 2-chloro-5-(5-formyl-furan-2-yl)-benzoic acid.
   ¹H NMR: δ = 7.02 (d, 1 H, *J =* 3.6 Hz), 7.42 (d, 1 H, *J =* 3.6 Hz), 7.49 (dd, 1 H, ³*J*_{HH} = ³*J*_{HH} = 9.1 Hz), 7.71 (dd, 1 H, *J*= 8.1 Hz, *J*= 2.3 Hz), 7.72 (m, 1 H), 7.86 (d, 1 H, *J*= 8.1 Hz), 7.95 (ddd, 1 H, ³*J*_{HH} = 9.1 Hz, ⁴*J*_{HF} = 4.3 Hz, ⁴*J*_{HH} = 2.6 Hz), 8.25 (dd, 1 H, ⁴*J*_{HF} = 6.8 Hz, ⁴*J*_{HH} = 2.6 Hz), 8.51 (s, 1 H), 9.98 (s, br., 1 H), 12.08 (s, br., 1 H), 13.49 (s, br., 1 H); LC/(+)-ESI-MS: *m*/*z* = 528 [M+H]⁺; LC/(-)-ESI-MS: *m*/*z* = 526 [M-H]⁻.
Example 177: **(3-Chloro-4-fluoro-phenyl)-[6-(*N*'-furan-2-ylmethylene-hydrazino)-[1,2,5]oxadiazolo[3,4-b]pyrazin-5-yl]-amine** was prepared from (3-chloro-4-fluoro-phenyl)-(6-hydrazino-[1,2,5]oxadiazolo[3,4-b]pyrazin-5-yl)-amine hydrochloride and furan-2-carbaldehyde.
   ¹H NMR: δ = 6.75 (dd, 1 H, *J=* 3.4 Hz, *J=* 1.7 Hz), 7.31 (d, 1 H, *J=* 3.4 Hz), 7.48 (dd, 1 H, ³*J*_{HH} = ³*J*_{HF} = 9.1 Hz), 7.94 (ddd, 1 H, ³*J*_{HH} = 9.1 Hz, ⁴*J*_{HF} = 4.3 Hz, ⁴*J*_{HH} = 2.6 Hz), 7.99 (d, 1 H, *J*= 1.7 Hz), 8.24 (dd, 1 H, ⁴*J*_{HF} = 6.8 Hz, ⁴*J*_{HH} = 2.6 Hz), 8.52 (s, 1 H), 9.97 (s, br., 1 H), 12.15 (s, br., 1 H); LC/(+)-ESI-MS: *m*/*z* = 374 [M+H]⁺; LC/(-)-ESI-MS: *m*/*z* = 372 [M-H]⁻.
Example 178: **3-(5-{[6-(3-Chloro-4-fluoro-phenylamino)-[1,2,5]oxadiazolo[3,4-b]-pyrazin-5-yl]-hydrazonomethyl}-furan-2-yl)-benzamide** was prepared from (3-chloro-4-fluoro-phenyl)-(6-hydrazino-[1,2,5]oxadiazolo[3,4-b]pyrazin-5-yl)-amine hydrochloride and 3-(5-formyl-furan-2-yl)-benzamide.
   ¹H NMR: δ = 7.30 (d, 1 H, *J =* 3.6 Hz), 7.43 (d, 1 H, *J*= 3.6 Hz), 7.48 (s, br., 1 H), 7.49 (dd, 1 H, ³*J*_{HH} = ³*J*_{HF} = 9.1 Hz), 7.59 (dd, 1 H, *J= J=* 7.7 Hz), 7.87 ("dq", 1 H, *J=* 7.7 Hz, *J = J =* 1.1 Hz), 7.95 (ddd, 1 H, ³*J*_{HH} = 9.1 Hz, ⁴*J*_{HF} = 4.3 Hz, ⁴*J*_{HH} = 2.6 Hz), 8.05 ("dq", 1 H, *J* = 7.7 Hz, *J ≈* 1.1 Hz), 8.06 (s, br., 1 H), 8.26 (dd, 1 H, ⁴*J*_{HF} = 6.8 Hz, ⁴*J*_{HH} = 2.6 Hz), 8.35 ("t", 1 H, J= 1.3 Hz), 8.55 (s, 1 H), 9.99 (s, br., 1 H), 12.16 (s, br., 1 H); LC/(+)-ESI-MS: *m*/*z* = 493 [M+H]⁺.
Example 179: **4-(5-{[6-(3-Chloro-4-fluoro-phenylamino)-[1,2,5]oxadiazolo[3,4-b]pyrazin-5-yl]-hydrazonomethyl}-furan-2-yl)-benzoic acid** was prepared from (3-chloro-4-fluoro-phenyl)-(6-hydrazino-[1,2,5]oxadiazolo[3,4-b]pyrazin-5-yl)-amine hydrochloride and 4-(5-formyl-furan-2-yl)-benzoic acid.
   ¹H NMR: δ = 7.40 (d, 1 H, *J*= 3.6 Hz), 7.44 (d, 1 H, *J*= 3.6 Hz), 7.49 (dd, 1 H, ³*J*_{HH} = ³*J*_{HF} = 9.1 Hz), 7.95 (ddd, 1 H, ³*J*_{HH} = 9.1 Hz, ⁴*J*_{HF} = 4.3 Hz, ⁴*J*_{HH} = 2.6 Hz), 8.01 (d, 2 H, *J=* 8.8 Hz), 8.05 (d, 2 H, *J*= 8.8 Hz), 8.25 (dd, 1 H, ⁴*J*_{HF} = 6.8 Hz, ⁴*J*_{HH} = 2.6 Hz), 8.55 (s, 1 H), 9.99 (s, br., 1 H), 12.15 (s, br., 1 H), 13.02 (s, br., 1 H); LC/(+)-ESI-MS: *m*/*z* = 494 [M+H]⁺.
Example 180: **(3-Chloro-4-fluoro-phenyl)-(6-{*N*'-[5-(4-chloro-phenyl)-furan-2-yl-methylene]-hydrazino}-[1,2,5]oxadiazolo[3,4-b]pyrazin-5-yl)-amine** was prepared from (3-chloro-4-fluoro-phenyl)-(6-hydrazino-[1,2,5]oxadiazolo[3,4-b]pyrazin-5-yl)-amine hydrochloride and 5-(4-chloro-phenyl)-furan-2-carbaldehyde.
   ¹H NMR: δ = 7.29 (d, 1 H, *J*= 3.6 Hz), 7.40 (d, 1 H, *J*= 3.6 Hz), 7.48 (dd, 1 H, ³*J*_{HH} = ³*J*_{HF} = 9.1 Hz), 7.57 (d, 2 H, *J*= 8.8 Hz), 7.92 (d, 2 H, *J*= 8.8 Hz), 7.94 (ddd, 1 H, ³*J*_{HH} = 9.1 Hz, ⁴*J*_{HF} = 4.3 Hz, ⁴*J*_{HH} = 2.6 Hz), 8.25 (dd, 1 H, ⁴*J*_{HF} = 6.8 Hz, ⁴*J*_{HH} = 2.6 Hz), 8.52 (s, 1 H), 9.98 (s, br., 1 H), 12.13 (s, br., 1 H); LC/(+)-ESI-MS: *m*/*z* = 484 [M+H]⁺.
Example 181: **5-{[6-(3-Chloro-4-fluoro-phenylamino)-[1,2,5]oxadiazolo[3,4-b]pyrazin-5-yl]-hydrazonomethyl}-furan-2-sulfonic acid** was prepared from (3-chloro-4-fluoro-phenyl)-(6-hydrazino-[1,2,5]oxadiazolo[3,4-b]pyrazin-5-yl)-amine hydrochloride and 5-formyl-furan-2-sulfonic acid.
   ¹H NMR: δ = 6.64 (d, 1 H, *J* = 3.4 Hz), 7.24 (d, 1 H, *J* = 3.4 Hz), 7.47 (dd, 1 H, ³*J*_{HH} = ³*J*_{HF} = 9.1 Hz), 7.94 (ddd, 1 H, ³*J*_{HH}=9.1 Hz, ⁴*J*_{HF} = 4.3 Hz, ⁴*J*_{HH} = 2.6 Hz), 8.25 (dd, br., 1 H, ⁴*J*_{HF} = 6.8 Hz, ⁴*J*_{HH} = 2.6 Hz), 8.50 (s, 1 H), 10.01 (s, br., 1 H), 12.32 (s, br., 1 H); LC/(+)-ESI-MS: *m*/*z* = 454 [M+H]⁺; LC/(-)-ESI-MS: *m*/*z* = 452 [M-H]-.
Example **182:3-1[6-(3-Chloro-4-fluoro-phenylamino)-[1,2,5]oxadiazolo[3,4-blpyrazin-5-yl]-hydrazono}-1,3-dihydro-indol-2-one** was prepared from (3-chloro-4-fluoro-phenyl)-(6-hydrazino-[1,2,5]oxadiazolo[3,4-b]pyrazin-5-yl)-amine hydrochloride and isatin.
   ¹H NMR: δ = 6.89 (d, 1 H, *J =* 7.7 Hz), 7.01 (td, 1 H, *J=* 7.7 Hz, *J=* 0.9 Hz), 7.39 (td, 1 H, *J=* 7.7 Hz, *J*= 1.3 Hz), 7.49 (dd, 1 H, ³*J*_{HH} = ³*J*_{HF} = 9.1 Hz), 7.94-7.99 (m, 1 H), 7.95 (ddd, 1 H, ³*J*_{HH} = 9.1 Hz, ⁴*J*_{HF} = 4.3 Hz, ⁴*J*_{HH} = 2.6 Hz), 8.25 (dd, br., 1 H, ⁴*J*_{HF} = 6.8 Hz, ⁴*J*_{HH} = 2.6 Hz), 10.16 (s, br., 1 H), 10.80 (s, br., 1 H), 12.20 (s, br., 1 H); LC/(+)-ESI-MS: *m*/*z=* 425 [M+H]⁺.
Example 183: **(3-Chloro-4-fluoro-phenyl)-{6-[*N*'-(1*H*-indol-3-ylmethylene)-hydrazino]-[1,2,5]ozadiazolo[3,4-b]pyrazin-5-yl}-amine** was prepared from (3-chloro-4-fluoro-phenyl)-(6-hydrazino-[1,2,5]oxadiazolo[3,4-b]pyrazin-5-yl)-amine hydrochloride and 1*H*-indole-3-carbaldehyde.
   ¹H NMR: δ = 7.21 (td, 1 H, *J=* 7.2 Hz, *J =* 1.5 Hz), 7.25 (td, 1 H, *J =* 7.2 Hz, *J =* 1.5 Hz), 7.46-7.50 (m, 1 H), 7.48 (dd, 1 H, ³*J*_{HH} = ³*J*_{HF} = 9.1 Hz), 7.96 (ddd, 1 H, ³*J*_{HH} = 9.1 Hz, ⁴*J*_{HF} = 4.3 Hz, ⁴*J*_{HH} = 2.6 Hz), 8.08 (d, 1 H, *J=* 3.0 Hz), 8.28 (dd, 1 H, ⁴*J*_{HF} = 6.8 Hz, ⁴*J*_{HH} = 2.6 Hz), 8.62 (dd, 1 H, *J ≈* 6 Hz, *J=* 2.3 Hz), 8.91 (s, 1 H), 9.95 (s, br., 1 H), 11.90 (s, br., 1 H); LC/(+)-ESI-MS: *m*/*z* = 423 [M+H]⁺.
Example 184: **(3-Chloro-4-fluoro-phenyl)-{6-[*N*'-(2-methyl-1*H* indol-3-ylmethylene)-hydrazinol-[1,2,5]oxadiazolo[3,4-blpyrazin-5-yl)-amine** was prepared from (3-chloro-4-fluoro-phenyl)-(6-hydrazino-[1,2,5]oxadiazolo[3,4-b]pyrazin-5-yl)-amine hydrochloride and 2-methyl-1*H* indole-3-carbaldehyde.
   ¹H NMR: δ = 2.62 (s, 3 H), 7.14-7.21 (m, 2 H), 7.32-7.39 (m, 1 H), 7.48 (dd, 1 H, ³*J*_{HH} = ³*J*_{HF} = 9.1 H_{z}), 7.99 (ddd, 1 H, ³*J*_{HH} = 9.1 Hz, ⁴*J*_{HF} = 4.3 Hz, ⁴*J*_{HH} = 2.6 Hz), 8.28 (dd, 1 H, ⁴*J*_{HF} = 6.8 Hz, ⁴*J*_{HH} = 2.6 Hz), 8.56-8.64 (m, 1 H), 8.98 (s, 1 H), 9.95 (s, br., 1 H), 11.64 (s, br., 1 H), 11.78 (s, br., 1 H); LC/(+)-ESI-MS: *m*/*z* = 437 [M+H]⁺; LC/(-)-ESI-MS: *m*/*z* = 435 [M-H]⁻.
Example 185: **2-{[6-(3,4-Dichloro-phenylamino)-[1,2,5]oxadiazolo[3,4-b]pyrazin-5-yl]-hydrazonomethyl}-benzoic acid ethyl** ester was prepared from (3,4-dichloro-phenyl)-(6-hydrazino-[1,2,5]oxadiazolo[3,4-b]pyrazin-5-yl)-amine hydrochloride and 2-formyl-benzoic acid. Due to esterification, a mixture of the ethyl ester and the free acid (ratio, 70 : 30) was obtained.
   ¹H NMR: δ = 1.36 (t, 3 H, *J=* 7.1 Hz), 4.37 (q, 2 H, *J* = 7.1 Hz), 7.57-7.76 (m, 2 H), 7.68 (d, 1 H, *J* = 8.9 Hz), 7.92 (dd, 1 H, *J =* 7.6 Hz, *J =* 1.2 Hz), 8.02 (dd, 1 H, *J =* 8.9 Hz, *J =* 2.5 Hz), 8.35 (d, 1 H, *J*= 2.5 Hz), 8.69-8.74 (m, 1 H), 9.30 (s, 1 H), 10.07 (s, br., 1 H), 12.30 (s, br., 1 H); LC/(+)-ESI-MS: *m*/*z* = 472 [M+H]⁺.
Example 186: **3-{[6-(3,4-Dichloro-phenylamino)-[1,2,5]ozadiazolo[3,4-b]pyrazin-5-yl]-hydrazonomethyl}-benzoic** acid was prepared from (3,4-dichloro-phenyl)-(6-hydrazino-[1,2,5]oxadiazolo[3,4-b]pyrazin-5-yl)-amine hydrochloride and 3-formyl-benzoic acid.
   ¹H NMR: δ = 7.64 (dd, 1 H, *J = J =* 7.7 Hz), 7.69 (d, 1 H, *J =* 8.9 Hz), 8.01 (dd, 1 H, *J =* 8.9 Hz, *J =* 2.5 Hz), 8.07 ("d", 1 H, *J =* 7.7 Hz), 8.36 (d, 1 H, *J =* 2.5 Hz), 8.37 ("d", 1 H, *J* ≈ 8 Hz), 8.57 ("s", 1 H), 8.74 (s, 1 H), 10.07 (s, br., 1 H), 12.36 (s, br., 1H), 13.17 (s, br., 1 H).
Example 187: **3-{[6-(3,4-Dichloro-phenylamino)-[1,2,5]oxadiazolo[3,4-b]pyrazin-5-yl]-hydrazonomethyl}-phenol** was prepared from (3,4-dichloro-phenyl)-(6-hydrazino-[1,2,5]oxadiazolo[3,4-b]pyrazin-5-yl)-amine hydrochloride and 3-hydroxy-benzaldehyde.
   ¹H NMR: δ = 6.94 ("d", 1 H, *J=* 7.8 Hz), 7.30 (dd, 1 H, *J=* 8.0 Hz), 7.47-7.52 (m, 2 H), 7.68 (d, 1 H, *J =* 8.9 Hz), 8.01 (dd, 1 H, *J =* 8.9 Hz, *J =* 2.5 Hz), 8.36 (d, 1 H, *J =* 2.5 Hz), 8.59 (s, 1 H), 9.62 (s, br., 1 H), 10.02 (s, br., 1 H), 12.24 (s, br., 1 H); LC/(+)-ESI-MS: *m*/*z* = 416 [M+H]⁺; LC/(-)-ESI-MS: *m*/*z* = 414 [M-H]⁻.
Example 188: **4-{[6-(3,4-Dichloro-phenylamino)-[1,2,5]ozadiazolo[3,4-b]pyrazin-5-yl]-hydrazonomethyl}-phenol** was prepared from (3,4-dichloro-phenyl)-(6-hydrazino-[1,2,5]oxadiazolo[3,4-b]pyrazin-5-yl)-amine hydrochloride and 4-hydroxy-benzaldehyde.
   ¹H NMR: δ = 6.87 (d, 2 H, *J=* 8.7 Hz), 7.67 (d, 1 H, *J=* 8.9 Hz), 7.94 (d, 2 H, *J*= 8.7 Hz), 8.00 (dd, 1 H, *J*= 8.9 Hz, *J*= 2.5 Hz), 8.36 (d, 1 H, *J*= 2.5 Hz), 8.57 (s, 1 H), 9.98 (s, br., 1 H), 10.11 (s, br., 1 H), 12.10 (s, br., 1 H); LC/(+)-ESI-MS: *m*/*z* = 416 [M+H]⁺.
Example 189: **4-{[6-(3,4-Dichloro-phenylamino)-[1,2,5]oxadiazolo[3,4-b]pyrazin-5-yl]-hydrazonomethyl}-benzene-1,3-diol** was prepared from (3,4-dichloro-phenyl)-(6-hydrazino-[1,2,5]oxadiazolo[3,4-b]pyrazin-5-yl)-amine hydrochloride and 2,4-dihydroxy-benzaldehyde.
   ¹H NMR: δ = 6.33-6.42 (m, 2 H), 7.67 (d, 1 H, *J =* 8.9 Hz), 7.82 (d, 1 H, *J =* 8.4 Hz), 8.03 (dd, 1 H, *J=* 8.9 Hz, *J=* 2.5 Hz), 8.35 (d, 1 H, *J*= 2.5 Hz), 8.84 (s, 1 H), 9.98 (s, br., 1 H), 10.11 (s, br., 1 H), 10.17 (s, br., 1 H), 12.23 (s, br., 1 H); LC/(+)-ESI-MS: *m*/*z* = 432 [M+H]⁺.
Example 190: **2-{[6-(3,4-Dichloro-phenylamino)-[1,2,5]oxadiazolo[3,4-b]pyrazin-5-yl]-hydrazonomethyl}-5-methozy-phenol** was prepared from (3,4-dichloro-phenyl)-(6-hydrazino-[1,2,5]oxadiazolo[3,4-b]pyrazin-5-yl)-amine hydrochloride and 2-hydroxy-4-methoxy-benzaldehyde.
   ¹H NMR: δ = 3.80 (s, 3 H), 6.50 (d, 1 H, *J*= 2.4 Hz), 6.56 (dd, 1 H, *J=* 8.6 Hz, *J=* 2.4 Hz), 7.67 (d, 1 H, *J*= 8.9 Hz), 7.93 (d, 1 H, *J*= 8.6 Hz), 8.03 (dd, 1 H, *J=* 8.9 Hz, *J=* 2.5 Hz), 8.35 (d, 1 H, *J*= 2.5 Hz), 8.88 (s, 1 H), 10.00 (s, br., 1 H), 10.33 (s, br., 1 H), 12.29 (s, br., 1 H); LC/(+)-ESI-MS: *m*/*z* = 446 [M+H]⁺.
Example 191: **2-{[6-(3,4-Dichloro-phenylamino)-[1,2,5]oxadiazolo[3,4-b]pyrazin-5-yl]-hydrazonomethyl}-4-methoxy-phenol** was prepared from (3,4-dichloro-phenyl)-(6-hydrazino-[1,2,5]oxadiazolo[3,4-b]pyrazin-5-yl)-amine hydrochloride and 2-hydroxy-5-methoxy-benzaldehyde.
   ¹H NMR: δ = 3.78 (s, 3 H), 6.88 (d, 1 H, *J*= 8.9 Hz), 6.99 (dd, 1 H, *J*= 8.9 Hz, *J*= 3.0 Hz), 7.68 (d, 1 H, *J*= 8.9 Hz), 7.72 (d, br., 1 H, *J=* 3.0 Hz), 8.05 (dd, 1 H, *J=* 8.9 Hz, *J=* 2.5 Hz), 8.37 (d, 1 H, *J=* 2.5 Hz), 8.96 (s, 1 H), 9.80 (s, br., 1 H), 10.04 (s, br., 1 H), 12.24 (s, br., 1 H); LC/(+)-ESI-MS: *m*/*z* = 446 [M+H]⁺.
Example 192: **2-{[6-(3,4-Dichloro-phenylamino)-[1,2,5]ozadiazolo[3,4-b]pyrazin-5-yl]-hydrazonomethyl)-6-methoxy-phenol** was prepared from (3,4-dichloro-phenyl)-(6-hydrazino-[1,2,5]oxadiazolo[3,4-b]pyrazin-5-yl)-amine hydrochloride and 2-hydroxy-3-methoxy-benzaldehyde.
   ¹H NMR: δ = 3.83 (s, 3 H), 6.86 (dd, 1 H, *J = J* = 7.7 Hz), 7.06 (d, 1 H*, J=* 7.7 Hz), -7.56 ("s", br., 1 H), 7.66 (d, 1 H, *J*= 8.9 Hz), 8.03 (dd, 1 H, J= 8.9 Hz, *J*= 2.5 Hz), 8.39 (d, 1 H, J= 2.5 Hz), 8.89 (s, br., 1 H), 10.02 (s, br., 1 H), 12.32 (s, br., 1 H); LC/(+)-ESI-MS: *m*/*z* = 446 [M+H]⁺.
Example 193: **[6-(*N*'-Benzofuran-2-ylmethylene-hydrazino)-[1,2,5]oxadiazolo[3,4-b]pyrazin-5-yl]-(3,4-dichloro-phenyl)-amine** was prepared from (3,4-dichloro-phenyl)-(6-hydrazino-[1,2,5]oxadiazolo[3,4-b]pyrazin-5-yl)-amine hydrochloride and benzofuran-2-carbaldehyde.
   ¹H NMR: δ = 7.35 (ddd, 1 H, *J = J =* 7.3 Hz, *J =* 0.9 Hz), 7.47 (ddd, 1 H, *J ≈* 8 Hz, *J=* 7.3 Hz, *J*= 1.3 Hz), 7.66-7.71 (m, 2 H), 7.73 (s, 1 H), 7.80 ("d", 1 H, *J* ≈ 8 Hz), 8.01 (dd, 1 H, *J=* 8.9 Hz, *J=* 2.5 Hz), 8.36 (d, 1 H, *J=* 2.5 Hz), 8.67 (s, 1 H), 10.07 (s, br., 1 H), 12.34 (s, br., 1 H); LC/(+)-ESI-MS: *m*/*z* = 440 [M+H]⁺.
Example 194: **3-(5-{[6-(3,4-Dichloro-phenylamino)-[1,2,5]oxadiazolo[3,4-b]pyrazin-5-yl]-hydrazonomethyl}-furan-2-yl)-benzoic acid** was prepared from (3,4-dichloro-phenyl)-(6-hydrazino-[1,2,5]oxadiazolo[3,4-b]pyrazin-5-yl)-amine hydrochloride and 3-(5-formyl-furan-2-yl)-benzoic acid.
   ¹H NMR: δ = 7.37 (d, 1 H, *J =* 3.7 Hz), 7.43 (d, 1 H, *J* = 3.7 Hz), 7.64 (dd, 1 H, *J = J=* 7.9 Hz), 7.69 (d, 1 H, *J* = 8.9 Hz), 7.94 ("d", 1 H, *J =* 7.9 Hz), 8.01 (dd, 1 H, *J =* 8.9 Hz, *J =* 2.5 Hz), 8.14 ("d", 1 H, *J =* 7.9 Hz), 8.36 (d, 1 H, *J =* 2.5 Hz), 8.41 (dd, 1 H, *J = J =* 1. 5 Hz), 8.56 (s, 1 H), 10.04 (s, br., 1 H), 12.22 (s, br., 1 H), 13.18 (s, br., 1 H); LC/(+)-ESI-MS: *m*/*z =* 510 [M+H]⁺.
Example 195: **(3,4-Dichloro-phenyl)-{6-[*N*'-(5-phenyl-furan-2-ylmethylene)-hydrazino]-[1,2,5]oxadiazolo[3,4-b]pyrazin-5-yl}-amine** was prepared from (3,4-dichloro-phenyl)-(6-hydrazino-[1,2,5]oxadiazolo[3,4-b]pyrazin-5-yl)-amine hydrochloride and 5-phenyl-furan-2-carbaldehyde.
   ¹H NMR: δ = 7.25 (d, 1 H, *J*= 3.6 Hz), 7.34-7.43 (m, 2 H), 7.51 (dd, 2 H, *J= J=* 7.5 Hz), 7.69 (d, 1 H, *J* = 8.9 Hz), 7.91 (d, 2 H, *J =* 7.5 Hz), 8.01 (dd, 1 H, *J =* 8.9 Hz, *J* = 2.5 Hz), 8.36 (d, 1 H, *J*= 2.5 Hz), 8.55 (s, 1 H), 10.03 (s, br.,1 H), 12.12 (s, br., 1 H); LC/(+)-ESI-MS: *m*/*z* = 466 [M+H]⁺.
Example 196: **2-Chloro-5-(5-{[6-(3,4-dichloro-phenylamino)-[1,2,5]oxadiazolo[3,4-b]pyrazin-5-yl]-hydrazonomethyl}-furan-2-yl)-benzoic acid** was prepared from (3,4-dichloro-phenyl)-(6-hydrazino-[1,2,5]oxadiazolo[3,4-b]pyrazin-5-yl)-amine hydrochloride and 2-chloro-5-(5-formyl-furan-2-yl)-benzoic acid.
   ¹H NMR: δ = 7.02 (d, 1 H, *J*= 3.6 Hz), 7.41 (d, 1 H, *J* = 3.6 Hz), 7.68-7.73 (m, 2 H), 7.69 (d, 1 H, *J* = 8.9 Hz), 7.86 ("d", 1 H, *J =* 8.1 Hz), 8.01 (dd, 1 H, *J =* 8.9 Hz*, J =* 2.5 Hz), 8.35 (d, 1 H, J= 2.5 Hz), 8.52 (s, 1 H), 10.03 (s, br., 1 H), 12.10 (s, br., 1 H), 13.55 (s, br., 1 H).
Example 197: **(3,4-Dichloro-phenyl)-[6-(*N*'-furan-2-ylmethylene-hydrazino)-[1,2,5]-oxadiazolo[3,4-b]pyrazin-5-yl]-amine** was prepared from (3,4-dichloro-phenyl)-(6-hydrazino-[1,2,5]oxadiazolo[3,4-b]pyrazin-5-yl)-amine hydrochloride and furan-2-carbaldehyde.
   ¹H NMR: δ = 6.75 (dd, 1 H, *J*= 3.4 Hz, *J*= 1.7 Hz), 7.31 (d, 1 H, *J*= 3.4 Hz), 7.68 (d, 1 H, *J=* 8.9 Hz), 7.98-8.00 (m, 1 H), 8.00 (dd, 1 H, *J*= 8.9 Hz, *J=* 2.5 Hz), 8.34 (d, 1 H, *J*= 2.5 Hz), 8.52 (s, 1 H), 10.01 (s, br., 1 H), 12.17 (s, br., 1 H); LC/(+)-ESI-MS: *m*/*z* = 390 [M+H]⁺.
Example 198: **3-(5-{[6-(3,4-Dichloro-phenylamino)-[1,2,5]oxadiazolo(3,4-b]pyrazin-5-yl]-hydrazonomethyl}-furan-2-yl)-benzamide** was prepared from (3,4-dichloro-phenyl)-(6-hydrazino-[1,2,5]oxadiazolo[3,4-b]pyrazin-5-yl)-amine hydrochloride and 3-(5-formyl-furan-2-yl)-benzamide.
   ¹H NMR: δ = 7.30 (d, 1 H, J= 3.6 Hz), 7.43 (d, 1 H, J= 3.6 Hz), 7.48 (s, br., 1 H), 7.59 (dd, 1 H, *J = J =* 7.8 Hz), 7.68 (d, 1 H, *J* = 8.9 Hz), 7.87 ("d", 1 H, *J* = 7.9 Hz), 8.01 (dd, 1 H, *J*= 8.9 Hz, *J*= 2.5 Hz), 8.04 ("d", 1 H, *J*= 7.9 Hz), 8.33-8.37 (m, 2 H), 8.56 (s, 1 H), 10.04 (s, br., 1 H), 12.16 (s, br., 1 H); LC/(+)-ESI-MS: *m*/*z* = 509 [M+H]⁺.
Example 199: **4-(5-{[6-(3,4-Dichloro-phenylamino)-[1,2,5]oxadiazolo[3,4-b]pyrazin-5-yl]-hydrazonomethyl}-furan-2-yl)-benzoic** acid was prepared from (3,4-dichloro-phenyl)-(6-hydrazino-[1,2,5]oxadiazolo[3,4-b]pyrazin-5-yl)-amine hydrochloride and 4-(5-formyl-furan-2-yl)-benzoic acid.
   ¹H NMR: δ = 7.41 (d, 1 H, *J*= 3.6 Hz), 7.44 (d, 1 H, *J* = 3.6 Hz), 7.69 (d, 1 H, *J* = 8.9 Hz), 7.98-8.07 (m, 5 H), 8.36 (d, 1 H, *J*= 2.5 Hz), 8.56 (s, 1 H), 10.04 (s, br., 1 H), 12.17 (s, br., 1 H), 13.03 (s, br., 1 H).
Example 200: **(6-{*N*'-[5-(4-Chloro-phenyl)-furan-2-ylmethylenel-hydrazino}-[1,2,5]-oxadiazolo[3,4-b]pyrazin-5-yl)-(3,4-dichloro-phenyl)-amine** was prepared from (3,4-dichloro-phenyl)-(6-hydrazino-[1,2,5]oxadiazolo[3,4-b]pyrazin-5-yl)-amine hydrochloride and 5-(4-chloro-phenyl)-furan-2-carbaldehyde.
   ¹H NMR: δ = 7.29 (d, 1 H, *J*= 3.6 Hz), 7.40 (d, 1 H, *J* = 3.6 Hz), 7.57 (d, 1 H, *J*= 8.7 Hz), 7.69 (d, 1 H, *J*= 8.9 Hz), 7.93 (d, 1 H, *J*= 8.7 Hz), 8.01 (dd, 1 H, *J=* 8.9 Hz, *J=* 2.5 Hz), 8.35 (d, 1 H, *J*= 2.5 Hz), 8.54 (s, 1 H), 10.03 (s, br., 1 H), 12.17 (s, br., 1 H).
Example 201: **3-{[6-(3,4-Dichloro-phenylamino)-[1,2,5]oxadiazolo[3,4-b]pyrazin-5-yl]-hydrazono}-1,3-dihydro-indol-2-one** was prepared from (3,4-dichloro-phenyl)-(6-hydrazino-[1,2,5]oxadiazolo[3,4-b]pyrazin-5-yl)-amine hydrochloride and isatin.
   ¹H NMR: δ = 6.90 (d, 1 H, *J* = 7.6 Hz), 7.01 (td, 1 H, *J*= 7.6 Hz, *J*= 0.8 Hz), 7.39 (td, 1 H, *J*= 7.6 Hz, *J*= 1.2 Hz), 7.69 (d, 1 H, *J*= 8.9 Hz), 7.96 (d, br., 1 H, *J*= 7.6 Hz), 8.01 (dd, 1 H, *J* = 8.9 Hz, *J* = 2.5 Hz), 8.35 (d, 1 H, *J* = 2.5 Hz), 10.21 (s, br., 1 H), 10.81 (s, br., 1 H), 12.22 (s, br., 1 H).
Example 202: **(3,4-Dichloro-phenyl)-{6-[*N*'-(1*H*-indol-3-ylmethylene)-hydrazino]-[1,2,5]oxadiazolo[3,4-b]pyrazin-5-yl}-amine** was prepared from (3,4-dichloro-phenyl)-(6-hydrazino-[1,2,5]oxadiazolo[3,4-b]pyrazin-5-yl)-amine hydrochloride and 1*H*-indole-3-carbaldehyde.
   ¹H NMR: δ = 7.21 (dd, 1 H, *J =* 7.1 Hz, *J =* 1.5 Hz), 7.26 (dd, 1 H, *J =* 7.1 Hz, *J =* 1.5 Hz), 7.48 ("d", 1 H, *J*= 7.1 Hz), 7.68 (d, 1 H, *J*= 8.9 Hz), 8.02 (dd, 1 H, *J*= 8.9 Hz, *J*= 2.5 Hz), 8.08 (d, 1 H, *J*= 2.9 Hz), 8.38 (d, 1 H, *J=* 2.5 Hz), 8.62 ("d", 1 H, *J* ≈ 7 Hz), 8.91 (s, 1 H), 10.00 (s, br., 1 H), 11.75 (s, br., 1 H), 11.90 (s, br., 1 H); LC/(+)-ESI-MS: *m*/*z* = 439 [M+H]⁺.

### Materials and methods

### Protein Kinase Assay

The effect of the furazanopyrazine derivatives was tested on recombinant, human protein kinases. All protein kinases were expressed in Sf9 insect cells as human recombinant GST-fusion proteins or as His-tagged proteins by means of the baculovirus expression system. Protein kinases were purified by affinity chromatography using either GSH-agarose or Ni-NTH-agarose. The purity and identity of each was checked by SDS-PAGE/silver staining and by western blot analysis with specific antibodies.
A proprietary protein kinase assay (³³PanQinase® Activity Assay) was used for measuring the kinase activity. All kinase assays were performed in 96-well FlashPlates™ in a 50 µl reaction volume. The assay for all enzymes contained 60 mM HEPES-NaOH, pH 7.5, 3 mM MgCl₂, 3 mM MnCl₂, 3 µM Na-orthovanadate, 1.2 mM DTT, 50 µg/ml PEG₂₀₀₀₀ and 1 µM [γ-³³P]-ATP (approx. 5x10⁵ cpm per well).
The reaction cocktails were incubated at 30°C for 80 minutes. The reaction was stopped with 50 µl of 2% (v/v) H₃PO₄, plates were aspirated and washed two times with 200 µl of 0.9% (w/v) NaCl. Incorporation of ³³Pi was determined with a microplate scintillation counter. All assays were performed with a BeckmanCoulter/ Sagian robotic system.

### Results

i) The following compounds are efficient inhibitors of Aurora B kinase showing IC₅₀ values < 10 µM: Examples 6, 7,13,23,47, 52, 53, 58, 60, 81, 89, 95,111, 117, 126, 147, 154, 165, 173, 176, 181, 189, 194, 196, 199.
ii) The following compounds are efficient inhibitors of EGF-R showing IC₅₀ values < 1 µM: Examples 2, 7, 13, 14, 15, 23, 52, 53, 55, 60, 135, 139, 151, 152, 154, 155, 160, 161, 169, 172, 175, 176, 178, 179, 180, 182, 185, 186, 189, 190, 191, 192, 193, 194, 195, 196, 197,198,199,200,201.
iii) The following compounds are efficient inhibitors of IGF-1R showing IC₅₀ values < 1 µM: Examples 2, 14, 23, 29, 30, 35, 36, 39, 52, 53, 55, 60, 95, 134, 135, 139, 143,151, 152, 154, 155, 172, 173, 174, 175, 176, 178, 179, 180, 182, 186, 191, 192, 193, 194, 196, 197,198,199,200,201.
iv) The following compounds are efficient inhibitors of VEGF-R2 showing IC₅₀ values < 2 µM: Examples 2, 6, 7, 13, 14, 15, 23, 35, 52, 53, 60, 134, 152, 154, 155, 165, 172, 174, 176, 178, 179, 180, 182, 186, 191, 193, 194, 196, 198, 199, 200, 201.
v) The following compounds are efficient inhibitors of SRC kinase showing IC₅₀ values < 1 µM: Examples 2, 7, 13, 14, 15, 23, 29, 30, 33, 35, 36, 37, 39, 52, 53, 55, 60, 88, 90, 135, 139, 151, 152, 154, 155, 167, 172, 173, 174, 175, 176, 178, 179, 180, 182, 186, 191, 193, 194, 195, 196, 197, 198, 199, 200, 201.

## Claims

1. A compound of the general formula (I) 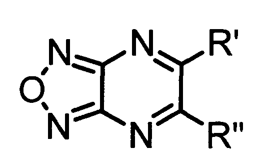 wherein:
R' represents -NR¹R² or -OR⁹
R" represents -NR⁵-NR³R⁴, -NR⁵-OR^{b}, -O-NR³R⁴;
R¹ represents hydrogen, C₁-C₆-alkyl, C₁-C₆-alkenyl, C₁-C₆-alkinyl, C₃-C₈-cycloalkyl,-COOR⁷, -SO₂R⁷, -CO-R⁹, -SO₂NHR⁹, -SO₂N(R⁹)₂, -OH, -SH, C₁-C₆-alkoxy, C₁-C₆-alkylthio, C₁-C₆-hydroxyalkyl, C₁-C₆-haloalkyloxy, C₅-C₁₅-aryl, or heteroaryl, or R¹ together with R⁵ form a 5-, 6- or 7-membered unsaturated or saturated heterocyclic ring which has optionally 1 to 3 substituents R⁸;
R² represents hydrogen, C₁-C₆-alkyl, C₁-C₆-alkenyl, C₁-C₆-alkinyl, C₃-C₈-cycloalkyl,-COOR⁷, -SO₂R⁷; -SO₂NHR⁹, -SO₂N(R⁹)₂, C₁-C₆-alkoxy, C₁-C₆-alkylthio, C₁-C₆-hydroxyalkyl, C₁-C₆-haloalkyloxy, C₅-C₁₅-aryl or heteroaryl;
R³ represents hydrogen, C₁-C₆-alkyl, C₁-C₆-alkenyl, C₁-C₆-alkinyl, C₃-C₈-cycloalkyl,-COOR⁷, -SO₂R⁷, -CO-R⁹, -SO₂NHR⁹, -SO₂N(R⁹)₂, -OH, -SH, C₁-C₆-alkoxy, C₁-C₆-alkylthio, C₁-C₆-hydroxyalkyl, C₁-C₆-haloalkyloxy, or C₅-C₁₅-aryl heteroaryl, or
R³ represents a double bond to the carbonylic carbon atom of a mono- or oligosaccaride and R⁴ is absent, or R³ represents 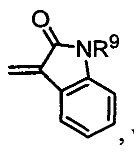 which has optionally 1 to 3 substituents R⁸ and R⁴ is absent, or R³ represents 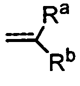 and is bonded to N via a single or double bond, and wherein
R^{a} and R^{b} are each independently hydrogen, C₁-C₆-alkyl, C₁-C₆-alkenyl, C₁-C₆-alkinyl, C₃-C₈-cycloalkyl, -COOR⁷, -SO₂R⁷, -CO-R⁹, -SO₂NHR⁹, -SO₂N(R⁹)₂, -OH, -SH, C₁-C₆-alkoxy, C₁-C₆-alkylthio, C₁-C₆ hydroxyalkyl, C₁-C₆-haloalkyloxy, C₅-C₁₅-aryl, heteroaryl, or R^{a} together with R⁴ form a 5-, 6- or 7-membered unsaturated or saturated heterocyclic ring, which has optionally 1 to 3 substituents R⁸,
or R^{a} together with R⁵ form a 5-, 6- or 7-membered unsaturated or saturated heterocyclic ring, which has optionally 1 to 3 substituents R⁸;
R⁴ represents hydrogen, C₁-C₆-alkyl, C₁-C₆-alkenyl, C₁-C₆-alkinyl, C₃-C₈-cycloalkyl,-COOR⁷, -SO₂R⁷; -SO₂NHR⁹, -SO₂N(R⁹)₂, C₁-C₆-alkoxy, C₁-C₆-alkylthio, C₁-C₆-hydroxyalkyl, C₁-C₆-haloalkyloxy, C₅-C₁₅-aryl,or heteroaryl or R⁴ together with R⁵ form a 5-, 6- or 7-membered unsaturated or saturated heterocyclic ring, which has optionally 1 to 3 substituents R⁸, or R⁴ represents a bond in case R⁴ and R^{a} form a ring system, and R⁴ is absent in case R³ is bonded to N via a double bond;
R⁵ represents hydrogen, C₁-C₆-alkyl, C₁-C₆-alkenyl, C₁-C₆-alkinyl, C₃-C₈-cycloalkyl,-COOR⁷, -SO₂R⁷, -SO₂NHR⁹, -SO₂N(R⁹)₂, C₁-C₆-alkoxy, C₁-C₆-alkylthio, C₁-C₆-hydroxyalkyl, C₁-C₆ haloalkyloxy, C₅-C₁₅-aryl, heteroaryl or R⁵ represents a bond in case R⁵ and R⁴ or R⁵ and R¹ or R⁵ and R^{a} form a ring system;
R⁷ represents hydrogen, C₁-C₆-alkyl, C₁-C₆-alkenyl, C₁-C₆-alkinyl, C₃-C₈-cycloalkyl, C₅-C₁₅-aryl or heteroaryl;
R⁸ represents independently of each other hydrogen, -COOR⁹, -CONHR⁹, -F, -Cl, -Br, -I, -CO-R⁹, -SO₂NR^{9'}R⁹, -NR⁹R^{9'}, -NR⁹-NR^{9'}, -O-CO-NR⁹R^{9'}, -NR⁹-CO-N⁹R^{9'}, -NO₂, -CN, -OH, -SH, -NR⁹-CO-(C₁-C₆)-haloalkyl, -NR⁹-SO₂-(C₁-C₆)-haloalkyl, C₁-C₆-alkyl, C₁-C₆-aminoalkyl, C₁-C₆-alkoxy, C₁-C₆-alkylthio, C₁-C₆-hydroxyalkylamino, C₁-C₆-hydroxyalkyl, amine, C₁-C₆-haloalkyloxy, C₅-C₁₅-aryl or heteroaryl;
R^{8'} represents independently of each other hydrogen, -COOR⁹, -CONHR⁹, -F, -Cl, -Br,-I, -CO-R⁹, -SO₂NR^{9'}R⁹, -NR⁹R^{9'}, -NR⁹-NR^{9'}, -O-CO-NR⁹R^{9'}, -NR⁹-CO-N⁹R^{9'}, -NR⁹-CO-(C₁-C₆)-haloalkyl, -NO₂, -CN, -NR⁹-SO₂-(C₁-C₆)-haloalkyl, C₁-C₆-alkyl, C₁-C₆-aminoalkyl, -OH, -SH, C₁-C₆-alkoxy, C₁-C₆-alkylthio, C₁-C₆-hydroxyalkylamino, C₁-C₆-hydroxyalkyl, amine, C₁-C₆-haloalkyloxy, C₅-C₁₅-aryl or heteroaryl;
R⁹ represents hydrogen, C₁-C₆-hydroxyalkyl, C₁-C₆-alkyl, C₁-C₆-aminoalkyl, C₅-C₁₅-aryl or heteroaryl;
R^{9'} represents hydrogen, C₁-C₆-hydroxyalkyl, C₁-C₆-alkyl, C₁-C₆-aminoalkyl, C₅-C₁₅-aryl or heteroaryl;
an alkyl group denotes a C₁-C₆-alkyl, C₁-C₆-alkenyl, C₁-C₆-alkinyl, or C₃-C₈-cycloalkyl residue;
an alkoxy group denotes an O-alkyl group, the alkyl group being as defined above;
an alkylthio group denotes an S-alkyl group, the alkyl group being as defined above;
an haloalkyl group denotes an alkyl group which is substituted by one to five halogen atoms, the alkyl group being as defined above;
a hydroxyalkyl group denotes an HO-alkyl group, the alkyl group being as defined above;
an haloalkyloxy group denotes an alkoxy group which is substituted by one to five halogen atoms, the alkyl group being as defined above;
a hydroxyalkyl group denotes an HO-alkyl- group, the alkyl group being as defined above;
a hydroxyalkylamino group denotes an (HO-alkyl)₂-N- group or HO-alkyl-NH- group, the alkyl group being as defined above;
an alkylamino group denotes an HN-alkyl or N-dialkyl group, the alkyl group being as defined above;
a halogen group is chlorine, bromine, fluorine or iodine;
a mono- or oligosaccharide, which is bonded through the double bond of the carbonyl to the nitrogen atom, wherein one or more of the OH groups could be protected by an acetyl or benzyl group;
a C₅-C₁₅-aryl group represents -Ph, -CH₂Ph, -C₂H₄Ph, -CH=CH-Ph, -C≡C-Ph, -o-C₆H₄-R⁸, m-C₆H₄-R⁸, -p-C₆H₄-R⁸, -o-CH₂-C₆H₄-R⁸, -m-CH₂-C₆H₄-R⁸, -p-CH₂-C₆H₄-R⁸, wherein R⁸ is as defined above or 1-naphthyl, 2-naphthyl, 1-anthracyl, 2-anthracyl optionally substituted by one or more R⁸ , which is as defined above;
a heteroaryl group represents a 5- or 6-membered heterocyclic group which contains at least one heteroatom selected from O, N, or S wherein this heterocyclic group can be fused to another ring;
and pharmaceutically acceptable salts thereof for the use as a medicament.

2. The compounds of claim 1, wherein R' is NR¹R².

3. The compounds of claim 1, wherein R" is NR⁵-NR³R⁴.

4. The compounds of claim 1, wherein R' is NR¹R² and R" is NR⁵-NR³R⁴.

5. The compounds of claim 1, wherein R' is NR¹R² and R" is NR⁵-NR³R⁴ and R² is optionally substituted phenyl and R³ is

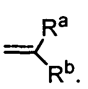

6. The use of a compound according to any one of claims 1 - 5 for the preparation of a pharmaceutical composition for the treatment of diseases which are cured or relieved by the inhibition of one or several kinases.

7. The use of a compound according to any one of claims 1 - 5 for the preparation of a pharmaceutical composition for the treatment of diseases like cell proliferation disorders, cardiovascular disorders, immunological diseases, inflammatory diseases, neuroimmunological diseases, neurodegenerative disorders, or autoimmune diseases.
